# FASCICULE DE BREVET EUROPEEN

(11) **EP 0 791 064 B1**
(45) Date de publication et mention de la délivrance du brevet: **18.02.2004**
(21) Numéro de dépôt: 95939338.0
(22) Date de dépôt: 07.11.1995
(51) Int. Cl.: C12N 15/31, C12N 15/62, A61K 39/385

(54) **PROCEDE POUR AMELIORER L'IMMUNOGENICITE D'UN COMPOSE IMMUNOGENE OU D'UN HAPTENE ET APPLICATION A LA PREPARATION DE VACCINS**
VERFAHREN ZUR VERBESSERUNG DER IMMUNOGENITÄT EINER IMMUNOGENEN ZUSAMMENSETZUNG ODER EINES HAPTENS UND VERWENDUNG ZUR HERSTELLUNG VON IMPFSTOFFEN
METHOD FOR ENHANCING THE IMMUNOGENICITY OF AN IMMUNOGENIC COMPOUND OR HAPTEN, AND USE THEREOF FOR PREPARING VACCINES

(30) Priorité: 07.11.1994 FR 9413310
(43) Date de publication de la demande: 27.08.1997
(73) Titulaire: PIERRE FABRE MEDICAMENT, 92100 Boulogne (FR)
(72) Inventeur: BINZ, Hans, F-74160 Beaumont (FR); NGUYEN NGOC, Thien, F-74160 Saint-Julien-en-Genevois (FR); ANDREONI, Christine, F-01130 Nantua (FR); NYGREN, Per, Ake, S-128 32 Skarpnack (SE); STAHL, Stefan, S-104 05 Stockholm (SE); UHLEN, Mathias, S-18351 TABY (SE)
(74) Mandataire: Ahner, Francis
(86) Numéro de dépôt international: PCT/FR1995/001466
(87) Numéro de publication internationale: WO 1996/014416

(56) Documents cités:
- EP-A- 0 327 522
- WO-A-91/16926
- WO-A-92/01471
- WO-A-93/06218
- US-A- 4 415 491
- IMMUNOMETHODS, vol. 2, no. 1, Février 1993 pages 79-92, SJÖLANDER ET AL 'BACTERIAL EXPRESSION SYSTEMS BASED ON PROTEIN A AND PROTEIN G DESIGNED FOR THE PRODUCTION OF IMMUNOGENS:APPLICATIONS TO PLASMODIUM FALCIPARUM MALARIA ANTIGENS'
- INFECTION AND IMMUNITY, vol. 58, no. 4, Avril 1990 pages 854-859, SJÖLANDER ET AL 'IMMUNOGENICITY AND ANTIGENICITY IN RABBITS OF A REPEATED SEQUENCE OF PLASMODIUM FALCIPARUM ANTIGEN PF155/RESA FUSED TO TWO IMMUNOGLOBULIN G-BINDING DOMAINS OF STAPHYLOCOCCAL PROTEIN A'
- DATABASE MEDLINE FILE SERVER STN KARLSRUHE ABR G 93202225, SJÖLANDER ET AL 'PLASMODIUM FALCIPARUM:THE IMMUNE RESPONSE IN RABBITS TO THE CLUSTERED ASPARAGINE-RICH PROTEIN (CARP) AFTER IMMUNIZATION IN FREUND'S ADJUVANT OR IMMUNOSTIMULATING COMPLEXES (ISCOMS)'
- JOURNAL OF MOLECULAR RECOGNITION, vol. 1, no. 2, Avril 1988 pages 69-74, NYGREN ET AL 'ANALYSIS AND USE OF THE SERUM ALBUMIN BINDING DOMAINS OF STREPTOCOCCAL PROTEIN G' cité dans la demande
- DATABASE CHEMICAL ABSTRACTS FILE SERVER STN KARLSRUHE ABSTRACT NO.124:84244, BERZINS ET AL 'IMMUNOGENICITY IN AOTUS MONKEYS OF ISCOM FORMULATED REPEAT SEQUENCES FROM THE PLASMODIUM FALCIPARUM ASEXUAL BLOOD STAGE ANTIGEN PF155/RESA'

## Description

Le VRS est la cause la plus fréquente d'hospitalisation des nourrissons de moins d'un an pour les infections respiratoires aiguës. Les enfants atteints de laryngotrachéobronchites, bronchiolites et pneumonies nécessitent des soins hospitaliers et chez les nourrissons présentant des maladies cardiaques congénitales, le taux de mortalité est supérieur à 37 %. D'autres troubles comme les dysplasies bronchopulmonaires, les maladies rénales et l'immunodéficience sont autant de facteurs responsables de mortalités élevées. Les infections au VRS peuvent également être une cause de mortalité chez les personnes âgées.

Dans les pays tempérés, l'épidémie du VRS se manifeste pendant la période hivernale de novembre à avril et la plus grande incidence de sérieuses maladies survient chez le nourrisson de 2 à 6 mois. On distingue deux types de VRS : VRS-A et VRS-B par la variation antigénique de la glycoprotéine G du VRS : sous-groupe A et sous-groupe B, qui circulent concurremment. Une étude récente en France de 1982 à 1990 a montré une alternance d'un sous-groupe à l'autre sur une période de 5 ans. La souche A est souvent la cause des atteintes d'infections plus graves que la souche B.

Dans les années 60, la tentative de mise au point de vaccins classiques, c'est-à-dire le VRS inactivé par le formol, analogue à des vaccins antirougeoleux, a échoué. Au lieu de conferer une protection chez l'enfant vacciné, ce type de vaccin a eu pour effet de potentialiser la maladie virale naturelle.

Le VRS humain appartient au genre pncumovirus, membre de la famille des *Paramyxoviridae*. Le génome du virus est constitué d'un brin d'ARN à polarité négative, non segmenté, codant pour 10 protéines distinctes : NS1, NS2, N, P, M, SH (ou 1A), G, F, M2 (ou 22K) et L

De nombreuses expériences publiées ont démontré que les protéines majeures impliquées dans la protection sont : F, G et N. La glycoprotéine de fusion F synthétisée comme précurseur F₀ est scindée en deux sous-unités F1 (48 kDa) et F2 (20 kDa) reliées par des ponts disulfures. La protéine F est conservée entre le VRS-A et le VRS-B (91 % homologie). A l'inverse, la glycoprotéine d'attachement G est très variable d'un sous-groupe à l'autre. Seulement une région de 13 acides aminés (aa 164 à aa 176) est hautement conservée et quatre résidus cystéine (173, 176, 182 et 186) sont maintenus dans chaque sous-groupe. Il a été démontré sur les modèles animaux que les deux glycoprotéines F et G jouent un rôle majeur dans l'immunologie du VRS. Les anticorps monoclonaux dirigés contre G et F sont capables de neutraliser le virus in vitro et passivement administrés, ils protègent le rat des cotonniers contre l'infection par le VRS.

Les traitements actuels contre l'aggravation de la maladie due au VRS chez le nourrisson sont les dégagements de l'encombrement des voies respiratoires par aspiration de mucosités et l'assistance respiratoire par ventilation. Un antiviral, la Ribavirine semble être efficace dans les cas gravement atteints. Cependant, son utilisation dans la thérapie pédiatrique est encore mal définie. L'immunisation passive avec des immunoglobulines anti-VRS est une voie alternative dans les traitements des infections graves au VRS : aucun effet secondaire indésirable n'a été observé. Néanmoins, ce type de traitement est très coûteux et difficilement extrapolable à grande échelle.

Les différentes approches de vaccination contre le VRS humain ont été entreprises : soit le vaccin protège contre l'infection du VRS chez l'animal (rongeurs, primates) mais induit une pathologie pulmonaire, soit le vaccin n'est pas assez immunogénique et ne protège pas (Connors et col, Vaccine 1992 ; 10 : 475-484).

Parmi les documents de l'art antérieur, on peut citer le document Sjôlander et al. (Immunomethods, 2, 1, 79-82, 1993), qui décrit deux systèmes d'expression bactérienne de protéines de fusion basés sur ZZ et BB ; ZZ étant un domaine synthétique dérivé de la protéine A du Staphylocoque ayant J'affinité avec l'IgG, BB étant dérivé de la protéine G du Streptocoque ayant l'affinité avec l'albumine humaine. Différents immunogènes de *Plasmodium falciparum*, P: M1, M2, M3, M5, CARP, N2-N4 ont été fusionnés avec ZZ et/ou BB.

L'administration peut notamment être entérale, parentérale, ou orale.

Le complexe entre l'immunogène et la molécule support voit son immunogénicité améliorée par rapport à celle de l'immunogène seul, en l'absence de tout autre immunostimulant.

On peut également citer le document Sjôlander et al. (Infection and Immunity, 58, 4, 858-859, 1990), qui décrit le même système dual d'expression basé sur ZZ-M1 et BB-M1, M1 étant un antigène de *Plasmodium falciparum*. Dans ce document, les lapins ont été immunisés avec ZZ-M1 en présence de l'Adjuvant Freund (FCA) et les réponses d'anticoprs conte M1 ont été détectées et analysées avec BB-M1 en ELISA.

On peut encore citer le document Sjôlander et al. (Experimental Parasitology, 76, 2, 134-145, 1993), qui décrit des expériences d'immunisation avec les protéines de fusion SpA-CARP, SpA étant la protéine A du Staphylocoque et CARP (Clustered Asparagine-Rich protein) étant une protéine de 50 kd de *Plasmodium falciparum*. La protéine SpA-CARP est utilisée comme immunogène chez le lapin, en présence dimmunostimulants tels que l'Adjuvant Freund (CPA) ou ISCOMS. Les auteurs décrivent également que des rappels d'immunisations sont effectués avec BB-CARP.

On peut enfin citer le document brevet européen publié sous le numéro EP 0 327 522 qui décrit la production d'une protéine de fusion recombinante comprenant un polypeptide dérivé de la protéine G du Streptocoque fusionné à un antigène de *Plasmodium falciparum*.

C'est pourquoi la présente invention a pour objet l'utilisation d'un immunogène, d'un antigène ou d'un haptène, et d'une molécule support pour la préparation d'un complexe destiné à être administré à un hôte et à améliorer l'immunogénicité dudit immunogène par rapport à celle de l'immunogène seul, indépendamment du mode d'administration et en absence de toute co-administration d'immunostimulant, caractérisée en ce que ledit antigène ou haptène est couplé de façon covalente à ladite molécule support, pour former ledit complexe, en ce que cette molécule support est un fragment polypeptidique issu de la protéine G du streptocoque capable de se lier spécifiquement à la sérumalbumine de mammifère, et en ce que ledit immunogène est choisi parmi le groupe suivant d'immunogènes dérivés de la glycoprotéine G du virus respiratoire syncytial (VRS) humain ou bovin, sous-groupe A ou B :
- la séquence SEQ ID No. 1, SEQ ID No. 2, SEQ ID No. 51 ou une séquence présentant au moins 80 % d'homologie avec ladite séquence SEQ ID No. 1, SEQ ID No. 2 ou SEQ ID No. 51 ; et
- les séquences SEQ ID No. 3, No. 4 et No. 14 à No. 70.

La présente invention a aussi pour objet ledit complexe formé tel que défini dans la présente utilisation selon l'invention ci-avant. Un complexe particulièrement adapté décrit dans la présente demande est obtenu par l'utilisation d'un conjugué avec un polypeptide dérivé de la protéine G du streptocoque ; cette protéine a été caractérisée par Nygren et col (J.Mol. Recognit. 1988 ; 1 : 69-74).

L'invention a pour objet l'utilisation selon la présente invention et le complexe dans lesquels la molécule support présente la séquence en acides aminés notée séquence ID n° : 74 ou une séquence présentant au moins 80 % et de préférence au moins 90 % d'homologie avec ladite séquence ID n° : 74.

Cette séquence peut être associée à des séquences de liaison favorisant son expression dans un hôte.

On peut également utiliser selon la présente description une molécule support présentant l'une des séquences ID n° : 75 ou n° : 78, ainsi que des molécules présentant au moins 80 % et de préférence au moins 90 % d'homologie avec lesdites séquences.

La séquence peptidique ID n° : 78 présente les caractéristiques suivantes :

| | | | | | |
|---|---|---|---|---|---|
| Séquence ID n° : 78 | | | | | |
| Poids Moléculaire : 26529 | | | | | |
| Gly | 10 (4.08 %); | Ala | 30 (12.24 %); | Ser | 14 ( 6.12 %); |
| Thr | 16 (6.53 %); | Val | 20 ( 8.16 %); | Leu | 23 ( 9.39 %): |
| Ile | 12 (4.90 %); | Pro | 4 ( 1.63 %); | Cys | 0 ( 0.00 %); |
| Met | 1 (0.41 %); | His | 2 ( 0.82 %); | Tyr | 9 ( 3.67 %); |
| Asp | 19 (7.76 %); | Glu | 19 ( 8.16 %); | Lys | 27 (11.02 %); |
| Arg | 5 (2.04 %); | Asn | 16 ( 6.94 %); | Gln | 8 ( 3.27 %); |
| Phe | 7 (2.86 %); | | | | |

Le complexe entre la molécule support et le composé dont on souhaite améliorer l'immunogénicité peut être produit selon la présente invention par les techniques d'ADN recombinant, notamment par insertion ou fusion dans la molécule d'ADN codant pour le support, de l'ADN codant pour l'immunogène ou l'haptène.

Selon un autre mode de mise en oeuvre de la présente invention le couplage covalent entre la molécule support et l'immunogène est réalisé par voie chimique, selon des techniques connues de l'homme du métier.

L'invention a également pour objet l'utilisation et un complexe selon la présente invention caractérisés en ce que la préparation dudit complexe comprend une étape dans laquelle on introduit dans une cellule hôte un gène de fusion comprenant une molécule d'ADN hybride produite par insertion ou fusion dans la molécule d'ADN codant pour la molécule support, de l'ADN codant pour l'innmunogène ou haptène, fusionnée avec un promoteur. Selon la présente invention, ledit gène de fusion peut être introduit par l'intermédiaire d'un vecteur pouvant avoir notamment pour origine un vecteur d'ADN qui provient d'un plasmide, d'un bactériophage, d'un virus et/ou d'un cosmide.

Un vecteur présentant la séquence ID n° : 76 ou 77 fait partie de l'invention, ainsi que le polypeptide correspondant. Ces polypeptides présentent les caractéristiques suivantes :

| | | | | | |
|---|---|---|---|---|---|
| Séquence ID n° : 76 | | | | | |
| Poids Moléculaire : 38681 | | | | | |
| Gly | 11 ( 3.15 %); | Ala | 31 (8.88 %); | Ser | 18 ( 5.16 %); |
| Thr | 37 (10.60 %); | Val | 25 (7.16 %); | Leu | 23 ( 6.59 %); |
| Ile | 15 ( 4.30 %); | Pro | 19 (5.44 %); | Cys | 4 ( 1.15 %); |
| Met | 2 ( 0.57 %); | His | 4 (1.15 %); | Tyr | 9 ( 2.58 %); |
| Asp | 22 ( 6.30 %); | Glu | 22 (6.30 %); | Lys | 48 (13.75 %); |
| Arg | 7 ( 2.01 %); | Asn | 26 (7.45 %); | Gln | 13 ( 3.72 %); |
| Phe | 12 ( 3.44 %); | Trp | 1 (0.29 %); | | |

| | | | | | |
|---|---|---|---|---|---|
| Séquence ID n° : 77 | | | | | |
| Poids Moléculaire : 39288 | | | | | |
| Gly | 12 ( 3.37 %); | Ala | 31 (8.71 %); | Ser | 22 ( 6.18 %); |
| Thr | 37 (10.39 %); | Val | 26 (7.30 %); | Leu | 23 ( 6.46 %); |
| Ile | 15 ( 4.21 %); | Pro | 21 (5.90 %); | Cys | 2 ( 0.56 %); |
| Met | 2 ( 0.56 %); | His | 4 (1.12 %); | Tyr | 9 ( 2.53 %); |
| Asp | 23 ( 6.46 %); | Glu | 22 (6.18 %); | Lys | 48 (13.48 %); |
| Arg | 7 ( 1.97 %); | Asn | 26 (7.30 %); | Gln | 13 ( 3.65 %); |
| Phe | 12 ( 3.37 %); | Trp | 1 (0.28 %); | | |

Selon la présente invention, la molécule d'ADN codant pour le complexe entre l'immunogène et la molécule support peut être intégrée dans le génome de la cellule hôte.

Dans la présente demande, il est décrit un procédé qui comprend, dans l'un de ses modes de mise en oeuvre, une étape de production du complexe, par génie génétique, dans une cellule hôte.

Selon la présente invention, la cellule hôte peut être de type procaryote et être notamment choisie dans le groupe comprenant : E. coli, Bacillus, Lactobacillus, Staphylococcus et Streptococcus ; il peut également s'agir d'une levure.

Selon un autre aspect de l'invention, la cellule hôte provient d'un mammifère.

Selon la présente invention, le gène de fusion codant pour le complexe ayant une immunogénicité améliorée peut notamment être introduit dans la cellule hôte par l'intermédiaire d'un vecteur viral.

Dans la présente description, il est indiaué que l'immunogène utilisé peut provenir de bactéries, de parasites et de virus.

Cet immunogène peut être un haptène : peptide, polysaccharide.

La présente invention a pour objet une utilisation et un complexe selon l'invention caractérisés en ce que la molécule de fusion est avantageusement exprimée, ancrée et exposée à la surface de la membrane des cellules hôtes. On utilise des molécules d'acides nucléiques qui sont capables de diriger la synthèse de l'antigène dans la cellule hôte.

Elles comprennent des séquences promoteur, signal de sécrétion liée de façon fonctionnelle et séquence codant pour une région d'ancrage membranaire, qui seront adaptées par l'homme du métier.

L'immunogène peut notamment dériver d'une glycoprotéine de surface du VRS : F et/ou G.

Des résultats particulièrement avantageux sont obtenus avec des fragments de la protéine G du VRS, sous-groupes A ou B.

Selon la présente invention, les protéines dérivées de la glycoprotéine G du sous-groupe A et du sous-groupe B du VRS peuvent être génétiquement fusionnées ou chimiquement couplées à BB.

La présente demande décrit un complexe obtenu à partir de la séquence comprise entre les amino acides 130 et 230 de la protéine G du VRS, ou une séquence présentant au moins 80% d'homologie avec ladite séquence de la protéine G.

Cette séquence peut être obtenue à partir de VRS humain ou bovin, appartenant aux sous-groupes A ou B.

La séquence comprise entre les amino acides 130 et 230 de la protéine G peut subir divers types de modifications destinées à moduler son activité immunogénique et son expression par le système hôte.

La Demanderesse a, en particulier, montré l'intérêt des polypeptides dans lesquels :
- l'acide aminé Cys en positions 173 et/ou 186 a été remplacé par un aminoacide ne formant pas de pont disulfure en particulier la serine, et/ou
- les acides aminés en positions 176 et 182 sont susceptibles de former un pont covalent autre qu'un pont disulfure notamment l'acide aspartique et l'ornithine, et/ou
- les acides aminés phénylalanine correspondant aux positions 163, 165, 168 et/ou 170 de la séquence de la protéine G sont remplacés par un acide aminé polaire, en particulier la sérine, et/ou
- la séquence comprise entre les acides aminés numérotés 162 et 170 est délétée.

Des peptides présentant l'une des séquences ID n° : 1 à 73, ou une séquence possédant au moins 90 % d'homologie avec l'une des séquences ID n° 1 à 73 sont particulièrement décrits dans la présente demande.

D'autres immunogènes sont également indiqués dans la présente demande. Ceux-ci comprennent un dérivé de la protéine de surface du virus de l'hépatite A, B et C, une protéine de surface du virus de la rougeole, une protéine de surface du virus parainfluenza 3, en particulier une glycoprotéine de surface telle que hémaglutinine, neuraminidase HN et la protéine de fusion F.

Les séquences nucléotidiques, ARN ou ADN, codant pour des complexes selon la présente invention, et pouvant comporter des éléments permettant de cibler l'expression dans certaines cellules hôtes spécifiques sont comprises dans l'invention, notamment lorsqu'il s'agit d'un gène de fusion permettant la préparation d'un tel complexe. Elles peuvent être incorporées dans un vecteur, viral ou plasmidique, ledit vecteur étant compris dans la présente invention ; ce vecteur sera administré à un mammifère, notamment au sein d'une composition pharmaceutique, pour permettre la production in situ du complexe entre l'immunogène et la molécule support.

L'invention a également pour objet un complexe entre un immunogène (P) et une molécule support, ou un vecteur selon la présente invention à titre de médicament. Les compositions pharmaceutiques contenant le gène ou le complexe avec des excipients physiologiquement acceptables sont décrits dans la présente demande.

La présente invention comprend également l'utilisation d'un complexe ou d'une séquence nucléique selon la présente invention pour la préparation d'un vaccin.

L'immunisation pourra être obtenue par l'administration de la séquence nucléotidique, seule ou par l'intermédiaire d'un vecteur viral. On peut également utiliser la cellule hôte, notamment une bactérie inactivée. Enfin, le complexe obtenu par couplage chimique ou sous forme de protéine de fusion induit une réponse d'anticorps très forte comparée à (P) seul couplé à l'adjuvant de Freund.

Dans le cadre d'un vaccin contre le VRS, la Demanderesse a montré l'efficacité de la protéine de fusion BBG2A, où G2A est un fragment de 101 acides aminés de la protéine G du VRS-A (G aa 130 - aa 230) Seq id n° 1. Immunisés chez les rongeurs, BBG2A et BBG2AδC couplés à l'Alum (Hydroxyde d'Aluminium) confèrent une protection totale contre l'épreuve de challenge contre le VRS-A (souche Long).

Les exemples qui suivent sont destinés à illustrer l'invention.

Dans ces exemples on se référera à la figure suivante :

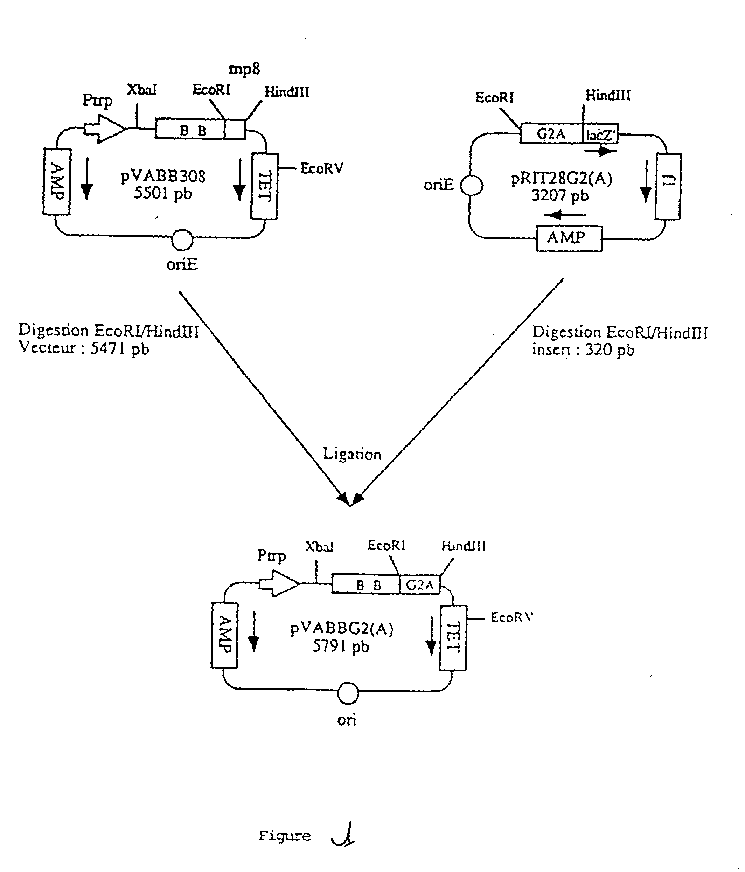

- Figure 1 : Construction de pVABBG2(A).

### EXEMPLE 1 : CLONAGE DE GENE G2A ET G2AδC DANS VECTEUR D'EXPRESSION pVABB308 ET PRODUCTION DE PROTEINES DE FUSION BBG2A, BBG2AδC DANS ESCHERICHIA COLI

### 1) Vecteur d'expression pVABB308

Le vecteur d'expression dans *E. coli*, pVABB308 (5,5 Kbp) renferme le promoteur de l'opéron tryptophane (Trp), suivi du gène codant pour la région de liaison à l'Albumine humaine BB, d'origine de la protéine G du Streptocoque (Nygren et col, J. Mol. Recognit. 1988 ; 1 : 69-74) et un site de clonage multiple mp8, auquel on peut insérer divers gènes hétérologues (voir figure 1). Le plasmide pVABB308 contient un gène de résistance à l'Ampicilline (AMP), un gène de résistance à la Tétracycline (Tet) et l'origine de réplication de E. coli. L'expression du gène est induite par addition de l'I.A.A. (Indole Acrylic Acid) dans le milieu de culture de E. coli en phase de croissance exponentielle.

### 2) Clonage de gène G2A et G2AδC dans pVABB308

### 2.1. BBG2A

Le gène codant pour G (130-230) du VRS-A a été obtenu par la méthode d'assemblage de gènes synthétiques en phase solide (selon Stahl et col, Biotechniques 1992 ; 14 : 424-434) et cloné dans le vecteur d'expression pVABB par les sites de restriction EcoRI et Hind III. Le vecteur résultant est nommé pVABBG2A (5791 pb). Le produit de fusion BBG2A est purifié à partir du cytosol de *E. coli* transformé par le vecteur pVABBG2A sous deux formes :
- une forme soluble, BBG2A (sol), après désintégration des cellules et centrifugation, le surnageant contenant les protéines solubles est directement chargé sur colonne d'affinité.
   Les produits sont récupérés après élution à pH acide.
- une forme insoluble, BBG2A (insoluble), obtenue après renaturation dans un milieu oxydant des corps d'inclusion dissous dans un agent chaotropique (Guanidine HCl) (31, 93) puis purifiée par affinité.

### 2.2. BBG2AδC

Les deux résidus cystéine (173, 186) sont remplacés par des sérines (Ser). Lors de l'assemblage de gènes, l'oligonucléotide qui renferme les 2 résidus Cys codés par le triplet (TGC) est substitué tout simplement par un autre oligonucléotide dont un des nucléotides a changé : (TCC) codant pour Ser. Nous avons voulu délibérément altérer un pont disulfure dans cette version pour garder uniquement le pont disulfure formé par les Cys (176,182), qui est critique pour la protection (Trudel et col, Virology 1991 ; 185: 749-757).

Nous avons introduit un résidu Met entre la queue d'affinité BB et G2A ou BB et G2AδC : BB-Met-G2A, BBM et G2AδC, ce qui permet d'effectuer un clivage chimique du produit de fusion par le bromure de cyanogène (CNBr) ; le mélange est passé sur colonne d'afinité HSA-Sepharose. Le peptide clivé G2A (G2AδC) n'est pas fixé et donc récupéré dans l'éluat, ensuite purifié par HPLC phase réverse.

### 3) Fermentation et purification de protéines de fusion

Dans deux erlenmeyers contenant 250 ml de milieu TSB (Triptic Soy Broth, Difco) avec de l'Ampicilline (100 µg/ml, Sigma) et de la Tétracycline (8 µg/ml, Sigma), on inocule avec *E. coli* RV308 transformés avec les plasmides pVABBG2A et pVABBG2AδC respectivement. On incube pendant 16 heures à T° = 32°C sous agitation. 200 ml de cette culture sont inoculés dans un fermenteur (CHEMAP CF3000, ALFA LAVAL) contenant 2 litres de milieu de culture. Le milieu contient (g/l) = glycérol, 5 ; sulfate d'ammonium, 2,6 ; dihydrogénophosphate de potassium, 3 ; hydrogénophosphate dipotassium, 2 ; citrate de sodium 0,5 ; extrait de levure, 1 ; Ampicilline, 0,1 ; Tétracycline 0,008 ; Thiamine, 0,07 ; sulfate de magnésium, 1 et 1 ml/l de solution de traces éléments et 0,65 ml/l de solution de vitamines. Les paramètres contrôlés durant la fermentation sont : le pH, l'agitation, la température, le taux d'oxygénation, l'alimentation de sources combinées (glycérol ou glucose). Le pH est régulé à 7,3. La température est fixée à 32°C. La croissance est contrôlée en alimentant du glycérol à un débit constant pour maintenir le signal de tension de l'oxygène dissous à 30 %. Lorsque la turbidité de la culture (mesurée à 580 nm) atteint la valeur de 80 (environ après 27 heures de culture), la production des protéines est induite par addition de l'acide indole acrylique (I.A.A.) à la concentration finale de 25 mg/l. Trois heures après induction, les cellules sont récoltées par centrifugation. Les rendements en biomasse obtenus sont environ 150 g/l de culture.

Une fraction de 30 g de biomasse humide est resuspendue dans 70 ml de solution de TST (Tris-HCl 50 mM pH 8,0, NaCl 200 mM, 0,05 % Tween 20 et EDTA 0,5 mM). Les cellules sont désintégrées par sonication (Vibracell 72401, Sonics & Materials). Après centrifugation du lysat cellulaire, le surnageant est filtré (1,2 µm) et dilué dans 500 ml de TST. Les protéines de fusion ainsi obtenues sous formes solubles sont purifiées sur colonne d'affinité : HSA-Sepharose (human scrum albumin) selon le protocole décrit par (Stahl et col, J. Immunol. Methods, 1989 ; 124 : 43-52).

Le lysat insoluble, après centrifugation, est lavé une fois avec un tampon (Tris-HCl 50 mM pH 8,5 ; MgCl₂ 5 mM). Après lavage, le culot est solubilisé dans 30 ml de chlorhydrate de guanidine 7 M, Tris-HCl 25 mM (pH 8,5), Dithiotreitol (DTT) 10 mM, suivi d'une incubation à 37°C pendant 2 heures. Les protéines solubilisées sont additionnées à un tampon de renaturation (Tris-HCl 25 mM (pH 8,5) ; NaCl 150 mM et 0,05 % Tween 20). La concentration du chlorhydrate de guanidine est ajustée à la concentration finale de 0,5 M dans le tampon de renaturation avant l'addition des protéines de fusion solubilisées. Le mélange est incubé à température ambiante, sous agitation modérée, pendant 16 heures. Après centrifugation, les produits de fusion solubles dans le surnageant sont purifiés sur colonne HSA-Sepharose. Les protéines de fusion purifiées sont analysées sur gel SDS-PAGE (12 %) dans des conditions réduites, sur l'appareil MINI PROTEAN II SYSTEM (BIORADS). Les protéines sont visualisées avec du Coomassie brilliant blue R250.

### EXEMPLE 2 : EFFET PORTEUR DU POLYPEPTIDE BB ET IMMUNOGENICITE DE BBG2AδC

### 1. Schéma d'immunisations

Des souris C57B1/6 (5 par lot) ont reçu 2 injections sous-cutanée de 10 µg d'équivalent G2AδC en présence d'adjuvants de Freund à J0 (adjuvant complet) et J14 (adjuvant incomplet). A J21, les sérums ont été testés individuellement en ELISA pour la production d'anticorps spécifiques de G2AδC. Le titre anticorps est déterminé comme étant l'inverse de la dilution du sérum donnant 2 fois l'absorbance du sérum de l'animal avant immunisation. Les résultats présentés sont la moyenne arithmétique des titres anticorps anti-G2AδC obtenus pour chacun des lots.

### TABLEAU DE RESULTATS

| ANTIGENE | Titre moyen d'anticorps anti G2AδC |
|---|---|
| 1) G2AδC + AF | 180 |
| 2) BBG2AδC + AF | 92 800 |
| 3) G2AδC + BB + AF | 1200 |

### 2. Résultats

Le tableau ci-dessus montre que G2AδC est un faible immunogène même en présence d'adjuvant de Freund. La protéine BB a un faible pouvoir adjuvant, puisqu'additionnée à G2AδC le titre anticorps anti-G2AδC n'augmente que d'un log. En revanche, la fusion de BB à G2AδC accroit la production d'anticorps anti-G2AδC d'environ 3 log.

Nous pouvons donc conclure que BB est une excellente protéine porteuse pour G2AδC et que la protéine de fusion BBG2AδC est très immunogène.

### EXEMPLE 3 :ETUDE DE PROTECTION INDUITE PAR DES PROTEINES DE FUSION BBG2A ET BBG2AδC CHEZ LES RONGEURS

### a) Protocoles d'étude

Des souris BALB/c et des rats des cotonniers (*Sigmodon hispidus*) femelles (IFFA-CREDO), modèles animaux pour l'infection par le VRS, sont utilisés dans les expériences d'immunisation.

Les groupes d'animaux reçoivent 1, 2, ou 3 doses de 200 µg, 20 µg, 2 µg ou 0,2 µg de candidat vaccin VRS-A dans 20 % d'hydroxyde d'aluminium (Al(OH)₃) (v/v) à 2 semaines d'intervalle. Les souris sont immunisées par voie intrapéritonéale (i.p.), les rats des cotonniers par injections intramusculaires (i.m.). Les groupes contrôles reçoivent 105 DICT₅₀ de VRS-A ou du PBS-A (PBS sans Ca²⁺ ni Mg²⁺) dans 20 % d'hydroxyde d'aluminium (v/v).

Trois à quatre semaines après la dernière immunisation, les animaux sont challengés par voie intranasale (i.n.) avec environ 10⁵ DICT₅₀ VRS-A. Ils sont sacrifiés 5 jours plus tard, après ponction sanguine intracardiaque. La présence du virus dans leurs poumons est testée selon Trudel et col, Virology 1991 ; **185**: 749-757).

Les différents produits testés sont BBG2A, BBG2AδC et BB seul.

### b) Tableau de résultats

### Résultats de protection chez les rongeurs

**Tableau 3.1**

| | Souris | | Rat des cotonniers | |
|---|---|---|---|---|
| Antigènes | Protection* | Protection complète° | Protection | Protection complète |
| BBG2A | 41/-41+ | 38/41 | 22/22 | 22/22 |
| BBG2AδC | 32/34 | 27/34 | 8/13 | 7/13 |
| BB | 0/20 | 0/20 | 0/3 | 0/3 |
| RSV-A | 28/28 | 28/28 | 17/17 | 17/17 |
| PBS-A | 0/29 | 0/29 | 0/21 | 0/21 |

| | | | | |
|---|---|---|---|---|
| * Protection = une réduction de virus dans les poumons de ≥ log₁₀2 par rapport au titre moyen de virus dans les poumons des souris immunisées avec PBS-A. | | | | |
| ° Protection complète = aucun virus détecté dans les poumons. | | | | |
| + X/Y où X = nombre des animaux protégés ou complètement protégés ; Y = nombre des animaux testés | | | | |

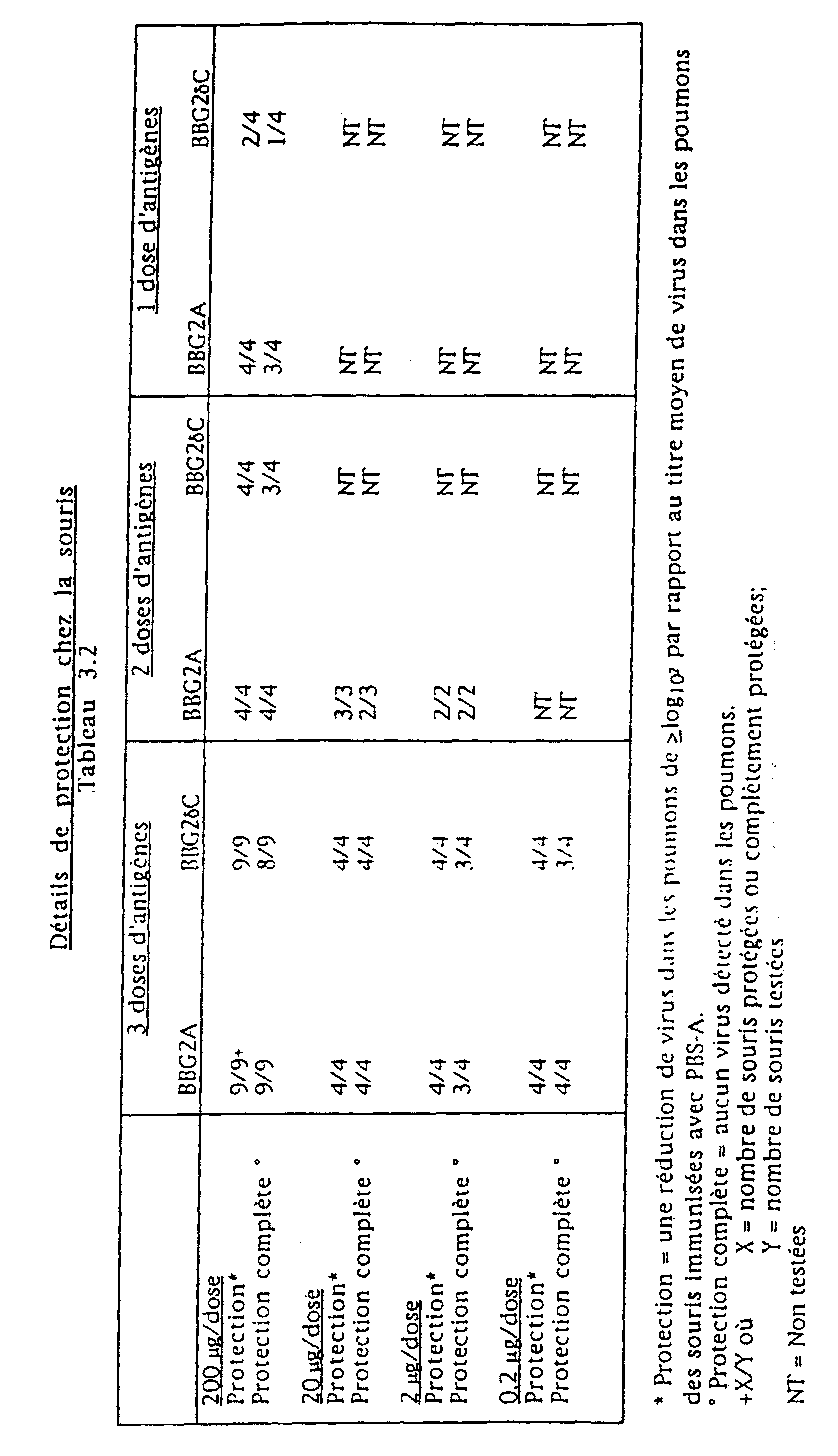

### Résultats des test immunologiques chez les souris

**Tableau 3.3**

| Antigènes | ELISA(LOG₁₀ moyen) | Anticorps neutralisants (titre moyen/25µl) |
|---|---|---|
| BBG2A | 5.09(28) | ≥ 512 (15) |
| BBG2AδC | 3.71 (29) | ≥ 256 (12) |
| RSV-A | 5.32(21) | ≥ 512 (12) |
| **( ) = nombre d'animaux testés** | | |

### c) Discussion

Les résultats expérimentaux de protection sont présentés dans les tableaux 3.1. et 3.2. Chaque molécule a été testée au cours de 2 expériences indépendantes au moins. Les résultats montrent clairement que, indépendamment des protocoles d'immunisation utilisés, BBG2A protège les rongeurs contre une infection pulmonaire par le VRS-A. Dans nos conditions expérimentales, une injection unique de 200 µg, 2 de 2 µg, ou 3 de seulement 0,2 µg de BHG2A sont suffisantes pour protéger les souris contre l'infection (Tableau 3.2). Du virus a été détecté chez un troisième animal du méme groupe mais à la limite de détection. Ces résultats suggèrent que BBG2A présente un potentiel et une efficacité très comparables à ceux du VRS-A chez les animaux immunisés contrôles et à ceux des vaccins candidats sous-unitaires du VRS-A décrits dans la littérature.

BBG2AδC a aussi été efficace chez la souris, protégeant 32 animaux sur 34 contre l'infection pulmonaire. Deux doses de 200 µg se sont révélées efficaces, tout comme 3 injections de 0,2 µg. Ainsi, dans ces schémas d'immunisation comportant plusieurs injections, BBG2AδC s'est montré comparable en activité et en efficacité chez la souris aux candidats vaccins sous-unitaires du VRS-A déjà décrits.

Les résultats des tests immunologiques de la réponse humorale et cellulaire, chez la souris BALB/c, sont présentés sur le tableau 3.3. En général, les titres moyens d'anticorps spécifiques anti-VRS-A obtenus en technique ELISA sont considérés comme un des reflets de l'activité protectrice des vaccins candidats. Les sérums des souris immunisées avec le VRS-A ont montré de façon constante des titres d'anticorps anti-VRS-A élevés. Le virus n'a jamais été détecté dans les poumons de ces animaux. Les souris immunisées par BBG2A ont montré des titres moyens d'anticorps anti-VRS-A semblablement élevés et ont toujours été protégées lors d'un challenge par le VRS-A.

BBG2AδC a permis d'induire des titres moyens d'anticorps anti-VRS-A inférieurs par rapport aux molécules mentionnées ci-dessus. De plus, les animaux immunisés par cette molécule ont montré une protection légèrement réduite. Si les sérums de quelques animaux immunisés par BBG2AδC ont montré des titres d'anticorps spécifiques anti-VRS-A très faibles (données non représentées), certains de ces animaux ont néanmoins été totalement protégés lors d'un challenge par le VRS-A.

Les études de protection mettent en évidence l'efficacité protectrice des vaccins candidats sous-unitaires anti-VRS-A. Deux molécules, BBG2A et BBG2AδC, se sont révélées très efficaces dans deux modeles de rongeurs pour l'infection au VRS-A, lors du challenge avec le virus homologue.

### EXEMPLE 4: EFFICACITÉ IMMUNOGÉNIQUE ET PROTECTRICE DE BBG2AδC PAR RAPPORT À G2AδC CHEZ LA SOURIS BALB/c.

### Matériels et méthodes:

Des groupes de 4 souris BALB/c, séronégatives vis-à-vis du VRS-A, ont été immunisées par injections intrapéritonéales (i.p.) 2 fois à 2 semaines d'intervalle avec 5.1, 0.51 et 0.051 nM de BBG2AδC et de G2AδC. La dernière molécule est dérivée d'un clivage chimique de BBG2AδC par le Bromure de Cyanogène. Un groupe de 3 souris a été immunisé 2 fois à 2 semaines d'intervalle par le tampon PBS pour servir de témoins négatifs. L'Alhydrogel (Al(OH)₃) (20% v/v) (Superfos BioSector, Danemark) a été utilisé comme adjuvant pour toutes les immunisations. Une ponction sanguine est réalisée 2 semaines après la dernière immunisation afin de déterminer les titres ELISA contre le G2AδC. Les souris ont été challengées avec le VRS-A (10⁵ DICT₅₀) 3 semaines après la dernière immunisation. Elles ont été sacrifiées 5 jours plus tard et soumises à une ponction cardiaque afin de titrer les anticorps anti-VRS-A post-challenge, et les poumons ont été prélevés afin de titrer le VRS-A pulmonaire.

### Résultats:

### Voir Tableau 4.

Les résultats d'ELISA anti-G2AδC indiquent que BBG2AδC est toujours plus immunogénique que G2AδC, quelle que soit la dose administrée (0.051 - 5.1 nM). Surtout à 0.051 nM, BBG2AδC induit un titre moyen anti-G2AδC de log₁₀ 3.27, alors que la même concentration de G2AδC n'induit pas des anticorps anti-G2AδC détectables. De même, pour ce qui concerne les ELISA anti-VRS-A; 4 souris sur 4 immunisées avec 5.1 ou 0.51 nM de BBG2AδC ont été séropositives, dont des titres moyens de log10 2.67 et 2.78. respectivement. Deux souris sur 4, cependant, immunisées avec 5.1 nM de G2AδC ont été séropositives, dont une à la limite de détection de l'essai et un titre moyen de log₁₀ ≤ 2.19. Les souris immunisées avec 0.51 ou 0.051 nM de G2AδC n'ont pas eu d'évidence d'anticorps anti-VRS-A.

Toutes les souris immunisées avec 5.1 ou 0.51 nM de BBG2AδC ont eu leurs poumons protégés contre un challenge avec le virus homologue. A part chez une souris immunisée avec 0.51 nM de BBG2AδC qui n'a présenté du virus qu'à la limite de détection de la méthode, la présence de virus pulmonaire n'a été mise en évidence chez aucun des autres animaux. Après immunisation avec 0.051 nM de BBG2AδC, 3 souris sur 4 ont été protégées, dont 2 sans évidence de virus pulmonaire. La 4éme a eu une diminution de virus pulmonaire de l'ordre de log₁₀ 1.16 par rapport au titre moyen des témoins immunisés avec le PBS-A.

Trois souris sur 4, immunisées avec 5.1 nM de G2AδC, ont eu les poumons protégés contre un challenge avec le VRS-A. La 4éme a eu une diminution du virus pulmonaire de l'ordre de log₁₀ 1.75 par rapport au titre moyen des témoins immunisés avec le tampon PBS-A. Parmi les souris protégées, il n'y a eu qu'une seule sans virus pulmonaire détecté. Nous observons les mêmes résultats après immunisation avec 0.51 nM de G2AδC, mise à part une souris non-protégée qui n'a pas présenté de diminution importante de virus pulmonaire par rapport aux témoins immunisés avec le tampon PBS-A. Les voies respiratoires inférieures des souris immunisées avec 0.051 nM de G2AδC n'ont pas été protégées contre un challenge avec le virus homologue.

### Conclusions :

Les résultats indiquent, selon les conditions de cette étude, que BBG2AδC est de l'ordre de 10 à 100 fois plus efficace queG2AδC pour l'induction des réponses immunitaires qui protègent les poumons contre un challenge avec le VRS-A.

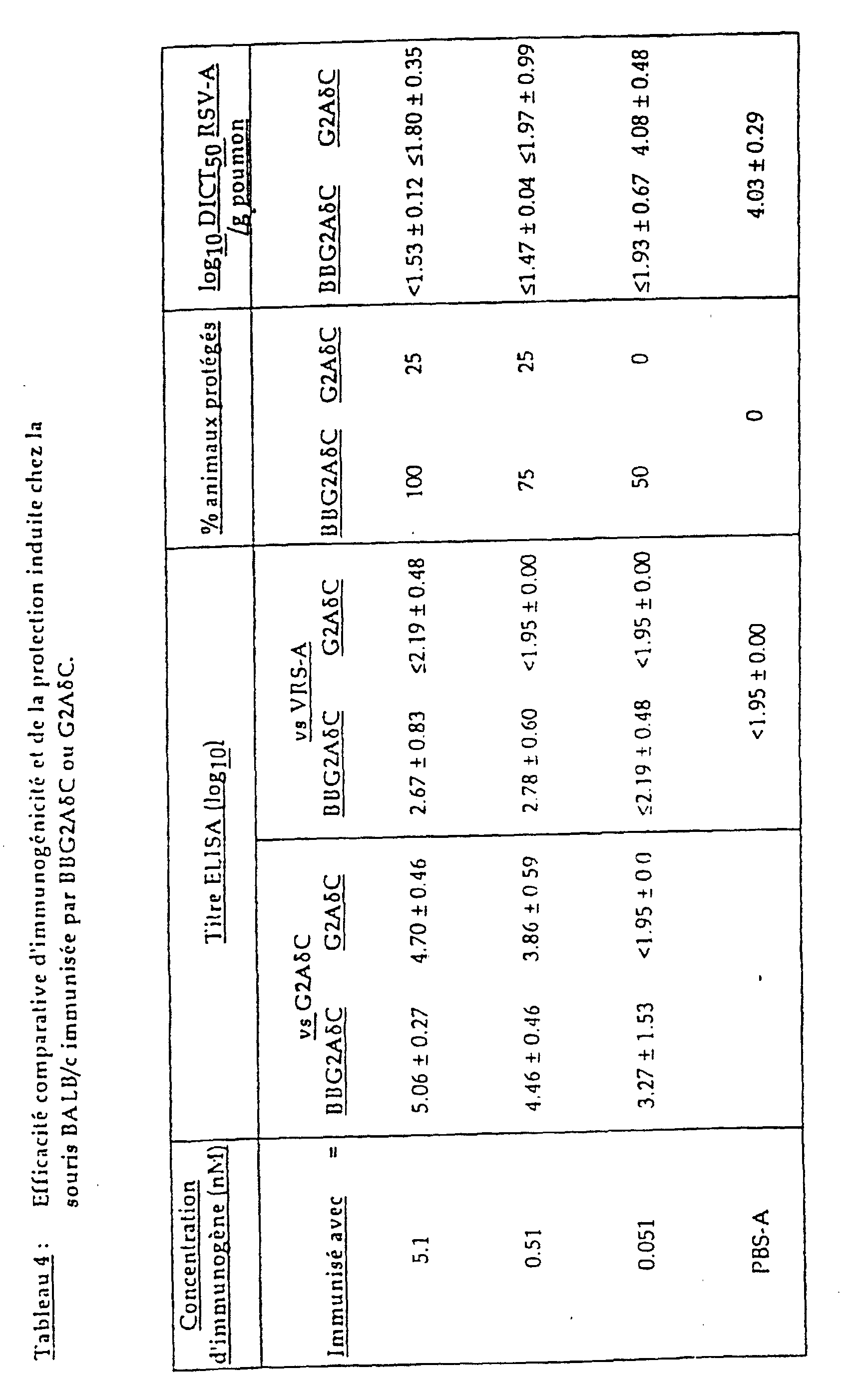

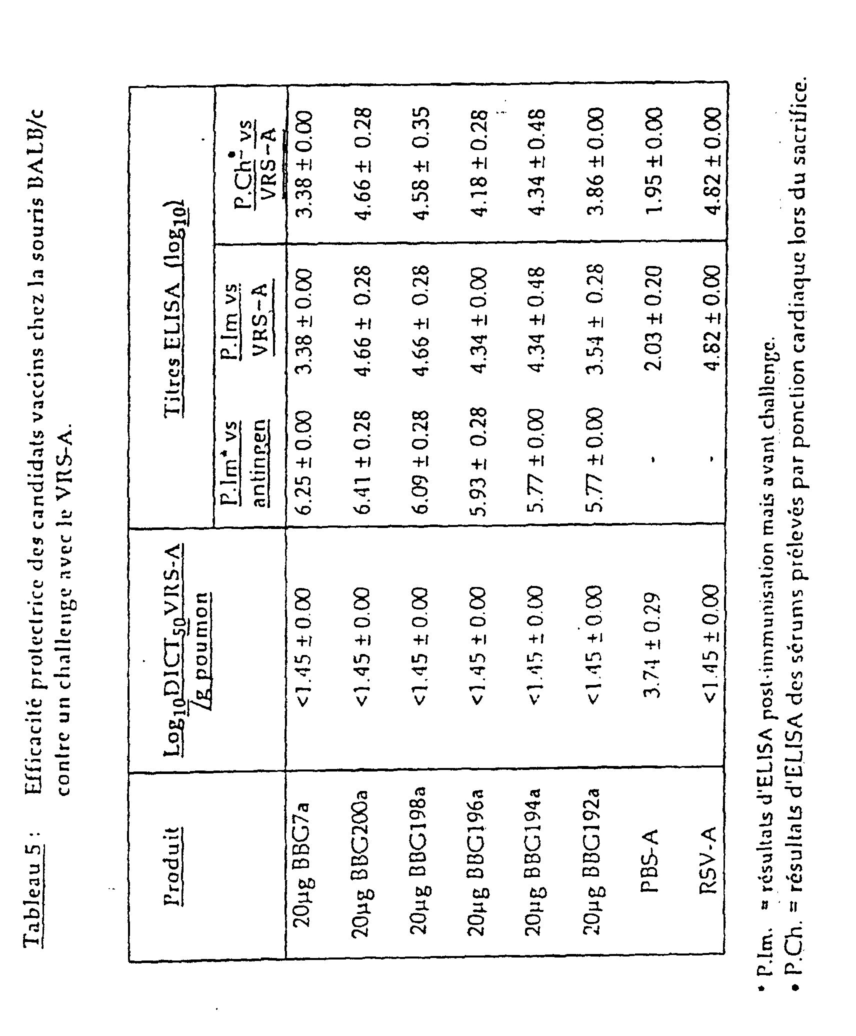

### EXEMPLE 5: EFFICACITÉ PROTECTRICE DES CANDIDATS VACCINS CHEZ LA SOURIS BALB/c CONTRE UN CHALLENGE AVEC LE VRS-A.

### Matériels et Méthodes :

Des groupes de 3 souris ont été immunisés 2 fois à 2 semaines d'intervalle avec 20 µg des produits suivants:
BBG7A, BBG200A, BBG198A, BBG196A, BBG194A et BBG192A,
G7A(Seq id 29) ; G200(Seq id 23) ; G198(Seq id 24) ; G196(Seq id 25); G194(Seq id 26) ; G192(Seq id 27).

Deux groupes de 6 et 4 souris ont été immunisés 2 fois à 2 semaines d'intervalle par le PBS-A et le VRS-A (10⁵ TCID₃), respectivement, comme témoins. L'Alhydrogel (Al(OH)₃) (20% v/ v) a été utilisé comme adjuvant pour chaque immunisation. Tous les animaux ont été prélevés à l'oeil avant la 1ère immunisation afin de vérifier leur séronégativité vis-à-vis du VRS-A. Tous étaient séronégatifs ou ont eu des titres à la limite de détection de l'essai ELISA. Deux semaines après la 2ème immunisation, ils ont été prélevés à l'oeil pour confirmer leur séroconversion vis-à-vis des antigènes et du VRS-A. Trois semaines après la dernière immunisation, les souris ont été challengées par voie intra-nasale avec 10⁵ TCID₅₀ de VRS-A. Les souris ont été sacrifiées 5 jours après le challenge: elles ont été soumises à une ponction cardiaque; les poumons ont été prélevés afin de titrer le virus dans les voies respiratoires inférieures. Les sérums post-challenge ont été testés en ELISA contre les antigènes viraux.

### Résultats :

### Voir tableau 5.

Les souris immunisées avec BBG200A, BBG198A, BBG196A, BBG194A, BBG192A, et BBG7A ont été protégées contre un challenge avec le VRS-A sans évidence de virus dans les poumons. Tous les produits ont induit des titres moyens d'anticorps élevés contre l'antigène d'immunisation (log₁₀ 5.77 - 6.41) et le VRS-A (log₁₀ 3.38 - 4.66).

Ces résultats sont en accord avec ceux issus des souris immunisées avec le VRS-A.

### Conclusions :

Les molécules ci-dessus sont très immunogéniques et induisent des réponses immunitaires capables de protéger les poumons de la souris BALB/c contre un challenge avec le VRS-A. Ils constituent donc des candidats potentiels vaccins contre le VRS-A.

### EXEMPLE 6: EFFICACITÉ PROTECTRICE DE BB-G4A CHEZ LA SOURIS BALB/c CONTRE UN CHALLENGE AVEC LE VRS-A.

### Matériels et Méthodes :

Deux groupes de 3 souris ont été immunisés 2 fois à 2 semaines d'intervalle avec 20 µg de BB-G4A ou TT-G4A. Les molécules sont dérivées d'un couplage chimique du peptide G4A (residues 172-187) sur les protéines porteuses (soit BB soit TT). Deux groupes de 6 et 4 souris ont été immunisés 2 fois à 2 semaines d'intervalle par le PBS-A et le VRS-A (10⁵ TCID₅₀), respectivement, comme témoins. L'Alhydrogel (Al(OH)₃) (20% v/v) a été utilisé comme adjuvant pour chaque immunisation. Tous les animaux ont été prélevés à l'oeil avant la 1ère immunisation afin de vérifier leur séronégaffvité vis-à-vis du VRS-A. Tous étaient séronégatifs ou ont eu des titres à la limite de détection de l'essai ELISA. Deux semaines après la 2ème immunisation, ils ont été prélevés à l'oeil pour confirmer leur séroconversion vis-à-vis des antigènes et du VRS-A. Trois semaines après la dernière immunisation, les souris ont été challengées par voie intra-nasale avec 10⁵ TCID₅₀ de VRS-A. Les souris ont été sacrifiées 5 jours après le challenge: elles ont été soumises à une ponction cardiaque; les poumons ont été prélevés afin de titrer le virus dans les voies respiratoires inférieures. Les sérums post-challenge ont été testés en ELISA contre les antigènes viraux.

### Résultats :

BB-G4A, protéine dérivée d'un couplage du peptide G4A sur BB, a protégé les souris sans évidence du virus pulmonaire. TT-G4A, protéine dérivée d'un couplage du peptide G4A sur TT a été moins efficace que BB-G4A en ce qui concerne la protection des poumons; 2 souris sur 3 ont été protégées, respectivement, dont 1 sans évidence de virus pulmonaire. La souris non-protégée a eu une diminution du taux de virus de l'ordre de log₁₀ 1.52 par rapport aux témoins immunisés par le PBS-A. Les rapports porteur:peptide pour BB-G4A et TT-G4A sont de ∼1:7 et ∼1:21, respectivement. Ces résultats indiquent donc que BB est un meilleur porteur de G4A que TT.

Les 2 produits ont induit des titres d'anticorps élevés contre l'antigène d'immunisation (log₁₀ 5.77 et 6.73, respectivement, pour les sérums anti-BB-G4A et anti-TT-G4A post-immunisation). Par contre, les animaux immunisés avec ces vaccins candidats ont eu des titres anti-VRS-A très faibles (log₁₀ 2.11 ± 0.28 et 2.43 ± 0.48, respectivement, pour les sérums anti-BB-G4A et anti-TT-G4A postimmunisation).

### Conclusions :

BB-G4A est capable de protéger les souris contre un challenge avec le VRS-A sans évidence du virus pulmonaire. Il confirme donc son potentiel comme vaccin anti-VRS-A. Les résultats indiquent également que BB est un meilleur porteur de G4A que TT.

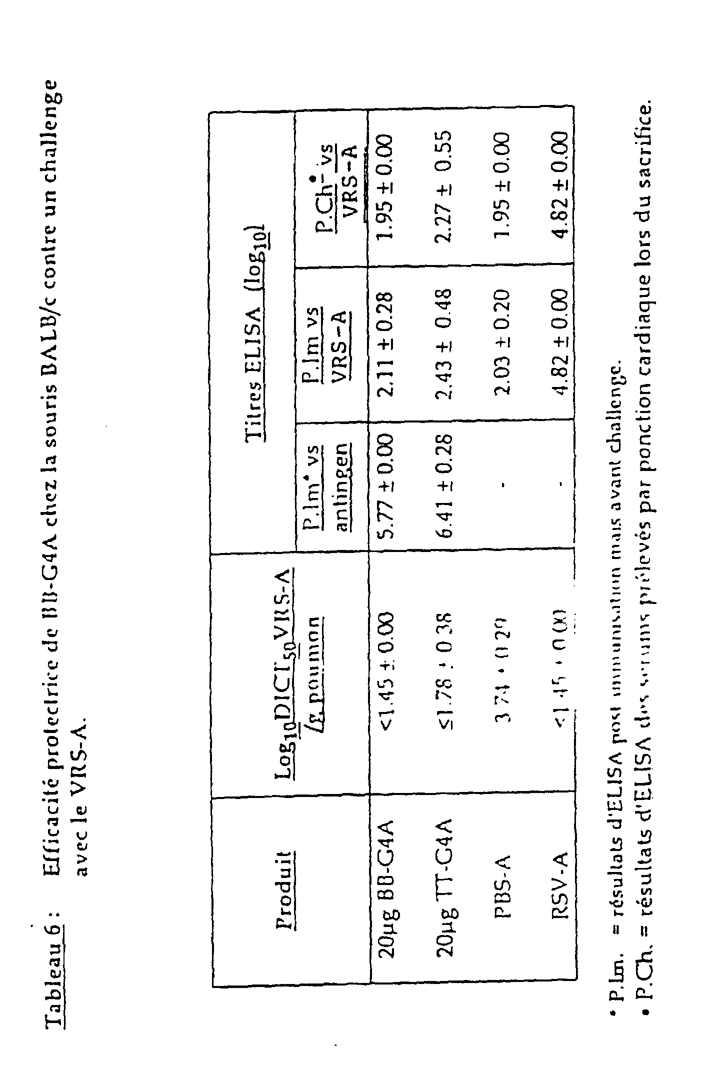

### EXEMPLE 7: PROTECTION CROISÉE DES POUMONS DES SOURIS BALB/c IMMUNISÉES AVEC BBG2A PAR VOIE INTRAPÉRITONÉALE VIS-À-VIS D'UN CHALLENGE HÉTÉROLOGUE AVEC LE VRS-B (SOUCHE 8/60).

### Matériels et Méthodes :

Des souris BALB/c ont été immunisées soit 2 fois soit 3 fois à 2 semaines d'intervalle avec 20 µg de BBG2A par injection intrapéritonéale. Un autre groupe de souris ont été immunisées de la même façon par le PBS-A comme témoins. L'Alhydrogel (Al(OH)₃) (20% v/v) a été utilisé comme adjuvant pour chaque immunisation. Un prélèvement de sang a été réalisé avant la 1ère immunisation afin de vérifier leur séronégativité vis-à-vis du VRS-A. Trois semaines après la dernière immunisation les souris ont été challengées par voie intra-nasale avec 10⁵ TCID₅₀ de VRS-A ou avec avec 10⁵ TCID₅₀ de VRS-B. Les souris ont été sacrifiées 5 jours après le challenge: elles ont été soumises à une ponction cardiaque; les poumons ont été prélevés afin de titrer le virus dans les voies respiratoires inférieures. Les sérums post-challenge ont été testés en ELISA contre les antigènes viraux.

### Résultats :

Toutes les souris étaient séronégatives pour le VRS-A au début de l'étude. Le premier groupe, 11 souris sur 11, immunisées avec 20 µg de BBG2A, ont été protégées vis-à-vis d'un challenge avec le VRS-A. Le deuxième groupe, 11 souris sur 11, ont été également protégées vis-à-vis d'un challenge hétérologue avec le VRS-B (tableau 7).

### Conclusions :

L'immunisation des souris BALB/c avec l'antigène BBG2A confère une protection non seulement contre le VRS-A mais également vis-à-vis d'un challenge avec le VRS-B. L'antigène BBG2A induit donc une protection croisée vis-à-vis d'un challenge hétérologue.

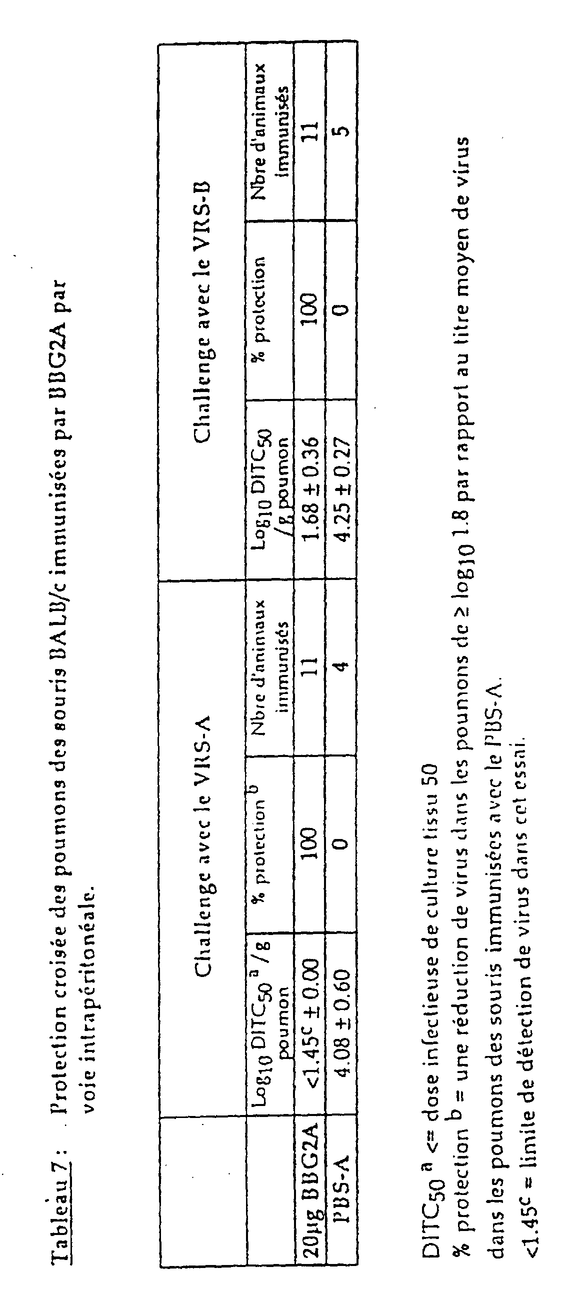

### EXEMPLE 8 : ETUDE DE L'EFFET PRIMING DE BB SUR L'IMMUNISATION AVEC BBG2A

Des souris BALB/c sont sensibilisées à la protéine BB puis reçoivent une injection de BBG2A. Les titres IgG anti-G2A obtenus chez ces animaux sont comparés de ceux obtenus avec des souris recevant deux injections de BBG2A.

### Matériel et Méthodes

Deux souris BALB/C (N=5/lot) sont immunisées en sous-cutané comme décrit ci-dessous :

| | **J0** | **J14** |
|---|---|---|
| lot 1 | 0.1 ml PBS | 0.1 ml PBS |
| lot 2 | 20 µg BBG2A + AFC | 20 µg BBG2A + AFI |
| lot 3 | 100 µg BB + AFC | 20 µg BBG2A + AFI |
| AFC : Adjuvant Freund complet ; AFI : Adjuvam Freund incomplet Le sang des animaux est prélevé à J7 et J21 et le titre IgG sérique anti-G2A est déterminé individuellement par ELISA. | | |

### Résultats

| Tableau de titres IgG anti-G2A | | | | |
|---|---|---|---|---|
| | **J7** | **J21** | | |
| | **LOT 2** | **LOT 1** | **LOT 2** | **LOT 3** |
| S1 | 2 | 2 | 3.81 | 3.51 |
| S2 | 2 | 2 | 3.81 | 4.11 |
| S3 | 2 | 2 | 3.81 | 4.41 |
| S4 | 2 | 2 | 4.41 | 3.51 |
| S5 | 2 | 2 | 3.81 | 4.71 |
| m ± σ | 2 | 2 | 3.93 ± 0.27 | 4.05 ± 0.54 |

En résumé, le tableau de titres IgG anti-G2A à J7 et J21 :

| | **J0** | **J7** | **J14** | **J21** |
|---|---|---|---|---|
| lot 1 | 0.1 ml PBS | - | 0.1 ml PBS | 2 |
| lot 2 | 20 µg BBG2A + AFC | 2 | 20 µg BBG2A + AFI | 3.93 ± 0.27 |
| lot 3 | 100 µg BB + AFC | - | 20 µg BBG2A + AFI | 4.05 ± 0.54 |

### LOT 2 : 2 iniections de BBG2A

Une semaine après la première injection de 20 µg de BBG2A, on ne détecte pas d'IgG anti-G2A. En revanche, une semaine après la seconde injection de BBG2A il y a une forte production d'IgG anti-G2A : environ 4log10.

### LOT 3 : injection n° 1 = BB, injection n° 2 = BBG2A

Après sensibilisation avec 100 µg de BB, une injection de 20 µg de BBG2A suffit pour induire un titre IgG anti-G2A de 4 log10, titre semblable à celui obtenu avec 2 injections de 20 µg de BBG2A.

### Conclusion :

Ces résultats montrent que BB induit la production de cellules Th mémoires qui ont fourni le "help" nécessaire aux cellules B spécifiques de G2A lors de l'immunisation primaire avec BBG2A, ce qui aboutit à une réponse secondaire de type IgG. Ainsi, des cellules B naïves peuvent donc êtres stimulées pour produire des anticorps anti-G2A.

BB fournit donc le "T cell help" adéquat à la production d'anticorps dirigés contre G2A ; en cela, il se comporte comme une protéine porteuse.

## Revendications

1. Utilisation d'un immunogène, d'un antigène ou d'un haptène, et d'une molécule support pour la préparation d'un complexe destiné à être administré à un hôte et à améliorer l'immunogénicité dudit immunogène par rapport à celle de l'immunogène seul, indépendamment du mode d'administration et en absence de toute co-administration d'immunostimulant, **caractérisée en ce que** ledit antigène ou haptène est couplé de façon covalente à ladite molécule support, pour former ledit complexe, **en ce que** cette molécule support est un fragment polypeptidique issu de la protéine G du streptocoque capable de se lier spécifiquement à la sérumalbumine de mammifère, et **en ce que** ledit immunogène est choisi parmi le groupe suivant d'immunogènes dérivés de la glycoprotéine G du virus respiratoire syncytial (VRS) humain ou bovin, sous-groupe A ou B :
- la séquence SEQ ID No. 1, SEQ ID No. 2, SEQ ID No. 51 ou une séquence présentant au moins 80 % d'homologie avec ladite séquence SEQ ID No. 1, SEQ ID No. 2 ou SEQ ID No. 51 ; et
- les séquences SEQ ID No. 3, No. 4 et No. 14 à No. 70.

2. Utilisation selon la revendication 1, **caractérisée en ce que** la molécule support présente la séquence en acides aminés SEQ ID No. 74 ou une séquence présentant au moins 80 % d'homologie avec ladite séquence ID No. 74.

3. Utilisation selon l'une des revendications 1 et 2, **caractérisée en ce que** le couplage covalent est réalisé grâce à la technologie de l'ADN recombinant.

4. Utilisation selon la revendication 3, **caractérisée en ce que** le complexe est produit par insertion ou fusion dans la molécule d'ADN codant pour le support, de l'ADN codant pour l'antigène ou l'haptène.

5. Utilisation selon l'une des revendications 1 et 2, **caractérisée en ce que** ledit couplage covalent est réalisé par voie chimique.

6. Utilisation selon l'une des revendications 1 à 4, **caractérisée en ce que** la préparation dudit complexe comprend une étape dans laquelle on introduit dans une cellule hôte un gène de fusion, ledit gène de fusion comprenant une molécule d'ADN hybride produite par insertion ou fusion dans la molécule d'ADN codant pour la molécule support, de l'ADN codant pour l'antigène ou l'haptène, fusionné avec un promoteur.

7. Utilisation selon la revendication 6, **caractérisée en ce qu'**on introduit le gène de fusion par l'intermédiaire d'un vecteur d'ADN qui provient d'un plasmide, d'un bactériophage, d'un virus et/ou d'un cosmide.

8. Utilisation selon l'une des revendications 6 ou 7, **caractérisée en ce que** le gène de fusion est intégré dans le génome de la cellule hôte.

9. Utilisation selon l'une des revendications 6 à 8, **caractérisée en ce que** la cellule hôte est un procaryote.

10. Utilisation selon la revendication 9, **caractérisée en ce que** la cellule hôte est choisie dans le groupe comprenant : *E. coli, Bacillus, Lactobacillus, Staphylococcus* et *Streptococcus.*

11. Utilisation selon les revendications 6 à 8, **caractérisée en ce que** la cellule hôte est une levure.

12. Utilisation selon les revendications 6 à 8, **caractérisée en ce que** la cellule hôte est une cellule de mammifère.

13. Utilisation selon les revendications 7 et 8, **caractérisée en ce que** l'on utilise un vecteur viral.

14. Utilisation selon l'une des revendications 6 à 11 ou 13, **caractérisée en ce que** la molécule de fusion est exprimée, ancrée et exposée à la membrane des cellules hôtes.

15. Utilisation selon l'une des revendications 1 à 14, **caractérisé en ce que** les protéines dérivées de la glycoprotéine G du sous-groupe A et du sous-groupe B du VRS sont génétiquement fusionnées ou chimiquement couplées à BB.

16. Complexe **caractérisée en ce qu'**il est formé par couplage covalent entre un immunogène, un antigène ou un haptène, est une molécule support, ladite molécule support étant un fragment polypeptidique issu de la protéine G du streptocoque capable de se lier spécifiquement à la sérumalbumine de mammifère, et ledit immunogène étant choisi parmi le groupe suivant d'immunogènes dérivés de la glycoprotéine G du virus respiratoire syncytial (VRS) humain ou bovin, sous-groupe A ou B :
- la séquence SEQ ID No. 1, SEQ ID No. 2, SEQ ID No. 51 ou une séquence présentant au moins 80 % d'homologie avec ladite séquence SEQ ID No. 1, SEQ ID No. 2 ou SEQ ID No. 51 ; et
- les séquences SEQ ID No. 3, No. 4 et No. 14 à No. 70.

17. Complexe selon la revendication 16, **caractérisé en ce que** la molécule support présente la séquence en acides aminés SEQ ID No. 74 ou une séquence présentant au moins 80 % d'homologie avec ladite séquence ID No. 74.

18. Complexe selon la revendication 16 et 17, **caractérisé en ce que** le couplage covalent est réalisé grâce à la technologie de l'ADN recombinant.

19. Complexe selon la revendication 18, **caractérisé en ce que** le complexe est produit par insertion ou fusion dans la molécule d'ADN codant pour le support, de l'ADN codant pour l'antigène ou l'haptène.

20. Complexe selon la revendication 16 et 17, **caractérisé en ce que** ledit couplage covalent est réalisé par voie chimique.

21. Complexe selon l'une des revendications 16 à 19, **caractérisé en ce que** la préparation dudit complexe comprend une étape dans laquelle on introduit dans une cellule hôte un gène de fusion, ledit gène de fusion comprenant une molécule d'ADN hybride produite par insertion ou fusion dans la molécule d'ADN codant pour la molécule support, de l'ADN codant pour l'antigène ou l'haptène, fusionné avec un promoteur.

22. Complexe selon la revendication 21, **caractérisé en ce qu'**on introduit le gène de fusion par l'intermédiaire d'un vecteur d'ADN qui provient d'un plasmide, d'un bactériophage, d'un virus et/ou d'un cosmide.

23. Complexe selon la revendication 21 ou 22, **caractérisé en ce que** le gène de fusion est intégré dans le génome de la cellule hôte.

24. Complexe selon l'une des revendications 21 à 23, **caractérisé en ce que** la cellule hôte est un procaryote.

25. Complexe selon la revendication 24, **caractérisé en ce que** la cellule hôte est choisie dans le groupe comprenant : *E. coli, Bacillus, Lactobacillus, Staphylococcus* et *Streptococcus.*

26. Complexe selon les revendications 21 à 23, **caractérisé en ce que** la cellule hôte est une levure.

27. Complexe selon les revendications 21 à 23, **caractérisé en ce que** la cellule hôte est une cellule de mammifère.

28. Complexe selon les revendications 22 et 23, **caractérisé en ce que** l'on utilise un vecteur viral.

29. Complexe selon les revendications 21 à 26 et 28, **caractérisé en ce que** la molécule de fusion est exprimée, ancrée et exposée à la membrane des cellules hôtes.

30. Complexe selon les revendications 16 à 29, **caractérisé en ce que** les protéines dérivées de la glycoprotéine G du sous-groupe A et du sous-groupe B du VRS sont génétiquement fusionnées ou chimiquement couplées à BB.

31. Séquence nucléotidique codant pour un complexe selon les revendications 16 à 30.

32. Séquence nucléotidique selon la revendication 31, **caractérisée en ce qu'**elle comporte des éléments permettant de cibler l'expression du complexe dans une cellule hôte spécifique.

33. Séquence nucléotidique selon l'une des revendications 31 ou 32, **caractérisée en ce qu'**elle est choisie dans le groupe consistant en les constructions d'ADN et les constructions d'ARN.

34. Séquence selon la revendication 31, **caractérisée en ce qu'**il s'agit d'un gène de fusion permettant la préparation dudit complexe de l'utilisation selon l'une des revendications 3, 4 ou 6 à 14.

35. Vecteur **caractérisé en ce qu'**il comprend une séquence nucléotidique selon l'une des revendications 31 à 34.

36. A titre de médicament complexe selon les revendications 16 à 30, ou vecteur d'ADN selon la revendication 35.

37. Utilisation pour la préparation d'un vaccin d'un complexe entre un immunogène et une molécule support selon les revendications 16 à 30, ou d'une séquence nucléotidique selon l'une des revendications 31 à 34.

## Claims

1. Use of an immunogen, of an antigen or of a hapten, and of a support molecule for preparing a complex which is intended to be administered to a host and to improve the immunogenicity of the said immunogen as compared with that of the immunogen on its own, independently of the mode of administration and in the absence of any coadministration of immunostimulant, **characterized in that** the said antigen or hapten is coupled covalently to the said support molecule in order to form the said complex, **in that** this support molecule is a polypeptide fragment derived from the streptococcal G protein which is able to bind specifically to mammalian serum albumin and **in that** the said immunogen is selected from the following group of immunogens derived from the G glycoprotein of subgroup A or B of the human or bovine respiratory syncytial virus (RSV):
- the sequence SEQ ID No. 1, SEQ ID No. 2 or SEQ ID No. 51 or a sequence exhibiting at least 80% homology with the said sequence SEQ ID No. 1, SEQ ID No. 2 or SEQ ID No. 51; and
- the sequences SEQ ID No. 3, No. 4 and No. 14 to No. 70.

2. Use according to Claim 1, **characterized in that** the support molecule exhibits the amino acid sequence SEQ ID No. 74 or a sequence which exhibits at least 80% homology with the said sequence ID No. 74.

3. Use according to one of Claims 1 and 2, **characterized in that** the covalent coupling is effected by means of recombinant DNA technology.

4. Use according to Claim 3, **characterized in that** the complex is produced by inserting or fusing the DNA encoding the antigen or hapten into the DNA molecule encoding the support.

5. Use according to one of Claims 1 and 2, **characterized in that** the said covalent coupling is effected chemically.

6. Use according to one of Claims 1 to 4, **characterized in that** the preparation of the said complex includes a step in which a fusion gene is introduced into a host cell, the said fusion gene comprising a hybrid DNA molecule which is produced by inserting or fusing the DNA encoding the antigen or hapten into the DNA molecule encoding the support molecule and which is fused with a promoter.

7. Use according to Claim 6, **characterized in that** the fusion gene is introduced with the agency of a DNA vector which is derived from a plasmid, a bacteriophage, a virus and/or a cosmid.

8. Use according to one of Claims 6 or 7, **characterized in that** the fusion gene is integrated into the genome of the host cell.

9. Use according to one of Claims 6 to 8, **characterized in that** the host cell is a prokaryote.

10. Use according to Claim 9, **characterized in that** the host cell is selected from the group comprising: *E*. *coli*, *Bacillus, Lactobacillus*, *Staphylococcus* and *Streptococcus.*

11. Use according to Claims 6 to 8, **characterized in that** the host cell is a yeast.

12. Use according to Claims 6 to 8, **characterized in that** the host cell is a mammalian cell.

13. Use according to Claims 7 and 8, **characterized in that** a viral vector is employed.

14. Use according to one of Claims 6 to 11 or 13, **characterized in that** the fusion molecule is expressed, anchored and exposed at the membrane of the host cells.

15. Use according to one of Claims 1 to 14, **characterized in that** the proteins derived from the G glycoprotein of RSV subgroups A and B are genetically fused or chemically coupled to BB.

16. Complex, **characterized in that** it is formed by covalent coupling between an immunogen, an antigen or a hapten, and a support molecule, the said support molecule being a polypeptide fragment derived from the streptococcal G protein which is able to bind specifically to mammalian serum albumin, and the said immunogen being selected from the following group of immunogens derived from the G glycoprotein of subgroup A or B of the human or bovine respiratory syncytial virus (RSV) :
- the sequence SEQ ID No. 1, SEQ ID No. 2 or SEQ ID No. 51 or a sequence exhibiting at least 80% homology with the said sequence SEQ ID No. 1, SEQ ID No. 2 or SEQ ID No. 51; and
- the sequences SEQ ID No. 3, No. 4 and No. 14 to No. 70.

17. Complex according to Claim 16, **characterized in that** the support molecule exhibits the amino acid sequence SEQ ID No. 74 or a sequence which exhibits at least 80% homology with the said sequence ID No. 74.

18. Complex according to one of Claims 16 and 17, **characterized in that** the covalent coupling is effected by means of recombinant DNA technology.

19. Complex according to Claim 18, **characterized in that** the complex is produced by inserting or fusing the DNA encoding the antigen or hapten into the DNA molecule encoding the support.

20. Complex according to one of Claims 16 and 17, **characterized in that** the said covalent coupling is effected chemically.

21. Complex according to one of Claims 16 to 19, **characterized in that** the preparation of the said complex includes a step in which a fusion gene is introduced into a host cell, the said fusion gene comprising a hybrid DNA molecule which is produced by inserting or fusing the DNA encoding the antigen or hapten into the DNA molecule encoding the support molecule and which is fused with a promoter.

22. Complex according to Claim 21, **characterized in that** the fusion gene is introduced with the agency of a DNA vector which is derived from a plasmid, a bacteriophage, a virus and/or a cosmid.

23. Complex according to Claim 21 or 22, **characterized in that** the fusion gene is integrated into the genome of the host cell.

24. Complex according to one of Claims 21 to 23, **characterized in that** the host cell is a prokaryote.

25. Complex according to Claim 24, **characterized in that** the host cell is selected from the group comprising: *E. coli, Bacillus, Lactobacillus, Staphylococcus* and *Streptococcus.*

26. Complex according to Claims 21 to 23, **characterized in that** the host cell is a yeast.

27. Complex according to Claims 21 to 23, **characterized in that** the host cell is a mammalian cell.

28. Complex according to Claims 22 and 23, **characterized in that** a viral vector is employed.

29. Complex according to one of Claims 21 to 26 and 28, **characterized in that** the fusion molecule is expressed, anchored and exposed at the membrane of the host cells.

30. Complex according to Claims 16 to 29, **characterized in that** the proteins derived from the G glycoprotein of RSV subgroups A and B are genetically fused or chemically coupled to BB.

31. Nucleotide sequence encoding a complex according to Claims 16 to 30.

32. Nucleotide sequence according to Claim 31, **characterized in that** it comprises elements enabling expression of the complex to be targeted in a specific host cell.

33. Nucleotide sequence according to one of Claims 31 or 32, **characterized in that** it is selected from the group consisting of DNA constructs and RNA constructs.

34. Sequence according to Claim 31, **characterized in that** it is a fusion gene enabling the said complex to be prepared by the use according to one of Claims 3, 4 or 6 to 14.

35. Vector, **characterized in that** it comprises a nucleotide sequence according to one of Claims 31 to 34.

36. As a medicament, complex according to Claims 16 to 30, or a DNA vector according to Claim 35.

37. Use, for preparing a vaccine, of a complex between an immunogen and a support molecule according to Claims 16 to 30, or of a nucleotide sequence according to one of Claims 31 to 34.

## Patentansprüche

1. Verwendung eines Immunogens, eines Antigens oder eines Haptens, und eines Trägermoleküls für die Herstellung eines Komplexes, welcher dazu bestimmt ist, an einen Wirt verabreicht zu werden und die Immunogenität des Immunogens in Bezug auf jene des Immunogens allein zu erhöhen unabhängig von der Verabreichungsweise und in Abwesenheit einer jeglichen Koverabreichung von immunstimulierenden Mitteln, **dadurch gekennzeichnet, dass** das Antigen oder Hapten kovalent an das Trägermolekül gekoppelt ist, um den Komplex zu bilden, dass dieses Trägermolekül ein Polypeptidfragment ist, welches von dem Protein G aus Streptokokken abstammt, welches in der Lage ist, sich spezifisch an Serumalbumin aus Säugetieren zu binden, und dass das Immunogen ausgewählt wird unter der folgenden Gruppe von Immunogenen, welche sich von dem Glycoprotein G des Respiratory-Syncytial-Virus (RSV) vom Menschen oder vom Rind, Untergruppe A oder B, ableiten:
- der Sequenz SEQ ID Nr. 1, SEQ ID Nr. 2, SEQ ID Nr. 51 oder einer Sequenz, die wenigstens 80% Homologie zu der genannten Sequenz SEQ ID Nr. 1, SEQ ID Nr. 2 oder SEQ ID Nr. 51 aufweist; und
- den Sequenzen SEQ ID Nr. 3, Nr. 4 und Nr. 14 bis Nr. 70.

2. Verwendung nach Anspruch 1, **dadurch gekennzeichnet, dass** das Trägermolekül die Aminosäuresequenz SEQ ID Nr. 74 oder eine Sequenz, welche wenigstens 80% Homologie zu der genannten Sequenz ID Nr. 74 aufweist, aufweist.

3. Verwendung nach einem der Ansprüche 1 und 2, **dadurch gekennzeichnet, dass** die kovalente Kopplung dank der Technik der in vitro-DNA-Rekombination ausgeführt wird.

4. Verwendung nach Anspruch 3, **dadurch gekennzeichnet, dass** der Komplex durch Insertion oder Fusion der DNA, die das Antigen oder Hapten kodiert, in das bzw. mit dem DNA-Molekül, welches den Träger kodiert, hergestellt wird.

5. Verwendung nach einem der Ansprüche 1 und 2, **dadurch gekennzeichnet, dass** die kovalente Kopplung auf chemischem Wege ausgeführt wird.

6. Verwendung nach einem der Ansprüche 1 bis 4, **dadurch gekennzeichnet, dass** die Herstellung des Komplexes einen Schritt umfasst, in welchem man in eine Wirtszelle ein fusioniertes Gen einschleust, welches fusionierte Gen ein hybrides DNA-Molekül umfasst, welches durch Insertion oder Fusion der DNA, welche das Antigen oder das Hapten kodiert, fusioniert mit einem Promotor, in das bzw. mit dem DNA-Molekül, welches das Trägermolekül kodiert, hergestellt wird.

7. Verwendung nach Anspruch 6, **dadurch gekennzeichnet, dass** man das fusionierte Gen durch das Zwischenglied eines DNA-Vektors, der von einem Plasmid, einem Bakteriophagen, einem Virus und/oder einem Cosmid abstammt, einschleust.

8. Verwendung nach einem der Ansprüche 6 oder 7, **dadurch gekennzeichnet, dass** das fusionierte Gen in das Genom der Wirtszelle integriert wird.

9. Verwendung nach einem der Ansprüche 6 bis 8, **dadurch gekennzeichnet, dass** die Wirtszelle ein Prokaryot ist.

10. Verwendung nach Anspruch 9, **dadurch gekennzeichnet, dass** die Wirtszelle in der Gruppe, umfassend: *E. coli, Bacillus, Lactobacillus, Staphylococcus* und *Streptococcus*, ausgewählt wird.

11. Verwendung nach den Ansprüchen 6 bis 8, **dadurch gekennzeichnet, dass** die Wirtszelle eine Hefe ist.

12. Verwendung nach den Ansprüchen 6 bis 8, **dadurch gekennzeichnet, dass** die Wirtszelle eine Säugetierzelle ist.

13. Verwendung nach den Ansprüchen 7 und 8, **dadurch gekennzeichnet, dass** man einen viralen Vektor einsetzt.

14. Verwendung nach einem der Ansprüche 6 bis 11 oder 13, **dadurch gekennzeichnet, dass** das fusionierte Molekül exprimiert, auf der Membran der Wirtszellen verankert und exponiert wird.

15. Verwendung nach einem der Ansprüche 1 bis 14, **dadurch gekennzeichnet, dass** die von dem Glycoprotein G der Untergruppe A und der Untergruppe B von RSV abgeleiteten Proteine genetisch mit BB fusioniert oder chemisch an BB gekoppelt sind.

16. Komplex, **gekennzeichnet dadurch, dass** er durch kovalente Kopplung zwischen einem Immunogen, einem Antigen oder einem Hapten, und einem Trägermolekül gebildet wird, wobei das Trägermolekül ein Polypeptidfragment ist, welches von dem Protein G aus Streptokokken abstammt, welches in der Lage ist, sich spezifisch an Serumalbumin aus Säugetieren zu binden, und wobei das Immunogen ausgewählt wird unter der folgenden Gruppe von Immunogenen, welche sich von dem Glycoprotein G des Respiratory-Syncytial-Virus (RSV) vom Menschen oder vom Rind, Untergruppe A oder B, ableiten:
- der Sequenz SEQ ID Nr. 1, SEQ ID Nr. 2, SEQ ID Nr. 51 oder einer Sequenz, die wenigstens 80% Homologie zu der genannten Sequenz SEQ ID Nr. 1, SEQ ID Nr. 2 oder SEQ ID Nr. 51 aufweist; und
- den Sequenzen SEQ ID Nr. 3, Nr. 4 und Nr. 14 bis Nr. 70.

17. Komplex nach Anspruch 16, **dadurch gekennzeichnet, dass** das Trägermolekül die Aminosäuresequenz SEQ ID Nr. 74 oder eine Sequenz, welche wenigstens 80% Homologie zu der genannten Sequenz ID Nr. 74 aufweist, aufweist.

18. Komplex nach Anspruch 16 und 17, **dadurch gekennzeichnet, dass** die kovalente Kopplung dank der Technik der in vitro-DNA-Rekombination ausgeführt wird.

19. Komplex nach Anspruch 18, **dadurch gekennzeichnet, dass** der Komplex durch Insertion oder Fusion der DNA, die das Antigen oder Hapten kodiert, in das bzw. mit dem DNA-Molekül, welches den Träger kodiert, hergestellt wird.

20. Komplex nach Anspruch 16 und 17, **dadurch gekennzeichnet, dass** die kovalente Kopplung auf chemischem Wege ausgeführt wird.

21. Komplex nach einem der Ansprüche 16 bis 19, **dadurch gekennzeichnet, dass** die Herstellung des Komplexes einen Schritt umfasst, in welchem man in eine Wirtszelle ein fusioniertes Gen einschleust, welches fusionierte Gen ein hybrides DNA-Molekül umfasst, welches durch Insertion oder Fusion der DNA, welche das Antigen oder das Hapten kodiert, fusioniert mit einem Promotor, in das bzw. mit dem DNA-Molekül, welches das Trägermolekül kodiert, hergestellt wird.

22. Komplex nach Anspruch 21, **dadurch gekennzeichnet, dass** man das fusionierte Gen durch das Zwischenglied eines DNA-Vektors, der von einem Plasmid, einem Bakteriophagen, einem Virus und/oder einem Cosmid abstammt, einschleust.

23. Komplex nach Anspruch 21 oder 22, **dadurch gekennzeichnet, dass** das fusionierte Gen in das Genom der Wirtszelle integriert wird.

24. Komplex nach einem der Ansprüche 21 bis 23, **dadurch gekennzeichnet, dass** die Wirtszelle ein Prokaryot ist.

25. Komplex nach Anspruch 24, **dadurch gekennzeichnet, dass** die Wirtszelle in der Gruppe, umfassend: *E. coli, Bacillus, Lactobacillus, Staphylococcus* und *Streptococcus,* ausgewählt wird.

26. Komplex nach den Ansprüchen 21 bis 23, **dadurch gekennzeichnet, dass** die Wirtszelle eine Hefe ist.

27. Komplex nach den Ansprüchen 21 bis 23, **dadurch gekennzeichnet, dass** die Wirtszelle eine Säugetierzelle ist.

28. Komplex nach den Ansprüchen 22 und 23, **dadurch gekennzeichnet, dass** man einen viralen Vektor einsetzt.

29. Komplex nach den Ansprüchen 21 bis 26 und 28, **dadurch gekennzeichnet, dass** das fusionierte Molekül exprimiert, auf der Membran der Wirtszellen verankert und exponiert wird.

30. Komplex nach den Ansprüchen 16 bis 29, **dadurch gekennzeichnet, dass** die von dem Glycoprotein G der Untergruppe A und der Untergruppe B von RSV abgeleiteten Proteine genetisch mit BB fusioniert oder chemisch an BB gekoppelt sind.

31. Nukleotidsequenz, welche einen Komplex nach den Ansprüchen 16 bis 30 kodiert.

32. Nukleotidsequenz nach Anspruch 31, **dadurch gekennzeichnet, dass** sie Elemente umfasst, welche es ermöglichen, die Expression des Komplexes zielgerichtet in einer speziellen Wirtszelle zu realisieren.

33. Nukleotidsequenz nach einem der Ansprüche 31 oder 32, **dadurch gekennzeichnet, dass** sie in der Gruppe bestehend aus den DNA-Konstrukten und den RNA-Konstrukten ausgewählt wird.

34. Sequenz nach Anspruch 31, **dadurch gekennzeichnet, dass** es sich um ein fusioniertes Gen handelt, welches die Herstellung des Komplexes aus der Verwendung nach einem der Ansprüche 3, 4 oder 6 bis 14 ermöglicht.

35. Vektor, **gekennzeichnet dadurch, dass** er eine Nukleotidsequenz nach einem der Ansprüche 31 bis 34 umfasst.

36. Komplex nach den Ansprüchen 16 bis 30 oder DNA-Vektor nach Anspruch 35 als Arzneimittel.

37. Verwendung eines Komplexes zwischen einem Immunogen und einem Trägermolekül nach den Ansprüchen 16 bis 30 oder einer Nukleotidsequenz nach einem der Ansprüche 31 bis 34 für die Herstellung eines Impfstoffs.


### LISTE DE SEQUENCES

(1) INFORMATIONS GENERALES:
   (i) DEPOSANT:
      (A) NOM: PIERRE FABRE MEDICAMENT
      (B) RUE: 45 PLACE ABEL GANCE
      (C) VILLE: BOULOGNE
      (E) PAYS: FRANCE
      (F) CODE POSTAL: 92100
   (ii) TITRE DE L' INVENTION: PROCEDE POUR AMELIORER L'IMMUNOGENICITE D'UN COMPOSÉ IMMUNOGENE OU D'UN HAPTENE ET APPLICATION A LA PREPARATION DE VACCINS
   (iii) NOMBRE DE SEQUENCES: 78
   (iv) FORME DECHIFFRABLE PAR ORDINATEUR:
      (A) TYPE DE SUPPORT: Floppy disk
      (B) ORDINATEUR: Apple Macintosh
      (C) SYSTEME D' EXPLOITATION: MAC OS Systeme 7
      (D) LOGICIEL: PatentIn Release #1.0, Version #1.30 (OEB)
   (v) DONNEES DE LA DEMANDE ANTERIEURE:
      (A) NUMERO DE LA DEMANDE: FR 9413310
      (B) DATE DE DEPOT: 07-NOV-1994
(2) INFORMATIONS POUR La SEQ ID NO: 1:
   (i) CARACTERISTIQUES DE LA SEQUENCE:
      (A) LONGUEUR: 303 paires de bases
      (B) TYPE: nucléotide
      (C) NOMBRE DE BRINS: simple
      (D) CONFIGURATION: linéaire
   (ii) TYPE DE MOLECULE: ADN
   (ix) CARACTERISTIQUE:
      (A) NOM/CLE: CDS
      (B) EMPLACEMENT:1..303
   (xi) DESCRIPTION DE LA SEQUENCE: SEQ ID NO: 1:
   ACC GTG AAA ACC AAA AAC ACC ACG ACC ACC CAG ACC CAG CCG AGC AAA       48
   Thr Val Lys Thr Lys Asn Thr Thr Thr Thr Gln Thr Gln Pro Ser Lys
     1               5                  10                  15
   CCG ACC ACC AAA CAG CGT CAG AAC AAA CCG CCG AAC AAA CCG AAC AAC      96
   Pro Thr Thr Lys Gln Arg Gln Asn Lys Pro Pro Asn Lys Pro Asn Asn
                   20                  25                  30
   GAT TTC CAT TTC GAA GTG TTC AAC TTC GTG CCG TGC AGC ATC TGC AGC     144
   Asp Phe His Phe Glu Val Phe Asn Phe Val Pro Cys Ser Ile Cys Ser
               35                  40                  45
   AAC AAC CCG ACC TGC TGG GCG ATC TGC AAA CGT ATC CCG AAC AAA AAA     192
   Asn Asn Pro Thr Cys Trp Ala Ile Cys Lys Arg Ile Pro Asn Lys Lys
       50                  55                  60
   CCG GGC AAA AAA ACC ACG ACC AAA CCG ACC AAA AAA CCG ACC TTC AAA     240
   Pro Gly Lys Lys Thr Thr Thr Lys Pro Thr Lys Lys Pro Thr Phe Lys
   65                  70                  75                  80
   ACC ACC AAA AAA GAT CAT AAA CCG CAG ACC ACC AAA CCG AAA GAA GTG     288
   Thr Thr Lys Lys Asp His Lys Pro Gln Thr Thr Lys Pro Lys Glu Val
                   85                  90                  95
   CCG ACC ACC AAA CCG                                                 303
   Pro Thr Thr Lys Pro
               100
(2) INFORMATIONS POUR LA SEQ ID NO: 2:
   (i) CARACTERISTIQUES DE LA SEQUENCE:
      (A) LONGUEUR: 303 poires de bases
      (B) TYPE: nucléotide
      (C) NOMBRE DE BRINS: simple
      (D) CONFIGURATION: linéaire
   (ii) TYPE DE MOLECULE: ADN
   (ix) CARACTERISTIQUE:
      (A) NOM/CLE: CDS
      (B) EMPLACEMENT:1..303
   (xi) DESCRIPTION DE LA SEQUENCE: SEQ ID NO: 2:
   ACC GCG CAG ACC AAA GGC CGT ATC ACC ACC AGC ACC CAG ACC AAC AAA      48
   Thr Ala Gln Thr Lys Gly Arg Ile Thr Thr Ser Thr Gln Thr Asn Lys
    1               5                  10                  15
   CCG AGC ACC AAA AGC CGT AGC AAA AAC CCG CCG AAA AAA CCG AAA GAT        96
   Pro Ser Thr Lys Ser Arg Ser Lys Asn Pro Pro Lys Lys Pro Lys Asp
               20                  25                  30
   GAT TAC CAC TTC GAA GTG TTC AAC TTC GTG CCC TGC AGC ATC TGC GGC       144
   Asp Tyr His Phe Glu Val Phe Asn Phe Val Pro Cys Ser Ile Cys Gly
           35                  40                  45
   AAC AAC CAG CTG TGC AAA AGC ATC TGC AAA ACC ATC CCG AGC AAC AAA       192
   Asn Asn Gln Leu Cys Lys Ser Ile Cys Lys Thr Ile Pro Ser Asn Lys
       50                  55                  60
   CCG AAA AAG AAA CCG ACC ATC AAA CCG ACC AAC AAA CCG ACC ACC AAA       240
   Pro Lys Lys Lys Pro Thr Ile Lys Pro Thr Asn Lys Pro Thr Thr Lys
   65                  70                  75                  80
   ACC ACC AAC AAA CGT GAT CCG AAA ACC CCG GCG AAA ATG CCG AAG AAG       288
   Thr Thr Asn Lys Arg Asp Pro Lys Thr Pro Ala Lys Met Pro Lys Lys
                   85                  90                  95
   GAA ATC ATC ACC AAC                                                   303
   Glu Ile Ile Thr Asn
               100
(2) INFORMATIONS POUR LA SEQ ID NO: 3:
   (i) CARACTERISTIQUES DE LA SEQUENCE:
      (A) LONGUEUR: 303 poires de bases
      (B) TYPE: nucléotide
      (C) NOMBRE DE BRINS: simple
      (D) CONFIGURATION: linéaire
   (ii) TYPE DE MOLECULE: ADN
   (ix) CARACTERISTIQUE:
      (A) NOM/CLE: CDS
      (B) EMPLACEMENT:1..303
   (xi) DESCRIPTION DE LA SEQUENCE: SEQ ID NO: 3:
   ACC GTG AAA ACC AAA AAC ACC ACG ACC ACC CAG ACC CAG CCG AGC AAA        48
   Thr Val Lys Thr Lys Asn Thr Thr Thr Thr Gln Thr Gln Pro Ser Lys
   1               5                   10                  15
   CCG ACC ACC AAA CAG CGT CAG AAC AAA CCG CCG AAC AAA CCG AAC AAC        96
   Pro Thr Thr Lys Gln Arg Gln Asn Lys Pro Pro Asn Lys Pro Asn Asn
               20                  25                  30
   GAT TTC CAT TTC GAA GTG TTC AAC TTC GTG CCG AGC AGC ATC TGC AGC      144
   Asp Phe His Phe Glu Val Phe Asn Phe Val Pro Ser Ser Ile Cys Ser
            35                  40                  45
   AAC AAC CCG ACC TGC TGG GCG ATC AGC AAA CGT ATC CCG AAC AAA AAA      192
   Asn Asn Pro Thr Cys Trp Ala Ile Ser Lys Arg Ile Pro Asn Lys Lys
        50                  55                  60
   CCG GGC AAA AAA ACC ACG ACC AAA CCG ACC AAA AAA CCG ACC TTC AAA      240
   Pro Gly Lys Lys Thr Thr Thr Lys Pro Thr Lys Lys Pro Thr Phe Lys
   65                  70                  75                  80
   ACC ACC AAA AAA GAT CAT AAA CCG CAG ACC ACC AAA CCG AAA GAA GTG      288
   Thr Thr Lys Lys Asp His Lys Pro Gln Thr Thr Lys Pro Lys Glu Val
                   85                  90                  95
   CCG ACC ACC AAA CCG                                                  303
   Pro Thr Thr Lys Pro
               100
(2) INFORMATIONS POUR LA SEQ ID NO: 4:
   (i) CARACTERISTIQUES DE LA SEQUENCE:
      (A) LONGUEUR: 303 paires de bases
      (B) TYPE: nucléotide
      (C) NOMBRE DE BRINS: simple
      (D) CONFIGURATION: linéaire
   (ii) TYPE DE MOLECULE: ADN
   (ix) CARACTERISTIQUE:
      (A) NOM/CLE: CDS
      (B) EMPLACEMENT:1..303
   (xi) DESCRIPTION DE LA SEQUENCE: SEQ ID NO: 4:
   ACC GCG CAG ACC AAA GGC CGT ATC ACC ACC AGC ACC CAG ACC AAC AAA      48
   Thr Ala Gln Thr Lys Gly Arg Ile Thr Thr Ser Thr Gln Thr Asn Lys
    1               5                  10                  15
   CCG AGC ACC AAA AGC CGT AGC AAA AAC CCG CCG AAA AAA CCG AAA GAT      96
   Pro Ser Thr Lys Ser Arg Ser Lys Asn Pro Pro Lys Lys Pro Lys Asp
                20                  25                  30
   GAT TAC CAC TTC GAA GTG TTC AAC TTC GTG CCC AGC AGC ATC TGC GGC      144
   Asp Tyr His Phe Glu Val Phe Asn Phe Val Pro Ser Ser Ile Cys Gly
            35                  40                  45
   AAC AAC CAG CTG TGC AAA AGC ATC AGC AAA ACC ATC CCG AGC AAC AAA      192
   Asn Asn Gln Leu Cys Lys Ser Ile Ser Lys Thr Ile Pro Ser Asn Lys
        50                  55                  60
   CCG AAA AAG AAA CCG ACC ATC AAA CCG ACC AAC AAA CCG ACC ACC AAA      240
   Pro Lys Lys Lys Pro Thr Ile Lys Pro Thr Asn Lys Pro Thr Thr Lys
    65                  70                  75                  80
   ACC ACC AAC AAA CGT GAT CCG AAA ACC CCG GCG AAA ATG CCG AAG AAG      288
   Thr Thr Asn Lys Arg Asp Pro Lys Thr Pro Ala Lys Met Pro Lys Lys
                    85                  90                  95
   GAA ATC ATC ACC AAC                                                  303
   Glu Ile Ile Thr Asn
               100
(2) INFORMATIONS POUR LA SEQ ID NO: 5:
   (i) CARACTERISTIQUES DE LA SEQUENCE:
      (A) LONGUEUR: 42 paires de bases
      (B) TYPE: nucléotide
      (C) NOMBRE DE BRINS: simple
      (D) CONFIGURATION: linéaire
   (ii) TYPE DE MOLECULE: ADN
   (ix) CARACTERISTIQUE:
      (A) NOM/CLE: CDS
      (B) EMPLACEMENT:1..42
   (xi) DESCRIPTION DE LA SEQUENCE: SEQ ID NO: 5:
   AGC ATC TGC AGC AAC AAC CCG ACC TGC TGG GCG ATC TGC AAA      42
   Ser Ile Cys Ser Asn Asn Pro Thr Cys Trp Ala Ile Cys Lys
    1               5                  10
(2) INFORMATIONS POUR LA SEQ ID NO: 6:
   (i) CARACTERISTIQUES DE LA SEQUENCE:
      (A) LONGUEUR: 42 paires de bases
      (B) TYPE: nucléotide
      (C) NOMBRE DE BRINS: simple
      (D) CONFIGURATION: linéaire
   (ii) TYPE DE MOLECULE: ADNc
   (ix) CARACTERISTIQUE:
      (A) NOM/CLE: CDS
      (B) EMPLACEMENT:1..42
   (xi) DESCRIPTION DE LA SEQUENCE: SEQ ID NO: 6:
   AGC ATC TGC GGC AAC AAC CAG CTG TGC AAA AGC ATC TGC AAA        42
   Ser Ile Cys Gly Asn Asn Gln Leu Cys Lys Ser Ile Cys Lys
    1               5                  10
(2) INFORMATIONS POUR LA SEQ ID NO: 7:
   (i) CARACTERISTIQUES DE LA SEQUENCE:
      (A) LONGUEUR: 42 poires de bases
      (B) TYPE: nucléotide
      (C) NOMBRE DE BRINS: simple
      (D) CONFIGURATION: linéaire
   (ii) TYPE DE MOLECULE: ADN
   (ix) CARACTERISTIQUE:
      (A) NOM/CLE: CDS
      (B) EMPLACEMENT:1..42
   (xi) DESCRIPTION DE LA SEQUENCE: SEQ ID NO: 7:
   AGC ATC TGC AGC AAC AAC CCG ACC TGC TGG GCG ATC AGC AAA        42
   Ser Ile Cys Ser Asn Asn Pro Thr Cys Trp Ala Ile Ser Lys
    1               5                  10
(2) INFORMATIONS POUR LA SEQ ID NO: 8:
   (i) CARACTERISTIQUES DE LA SEQUENCE:
      (A) LONGUEUR: 42 paires de bases
      (B) TYPE: nucléotide
      (C) NOMBRE DE BRINS: simple
      (D) CONFIGURATION: linéaire
   (ii) TYPE DE MOLECULE: ADN
   (ix) CARACTERISTIQUE:
      (A) NOM/CLE: CDS
      (B) EMPLACEMENT:1..42
   (xi) DESCRIPTION DE LA SEQUENCE: SEQ ID NO: 8:
   AGC ATC TGC GGC AAC AAC CAG CTG TGC AAA AGC ATC AGC AAA 42
   Ser Ile Cys Gly Asn Asn Gln Leu Cys Lys Ser Ile Ser Lys
   1 5 10
(2) INFORMATIONS POUR LA SEQ ID NO: 9:
   (i) CARACTERISTIQUES DE LA SEQUENCE:
      (A) LONGUEUR: 14 acides aminés
      (B) TYPE: acide aminé
      (C) NOMBRE DE BRINS: simple
      (D) CONFIGURATION: linéaire
   (ii) TYPE DE MOLECULE: peptide
   (ix) CARACTERISTIQUE:
      (A) NOM/CLE: Modified-site
      (B) EMPLACEMENT:9
      (D) AUTRES INFORMATIONS:/Xaa signifie Orn
   (xi) DESCRIPTION DE LA SEQUENCE: SEQ ID NO: 9:
   Ser Ile Asp Ser Asn Asn Pro Thr Xaa Trp Ala Ile Cys Lys
   1 5 10
(2) INFORMATIONS POUR LA SEQ ID NO: 10:
   (i) CARACTERISTIQUES DE LA SEQUENCE:
      (A) LONGUEUR: 14 acides aminés
      (B) TYPE: acide aminé
      (C) NOMBRE DE BRINS: simple
      (D) CONFIGURATION: linéaire
   (ii) TYPE DE MOLECULE: peptide
   (ix) CARACTERISTIQUE:
      (A) NOM/CLE: Modified-site
      (B) EMPLACEMENT:9
      (D) AUTRES INFORMATIONS:/Xaa signifie Orn
   (xi) DESCRIPTION DE LA SEQUENCE: SEQ ID NO: 10:
   Ser Ile Asp Gly Asn Asn Gln Leu Xaa Lys Ser Ile Cys Lys
   1 5 10
(2) INFORMATIONS POUR LA SEQ ID NO: 11:
   (i) CARACTERISTIQUES DE LA SEQUENCE:
      (A) LONGUEUR: 14 acides aminés
      (B) TYPE: acide aminé
      (C) NOMBRE DE BRINS: simple
      (D) CONFIGURATION: linéaire
   (ii) TYPE DE MOLECULE: peptide
   (ix) CARACTERISTIQUE:
      (A) NOM/CLE: Modified-site
      (B) EMPLACEMENT:9
      (D) AUTRES INFORMATIONS:/Xaa signifie Orn
   (xi) DESCRIPTION DE LA SEQUENCE: SEQ ID NO: 11:
   Ser Ile Asp Ser Asn Asn Pro Thr Xaa Trp Ala Ile Ser Lys
   1               5                   10
(2) INFORMATIONS POUR LA SEQ ID NO: 12:
   (i) CARACTERISTIQUES DE LA SEQUENCE:
      (A) LONGUEUR: 14 acides aminés
      (B) TYPE: acide aminé
      (C) NOMBRE DE BRINS: simple
      (D) CONFIGURATION: linéaire
   (ii) TYPE DE MOLECULE: peptide
   (ix) CARACTERISTIQUE:
      (A) NOM/CLE: Modified-site
      (B) EMPLACEMENT:9
      (D) AUTRES INFORMATIONS:/Xaa signifie Orn
   (xi) DESCRIPTION DE LA SEQUENCE: SEQ ID NO: 12:
   Ser Ile Asp Gly Asn Asn Gln Leu Xaa Lys Ser Ile Ser Lys
   1               5                   10
(2) INFORMATIONS POUR LA SEQ ID NO: 13:
   (i) CARACTERISTIQUES DE LA SEQUENCE:
      (A) LONGUEUR: 48 paires de bases
      (B) TYPE: nucléotide
      (C) NOMBRE DE BRINS: simple
      (D) CONFIGURATION: linéaire
   (ii) TYPE DE MOLECULE: ADN
   (ix) CARACTERISTIQUE:
      (A) NOM/CLE: CDS
      (B) EMPLACEMENT:1..48
   (xi) DESCRIPTION DE LA SEQUENCE: SEQ ID NO: 13:
   AGC AAA CCG ACC ACC AAA CAG CGT CAG AAC AAA CCG CCG AAC AAA CCG        48
   Ser Lys Pro Thr Thr Lys Gln Arg Gln Asn Lys Pro Pro Asn Lys Pro
    1               5                  10                  15
(2) INFORMATIONS POUR LA SEQ ID NO: 14:
   (i) CARACTERISTIQUES DE LA SEQUENCE:
      (A) LONGUEUR: 303 paires de base
      (B) TYPE: nucléotide
      (C) NOMBRE DE BRINS: simple
      (D) CONFIGURATION: linéaire
   (ii) TYPE DE MOLECULE: ADN
   (ix) CARACTERISTIQUE:
      (A) NOM/CLE: CDS
      (B) EMPLACEMENT:1..303
   (xi) DESCRIPTION DE LA SEQUENCE: SEQ ID NO: 14:
   ACC GTG AAA ACC AAA AAC ACC ACG ACC ACC CAG ACC CAG CCG AGC AAA     48
   Thr Val Lys Thr Lys Asn Thr Thr Thr Thr Gln Thr Gln Pro Ser Lys
    1               5                  10                  15
   CCG ACC ACC AAA CAG CGT CAG AAC AAA CCG CCG AAC AAA CCG AAC AAC     96
   Pro Thr Thr Lys Gln Arg Gln Asn Lys Pro Pro Asn Lys Pro Asn Asn
               20                  25                  30
   GAT TCC CAT TCC GAA GTG TCC AAC TCC GTG CCG AGC AGC ATC TGC AGC    144
   Asp Ser His Ser Glu Val Ser Asn Ser Val Pro Ser Ser Ile Cys Ser
           35                  40                  45
   AAC AAC CCG ACC TGC TGG GCG ATC AGC AAA CGT ATC CCG AAC AAA AAA    192
   Asn Asn Pro Thr Cys Trp Ala Ile Ser Lys Arg Ile Pro Asn Lys Lys
       50                  55                  60
   CCG GGC AAA AAA ACC ACG ACC AAA CCG ACC AAA AAA CCG ACC TTC AAA    240
   Pro Gly Lys Lys Thr Thr Thr Lys Pro Thr Lys Lys Pro Thr Phe Lys
   65                  70                  75                  80
   ACC ACC AAA AAA GAT CAT AAA CCG CAG ACC ACC AAA CCG AAA GAA GTG    288
   Thr Thr Lys Lys Asp His Lys Pro Gln Thr Thr Lys Pro Lys Glu Val
                   85                  90                  95
   CCG ACC ACC AAA CCG                                                303
   Pro Thr Thr Lys Pro
               100
(2) INFORMATIONS POUR LA SEQ ID NO: 15:
   (i) CARACTERISTIQUES DE LA SEQUENCE:
      (A) LONGUEUR: 51 paires de bases
      (B) TYPE: nucléotide
      (C) NOMBRE DE BRINS: simple
      (D) CONFIGURATION: linéaire
   (ii) TYPE DE MOLECULE: ADN
   (ix) CARACTERISTIQUE:
      (A) NOM/CLE: CDS
      (B) EMPLACEMENT:1..51
   (xi) DESCRIPTION DE LA SEQUENCE: SEQ ID NO: 15:
   GTG CCG TGC AGC ATC TGC AGC AAC AAC CCG ACC TGC TGG GCG ATC TGC        48
   Val Pro Cys Ser Ile Cys Ser Asn Asn Pro Thr Cys Trp Ala Ile Cys
     1               5                  10                  15
   AAA                                                                    51
   Lys
(2) INFORMATIONS POUR LA SEQ ID NO: 16:
   (i) CARACTERISTIQUES DE LA SEQUENCE:
      (A) LONGUEUR: 51 paires de bases
      (B) TYPE: nucléotide
      (C) NOMBRE DE BRINS: simple
      (D) CONFIGURATION: linéaire
   (ii) TYPE DE MOLECULE: ADN
   (ix) CARACTERISTIQUE:
      (A) NOM/CLE: CDS
      (B) EMPLACEMENT:1..51
   (xi) DESCRIPTION DE LA SEQUENCE: SEQ ID NO: 16:
   GTG CCG AGC AGC ATC TGC AGC AAC AAC CCG ACC TGC TGG GCG ATC AGC        48
   Val Pro Ser Ser Ile Cys Ser Asn Asn Pro Thr Cys Trp Ala Ile Ser
     1               5                  10                  15
   AAA                                                                    51
   Lys
(2) INFORMATIONS POUR LA SEQ ID NO: 17:
   (i) CARACTERISTIQUES DE LA SEQUENCE:
      (A) LONGUEUR: 51 paires de bases
      (B) TYPE: nucléotide
      (C) NOMBRE DE BRINS: simple
      (D) CONFIGURATION: linéaire
   (ii) TYPE DE MOLECULE: ADN
   (ix) CARACTERISTIQUE:
      (A) NOM/CLE: CDS
      (B) EMPLACEMENT:1..51
   (xi) DESCRIPTION DE LA SEQUENCE: SEQ ID NO: 17:
   GTG CCC TGC AGC ATC TGC GGC AAC AAC CAG CTG TGC AAA AGC ATC TGC      48
   Val Pro Cys Ser Ile Cys Gly Asn Asn Gln Leu Cys Lys Ser Ile Cys
    1               5                  10                  15
   AAA                                                                  51
   Lys
(2) INFORMATIONS POUR LA SEQ ID NO: 18:
   (i) CARACTERISTIQUES DE LA SEQUENCE:
      (A) LONGUEUR: 51 paires de bases
      (B) TYPE: nucléotide
      (C) NOMBRE DE BRINS: simple
      (D) CONFIGURATION: linéaire
   (ii) TYPE DE MOLECULE: ADN
   (ix) CARACTERISTIQUE:
      (A) NOM/CLE: CDS
      (B) EMPLACEMENT:1..51
   (xi) DESCRIPTION DE LA SEQUENCE: SEQ ID NO: 18:
   GTG CCC AGC AGC ATC TGC GGC AAC AAC CAG CTG TGC AAA AGC ATC AGC      48
   Val Pro Ser Ser Ile Cys Gly Asn Asn Gln Leu Cys Lys Ser Ile Ser
    1               5                  10                  15
   AAA                                                                  51
   Lys
(2) INFORMATIONS POUR LA SEQ ID NO: 19:
   (i) CARACTERISTIQUES DE LA SEQUENCE:
      (A) LONGUEUR: 17 acides aminés
      (B) TYPE: acide aminé
      (C) NOMBRE DE BRINS: simple
      (D) CONFIGURATION: linéaire
   (ii) TYPE DE MOLECULE: peptide
   (ix) CARACTERISTIQUE:
      (A) NOM/CLE: Modified-site
      (B) EMPLACEMENT:12
      (D) AUTRES INFORMATIONS:/Xaa signifie Orn
   (ix) CARACTERISTIQUE:
      (A) NOM/CLE: Modified-site
      (B) EMPLACEMENT:16
      (D) AUTRES INFORMATIONS:/Xaa signifie Orn
   (xi) DESCRIPTION DE LA SEQUENCE: SEQ ID NO: 19:
   Val Pro Asp Ser Ile Asp Ser Asn Asn Pro Thr Xaa Trp Ala Ile Xaa
   1               5                   10                  15
   Lys
(2) INFORMATIONS POUR LA SEQ ID NO: 20:
   (i) CARACTERISTIQUES DE LA SEQUENCE:
      (A) LONGUEUR: 17 acides aminés
      (B) TYPE: acide aminé
      (C) NOMBRE DE BRINS: simple
      (D) CONFIGURATION: linéaire
   (ii) TYPE DE MOLECULE: peptide
   (ix) CARACTERISTIQUE:
      (A) NOM/CLE: Modified-site
      (B) EMPLACEMENT:12
      (D) AUTRES INFORMATIONS:/Xaa signifie Orn
   (xi) DESCRIPTION DE LA SEQUENCE: SEQ ID NO: 20:
   Val Pro Ser Ser Ile Asp Ser Asn Asn Pro Thr Xaa Trp Ala Ile Ser
   1               5                   10                  15
   Lys
(2) INFORMATIONS POUR LA SEQ ID NO: 21:
   (i) CARACTERISTIQUES DE LA SEQUENCE:
      (A) LONGUEUR: 17 acides aminés
      (B) TYPE: acide aminé
      (C) NOMBRE DE BRINS: simple
      (D) CONFIGURATION: linéaire
   (ii) TYPE DE MOLECULE: peptide
   (ix) CARACTERISTIQUE:
      (A) NOM/CLE: Modified-site
      (B) EMPLACEMENT:12
      (D) AUTRES INFORMATIONS:/Xaa signifie Orn
   (ix) CARACTERISTIQUE:
      (A) NOM/CLE: Modified-site
      (B) EMPLACEMENT:16
      (D) AUTRES INFORMATIONS:/Xaa signifie Orn
   (xi) DESCRIPTION DE LA SEQUENCE: SEQ ID NO: 21:
   Val Pro Asp Ser Ile Asp Gly Asn Asn Gln Leu Xaa Lys Ser Ile Xaa
   1               5                   10                  15
   Lys
(2) INFORMATIONS POUR LA SEQ ID NO: 22:
   (i) CARACTERISTIQUES DE LA SEQUENCE:
      (A) LONGUEUR: 17 acides aminés
      (B) TYPE: acide aminé
      (C) NOMBRE DE BRINS: simple
      (D) CONFIGURATION: linéaire
   (ii) TYPE DE MOLECULE: peptide
   (ix) CARACTERISTIQUE:
      (A) NOM/CLE: Modified-site
      (B) EMPLACEMENT:12
      (D) AUTRES INFORMATIONS:/Xaa signifie Orn
   (xi) DESCRIPTION DE LA SEQUENCE: SEQ ID NO: 22:
   Val Pro Ser Ser Ile Asp Gly Asn Asn Gln Leu Xaa Lys Ser Ile Ser
   1               5                   10                  15
   Lys
(2) INFORMATIONS POUR LA SEQ ID NO: 23:
   (i) CARACTERISTIQUES DE LA SEQUENCE:
      (A) LONGUEUR: 183 paires de bases
      (B) TYPE: nucléotide
      (C) NOMBRE DE BRINS: simple
      (D) CONFIGURATION: linéaire
   (ii) TYPE DE MOLECULE: ADN
   (ix) CARACTERISTIQUE:
      (A) NOM/CLE: CDS
      (B) EMPLACEMENT:1..183
   (xi) DESCRIPTION DE LA SEQUENCE: SEQ ID NO: 23:
   CAG ACC CAG CCG AGC AAA CCG ACC ACC AAA CAG CGT CAG AAC AAA CCG      48
   Gln Thr Gln Pro Ser Lys Pro Thr Thr Lys Gln Arg Gln Asn Lys Pro
     1               5                  10                  15
   CCG AAC AAA CCG AAC AAC GAT TTC CAT TTC GAA GTG TTC AAC TTC GTG      96
   Pro Asn Lys Pro Asn Asn Asp Phe His Phe Glu Val Phe Asn Phe Val
               20                  25                  30
   CCG TGC AGC ATC TGC AGC AAC AAC CCG ACC TGC TGG GCG ATC TGC AAA     144
   Pro Cys Ser Ile Cys Ser Asn Asn Pro Thr Cys Trp Ala Ile Cys Lys
           35                  40                  45
   CGT ATC CCG AAC AAA AAA CCG GGC AAA AAA ACC ACG ACC                 183
   Arg Ile Pro Asn Lys Lys Pro Gly Lys Lys Thr Thr Thr
       50                  55                  60
(2) INFORMATIONS POUR LA SEQ ID NO: 24:
   (i) CARACTERISTIQUES DE LA SEQUENCE:
      (A) LONGUEUR: 177 paires de bases
      (B) TYPE: nucléotide
      (C) NOMBRE DE BRINS: simple
      (D) CONFIGURATION: linéaire
   (ii) TYPE DE MOLECULE: ADN
   (ix) CARACTERISTIQUE:
      (A) NOM/CLE: CDS
      (B) EMPLACEMENT:1..177
   (xi) DESCRIPTION DE LA SEQUENCE: SEQ ID NO: 24:
   CAG ACC CAG CCG AGC AAA CCG ACC ACC AAA CAG CGT CAG AAC AAA CCG      48
   Gln Thr Gln Pro Ser Lys Pro Thr Thr Lys Gln Arg Gln Asn Lys Pro
     1               5                  10                  15
   CCG AAC AAA CCG AAC AAC GAT TTC CAT TTC GAA GTG TTC AAC TTC GTG      96
   Pro Asn Lys Pro Asn Asn Asp Phe His Phe Glu Val Phe Asn Phe Val
               20                  25                  30
   CCG TGC AGC ATC TGC AGC AAC AAC CCG ACC TGC TGG GCG ATC TGC AAA     144
   Pro Cys Ser Ile Cys Ser Asn Asn Pro Thr Cys Trp Ala Ile Cys Lys
           35                  40                  45
   CGT ATC CCG AAC AAA AAA CCG GGC AAA AAA ACC                         177
   Arg Ile Pro Asn Lys Lys Pro Gly Lys Lys Thr
       50                  55
(2) INFORMATIONS POUR LA SEQ ID NO: 25:
   (i) CARACTERISTIQUES DE LA SEQUENCE:
      (A) LONGUEUR: 171 paires de bases
      (B) TYPE: nucléotide
      (C) NOMBRE DE BRINS: simple
      (D) CONFIGURATION: linéaire
   (ii) TYPE DE MOLECULE: ADN
   (ix) CARACTERISTIQUE:
      (A) NOM/CLE: CDS
      (B) EMPLACEMENT:1..171
   (xi) DESCRIPTION DE LA SEQUENCE: SEQ ID NO: 25:
   CAG ACC CAG CCG AGC AAA CCG ACC ACC AAA CAG CGT CAG AAC AAA CCG      48
   Gln Thr Gln Pro Ser Lys Pro Thr Thr Lys Gln Arg Gln Asn Lys Pro
    1               5                  10                  15
   CCG AAC AAA CCG AAC AAC GAT TTC CAT TTC GAA GTG TTC AAC TTC GTG      96
   Pro Asn Lys Pro Asn Asn Asp Phe His Phe Glu Val Phe Asn Phe Val
               20                  25                  30
   CCG TGC AGC ATC TGC AGC AAC AAC CCG ACC TGC TGG GCG ATC TGC AAA     144
   Pro Cys Ser Ile Cys Ser Asn Asn Pro Thr Cys Trp Ala Ile Cys Lys
           35                  40                  45
   CGT ATC CCG AAC AAA AAA CCG GGC AAA                                 171
   Arg Ile Pro Asn Lys Lys Pro Gly Lys
       50                  55
(2) INFORMATIONS POUR LA SEQ ID NO: 26:
   (i) CARACTERISTIQUES DE LA SEQUENCE:
      (A) LONGUEUR: 165 paires de bases
      (B) TYPE: nucléotide
      (C) NOMBRE DE BRINS: simple
      (D) CONFIGURATION: linéaire
   (ii) TYPE DE MOLECULE: ADN
   (ix) CARACTERISTIQUE:
      (A) NOM/CLE: CDS
      (B) EMPLACEMENT:1..165
   (xi) DESCRIPTION DE LA SEQUENCE: SEQ ID NO: 26:
   CAG ACC CAG CCG AGC AAA CCG ACC ACC AAA CAG CGT CAG AAC AAA CCG      48
   Gln Thr Gln Pro Ser Lys Pro Thr Thr Lys Gln Arg Gln Asn Lys Pro
     1               5                  10                  15
   CCG AAC AAA CCG AAC AAC GAT TTC CAT TTC GAA GTG TTC AAC TTC GTG      96
   Pro Asn Lys Pro Asn Asn Asp Phe His Phe Glu Val Phe Asn Phe Val
                20                  25                  30
   CCG TGC AGC ATC TGC AGC AAC AAC CCG ACC TGC TGG GCG ATC TGC AAA     144
   Pro Cys Ser Ile Cys Ser Asn Asn Pro Thr Cys Trp Ala Ile Cys Lys
            35                  40                  45
   CGT ATC CCG AAC AAA AAA CCG                                         165
   Arg Ile Pro Asn Lys Lys Pro
        50                  55
(2) INFORMATIONS POUR LA SEQ ID NO: 27:
   (i) CARACTERISTIQUES DE LA SEQUENCE:
      (A) LONGUEUR: 159 paires de bases
      (B) TYPE: nucléotide
      (C) NOMBRE DE BRINS: simple
      (D) CONFIGURATION: linéaire
   (ii) TYPE DE MOLECULE: ADN
   (ix) CARACTERISTIQUE:
      (A) NOM/CLE: CDS
      (B) EMPLACEMENT:1..159
   (xi) DESCRIPTION DE LA SEQUENCE: SEQ ID NO: 27:
   CAG ACC CAG CCG AGC AAA CCG ACC ACC AAA CAG CGT CAG AAC AAA CCG      48
   Gln Thr Gln Pro Ser Lys Pro Thr Thr Lys Gln Arg Gln Asn Lys Pro
     1               5                  10                  15
   CCG AAC AAA CCG AAC AAC GAT TTC CAT TTC GAA GTG TTC AAC TTC GTG      96
   Pro Asn Lys Pro Asn Asn Asp Phe His Phe Glu Val Phe Asn Phe Val
                20                  25                  30
   CCG TGC AGC ATC TGC AGC AAC AAC CCG ACC TGC TGG GCG ATC TGC AAA     144
   Pro Cys Ser Ile Cys Ser Asn Asn Pro Thr Cys Trp Ala Ile Cys Lys
            35                  40                  45
   CGT ATC CCG AAC AAA                                                 159
   Arg Ile Pro Asn Lys
        50
(2) INFORMATIONS POUR LA SEQ ID NO: 28:
   (i) CARACTERISTIQUES DE LA SEQUENCE:
      (A) LONGUEUR: 153 poires de bases
      (B) TYPE: nucléotide
      (C) NOMBRE DE BRINS: simple
      (D) CONFIGURATION: linéaire
   (ii) TYPE DE MOLECULE: ADN
   (ix) CARACTERISTIQUE:
      (A) NOM/CLE: CDS
      (B) EMPLACEMENT:1..153
   (xi) DESCRIPTION DE LA SEQUENCE: SEQ ID NO: 28:
   CAG ACC CAG CCG AGC AAA CCG ACC ACC AAA CAG CGT CAG AAC AAA CCG      48
   Gln Thr Gln Pro Ser Lys Pro Thr Thr Lys Gln Arg Gln Asn Lys Pro
     1               5                  10                  15
   CCG AAC AAA CCG AAC AAC GAT TTC CAT TTC GAA GTG TTC AAC TTC GTG      96
   Pro Asn Lys Pro Asn Asn Asp Phe His Phe Glu Val Phe Asn Phe Val
                20                  25                  30
   CCG TGC AGC ATC TGC AGC AAC AAC CCG ACC TGC TGG GCG ATC TGC AAA     144
   Pro Cys Ser Ile Cys Ser Asn Asn Pro Thr Cys Trp Ala Ile Cys Lys
            35                  40                  45
   CGT ATC CCG                                                         153
   Arg Ile Pro
        50
(2) INFORMATIONS POUR LA SEQ ID NO: 29:
   (i) CARACTERISTIQUES DE LA SEQUENCE:
      (A) LONGUEUR: 99 paires de bases
      (B) TYPE: nucléotide
      (C) NOMBRE DE BRINS: simple
      (D) CONFIGURATION: linéaire
   (ii) TYPE DE MOLECULE: ADN
   (ix) CARACTERISTIQUE:
      (A) NOM/CLE: CDS
      (B) EMPLACEMENT:1..99
   (xi) DESCRIPTION DE LA SEQUENCE: SEQ ID NO: 29:
   AAA CCG AAC AAC GAT TTC CAT TTC GAA GTG TTC AAC TTC GTG CCG TGC      48
   Lys Pro Asn Asn Asp Phe His Phe Glu Val Phe Asn Phe Val Pro Cys
     1               5                  10                  15
   AGC ATC TGC AGC AAC AAC CCG ACC TGC TGG GCG ATC TGC AAA CGT ATC      96
   Ser Ile Cys Ser Asn Asn Pro Thr Cys Trp Ala Ile Cys Lys Arg Ile
                20                  25                  30
   CCG                                                                  99
   Pro
(2) INFORMATIONS POUR LA SEQ ID NO: 30:
   (i) CARACTERISTIQUES DE LA SEQUENCE:
      (A) LONGUEUR: 183 paires de bases
      (B) TYPE: nucléotide
      (C) NOMBRE DE BRINS: simple
      (D) CONFIGURATION: linéaire
   (ii) TYPE DE MOLECULE: ADN
   (ix) CARACTERISTIQUE:
      (A) NOM/CLE: CDS
      (B) EMPLACEMENT:1..183
   (xi) DESCRIPTION DE LA SEQUENCE: SEQ ID NO: 30:
   CAG ACC CAG CCG AGC AAA CCG ACC ACC AAA CAG CGT CAG AAC AAA CCG      48
   Gln Thr Gln Pro Ser Lys Pro Thr Thr Lys Gln Arg Gln Asn Lys Pro
     1               5                  10                  15
   CCG AAC AAA CCG AAC AAC GAT TTC CAT TTC GAA GTG TTC AAC TTC GTG      96
   Pro Asn Lys Pro Asn Asn Asp Phe His Phe Glu Val Phe Asn Phe Val
                20                  25                  30
   CCG AGC AGC ATC TGC AGC AAC AAC CCG ACC TGC TGG GCG ATC AGC AAA     144
   Pro Ser Ser Ile Cys Ser Asn Asn Pro Thr Cys Trp Ala Ile Ser Lys
            35                  40                  45
   CGT ATC CCG AAC AAA AAA CCG GGC AAA AAA ACC ACG ACC                 183
   Arg Ile Pro Asn Lys Lys Pro Gly Lys Lys Thr Thr Thr
        50                  55                  60
(2) INFORMATIONS POUR LA SEQ ID NO: 31:
   (i) CARACTERISTIQUES DE LA SEQUENCE:
      (A) LONGUEUR: 177 paires de bases
      (B) TYPE: nucléotide
      (C) NOMBRE DE BRINS: simple
      (D) CONFIGURATION: linéaire
   (ii) TYPE DE MOLECULE: ADN
   (ix) CARACTERISTIQUE:
      (A) NOM/CLE: CDS
      (B) EMPLACEMENT:1..177
   (xi) DESCRIPTION DE LA SEQUENCE: SEQ ID NO: 31:
   CAG ACC CAG CCG AGC AAA CCG ACC ACC AAA CAG CGT CAG AAC AAA CCG      48
   Gln Thr Gln Pro Ser Lys Pro Thr Thr Lys Gln Arg Gln Asn Lys Pro
     1               5                  10                  15
   CCG AAC AAA CCG AAC AAC GAT TTC CAT TTC GAA GTG TTC AAC TTC GTG      96
   Pro Asn Lys Pro Asn Asn Asp Phe His Phe Glu Val Phe Asn Phe Val
                20                  25                  30
   CCG AGC AGC ATC TGC AGC AAC AAC CCG ACC TGC TGG GCG ATC AGC AAA     144
   Pro Ser Ser Ile Cys Ser Asn Asn Pro Thr Cys Trp Ala Ile Ser Lys
            35                  40                  45
   CGT ATC CCG AAC AAA AAA CCG GGC AAA AAA ACC                         177
   Arg Ile Pro Asn Lys Lys Pro Gly Lys Lys Thr
        50                  55
(2) INFORMATIONS POUR LA SEQ ID NO: 32:
   (i) CARACTERISTIQUES DE LA SEQUENCE:
      (A) LONGUEUR: 171 paires de bases
      (B) TYPE: nucléotide
      (C) NOMBRE DE BRINS: simple
      (D) CONFIGURATION: linéaire
   (ii) TYPE DE MOLECULE: ADN
   (ix) CARACTERISTIQUE:
      (A) NOM/CLE: CDS
      (B) EMPLACEMENT:1..171
   (xi) DESCRIPTION DE LA SEQUENCE: SEQ ID NO: 32:
   CAG ACC CAG CCG AGC AAA CCG ACC ACC AAA CAG CGT CAG AAC AAA CCG     48
   Gln Thr Gln Pro Ser Lys Pro Thr Thr Lys Gln Arg Gln Asn Lys Pro
    1               5                  10                  15
   CCG AAC AAA CCG AAC AAC GAT TTC CAT TTC GAA GTG TTC AAC TTC GTG     96
   Pro Asn Lys Pro Asn Asn Asp Phe His Phe Glu Val Phe Asn Phe Val
               20                  25                  30
   CCG AGC AGC ATC TGC AGC AAC AAC CCG ACC TGC TGG GCG ATC AGC AAA    144
   Pro Ser Ser Ile Cys Ser Asn Asn Pro Thr Cys Trp Ala Ile Ser Lys
           35                  40                  45
   CGT ATC CCG AAC AAA AAA CCG GGC AAA                                171
   Arg Ile Pro Asn Lys Lys Pro Gly Lys
       50                  55
(2) INFORMATIONS POUR LA SEQ ID NO: 33:
   (i) CARACTERISTIQUES DE LA SEQUENCE:
      (A) LONGUEUR: 165 paires de bases
      (B) TYPE: nucléotide
      (C) NOMBRE DE BRINS: simple
      (D) CONFIGURATION: linéaire
   (ii) TYPE DE MOLECULE: ADN
   (ix) CARACTERISTIQUE:
      (A) NOM/CLE: CDS
      (B) EMPLACEMENT:1..165
   (xi) DESCRIPTION DE LA SEQUENCE: SEQ ID NO: 33:
   CAG ACC CAG CCG AGC AAA CCG ACC ACC AAA CAG CGT CAG AAC AAA CCG     48
   Gln Thr Gln Pro Ser Lys Pro Thr Thr Lys Gln Arg Gln Asn Lys Pro
    1               5                  10                  15
   CCG AAC AAA CCG AAC AAC GAT TTC CAT TTC GAA GTG TTC AAC TTC GTG     96
   Pro Asn Lys Pro Asn Asn Asp Phe His Phe Glu Val Phe Asn Phe Val
               20                  25                  30
   CCG AGC AGC ATC TGC AGC AAC AAC CCG ACC TGC TGG GCG ATC AGC AAA    144
   Pro Ser Ser Ile Cys Ser Asn Asn Pro Thr Cys Trp Ala Ile Ser Lys
           35                  40                  45
   CGT ATC CCG AAC AAA AAA CCG                                        165
   Arg Ile Pro Asn Lys Lys Pro
       50                  55
(2) INFORMATIONS POUR LA SEQ ID NO: 34:
   (i) CARACTERISTIQUES DE LA SEQUENCE:
      (A) LONGUEUR: 159 paires de bases
      (B) TYPE: nucléotide
      (C) NOMBRE DE BRINS: simple
      (D) CONFIGURATION: linéaire
   (ii) TYPE DE MOLECULE: ADN
   (ix) CARACTERISTIQUE:
      (A) NOM/CLE: CDS
      (B) EMPLACEMENT:1..159
   (xi) DESCRIPTION DE LA SEQUENCE: SEQ ID NO: 34:
   CAG ACC CAG CCG AGC AAA CCG ACC ACC AAA CAG CGT CAG AAC AAA CCG        48
   Gln Thr Gln Pro Ser Lys Pro Thr Thr Lys Gln Arg Gln Asn Lys Pro
    1               5                  10                  15
   CCG AAC AAA CCG AAC AAC GAT TTC CAT TTC GAA GTG TTC AAC TTC GTG        96
   Pro Asn Lys Pro Asn Asn Asp Phe His Phe Glu Val Phe Asn Phe Val
               20                  25                  30
   CCG AGC AGC ATC TGC AGC AAC AAC CCG ACC TGC TGG GCG ATC AGC AAA       144
   Pro Ser Ser Ile Cys Ser Asn Asn Pro Thr Cys Trp Ala Ile Ser Lys
           35                  40                  45
   CGT ATC CCG AAC AAA                                                   159
   Arg Ile Pro Asn Lys
       50
(2) INFORMATIONS POUR LA SEQ ID NO: 35:
   (i) CARACTERISTIQUES DE LA SEQUENCE:
      (A) LONGUEUR: 153 paires de bases
      (B) TYPE: nucléotide
      (C) NOMBRE DE BRINS: simple
      (D) CONFIGURATION: linéaire
   (ii) TYPE DE MOLECULE: ADN
   (ix) CARACTERISTIQUE:
      (A) NOM/CLE: CDS
      (B) EMPLACEMENT:1..153
   (xi) DESCRIPTION DE LA SEQUENCE: SEQ ID NO: 35:
   CAG ACC CAG CCG AGC AAA CCG ACC ACC AAA CAG CGT CAG AAC AAA CCG        48
   Gln Thr Gln Pro Ser Lys Pro Thr Thr Lys Gln Arg Gln Asn Lys Pro
    1               5                  10                  15
   CCG AAC AAA CCG AAC AAC GAT TTC CAT TTC GAA GTG TTC AAC TTC GTG        96
   Pro Asn Lys Pro Asn Asn Asp Phe His Phe Glu Val Phe Asn Phe Val
                   20                  25                  30
   CCG AGC AGC ATC TGC AGC AAC AAC CCG ACC TGC TGG GCG ATC AGC AAA       144
   Pro Ser Ser Ile Cys Ser Asn Asn Pro Thr Cys Trp Ala Ile Ser Lys
               35                  40                  45
   CGT ATC CCG                                                           153
   Arg Ile Pro
       50
(2) INFORMATIONS POUR LA SEQ ID NO: 36:
   (i) CARACTERISTIQUES DE LA SEQUENCE:
      (A) LONGUEUR: 99 paires de bases
      (B) TYPE: nucléotide
      (C) NOMBRE DE BRINS: simple
      (D) CONFIGURATION: linéaire
   (ii) TYPE DE MOLECULE: ADN
   (ix) CARACTERISTIQUE:
      (A) NOM/CLE: CDS
      (B) EMPLACEMENT:1..99
   (xi) DESCRIPTION DE LA SEQUENCE: SEQ ID NO: 36:
   AAA CCG AAC AAC GAT TTC CAT TTC GAA GTG TTC AAC TTC GTG CCG AGC        48
   Lys Pro Asn Asn Asp Phe His Phe Glu Val Phe Asn Phe Val Pro Ser
     1               5                  10                  15
   AGC ATC TGC AGC AAC AAC CCG ACC TGC TGG GCG ATC AGC AAA CGT ATC        96
   Ser Ile Cys Ser Asn Asn Pro Thr Cys Trp Ala Ile Ser Lys Arg Ile
                   20                  25                  30
   CCG                                                                    99
   Pro
(2) INFORMATIONS POUR LA SEQ ID NO: 37:
   (i) CARACTERISTIQUES DE LA SEQUENCE:
      (A) LONGUEUR: 183 paires de bases
      (B) TYPE: nucléotide
      (C) NOMBRE DE BRINS: simple
      (D) CONFIGURATION: linéaire
   (ii) TYPE DE MOLECULE: ADN
   (ix) CARACTERISTIQUE:
      (A) NOM/CLE: CDS
      (B) EMPLACEMENT:1..183
   (xi) DESCRIPTION DE LA SEQUENCE: SEQ ID NO: 37:
   AGC ACC CAG ACC AAC AAA CCG AGC ACC AAA AGC CGT AGC AAA AAC CCG        48
   Ser Thr Gln Thr Asn Lys Pro Ser Thr Lys Ser Arg Ser Lys Asn Pro
     1               5                  10                  15
   CCG AAA AAA CCG AAA GAT GAT TAC CAC TTC GAA GTG TTC AAC TTC GTG        96
   Pro Lys Lys Pro Lys Asp Asp Tyr His Phe Glu Val Phe Asn Phe Val
                20                  25                  30
   CCC TGC AGC ATC TGC GGC AAC AAC CAG CTG TGC AAA AGC ATC TGC AAA       144
   Pro Cys Ser Ile Cys Gly Asn Asn Gln Leu Cys Lys Ser Ile Cys Lys
            35                  40                  45
   ACC ATC CCG AGC AAC AAA CCG AAA AAG AAA CCG ACC ATC                   183
   Thr Ile Pro Ser Asn Lys Pro Lys Lys Lys Pro Thr Ile
        50                  55                  60
(2) INFORMATIONS POUR LA SEQ ID NO: 38:
   (i) CARACTERISTIQUES DE LA SEQUENCE:
      (A) LONGUEUR: 177 paires de bases
      (B) TYPE: nucléotide
      (C) NOMBRE DE BRINS: simple
      (D) CONFIGURATION: linéaire
   (ii) TYPE DE MOLECULE: ADN
   (ix) CARACTERISTIQUE:
      (A) NOM/CLE: CDS
      (B) EMPLACEMENT:1..177
   (xi) DESCRIPTION DE LA SEQUENCE: SEQ ID NO: 38:
   AGC ACC CAG ACC AAC AAA CCG AGC ACC AAA AGC CGT AGC AAA AAC CCG        48
   Ser Thr Gln Thr Asn Lys Pro Ser Thr Lys Ser Arg Ser Lys Asn Pro
     1               5                  10                  15
   CCG AAA AAA CCG AAA GAT GAT TAC CAC TTC GAA GTG TTC AAC TTC GTG      96
   Pro Lys Lys Pro Lys Asp Asp Tyr His Phe Glu Val Phe Asn Phe Val
               20                  25                  30
   CCC TGC AGC ATC TGC GGC AAC AAC CAG CTG TGC AAA AGC ATC TGC AAA     144
   Pro Cys Ser Ile Cys Gly Asn Asn Gln Leu Cys Lys Ser Ile Cys Lys
           35                  40                  45
   ACC ATC CCG AGC AAC AAA CCG AAA AAG AAA CCG                         177
   Thr Ile Pro Ser Asn Lys Pro Lys Lys Lys Pro
       50                  55
(2) INFORMATIONS POUR LA SEQ ID NO: 39:
   (i) CARACTERISTIQUES DE LA SEQUENCE:
      (A) LONGUEUR: 171 paires de bases
      (B) TYPE: nucléotide
      (C) NOMBRE DE BRINS: simple
      (D) CONFIGURATION: linéaire
   (ii) TYPE DE MOLECULE: ADN
   (ix) CARACTERISTIQUE:
      (A) NOM/CLE: CDS
      (B) EMPLACEMENT:1..171
   (xi) DESCRIPTION DE LA SEQUENCE: SEQ ID NO: 39:
   AGC ACC CAG ACC AAC AAA CCG AGC ACC AAA AGC CGT AGC AAA AAC CCG      48
   Ser Thr Gln Thr Asn Lys Pro Ser Thr Lys Ser Arg Ser Lys Asn Pro
     1               5                  10                  15
   CCG AAA AAA CCG AAA GAT GAT TAC CAC TTC GAA GTG TTC AAC TTC GTG      96
   Pro Lys Lys Pro Lys Asp Asp Tyr His Phe Glu Val Phe Asn Phe Val
                20                  25                  30
   CCC TGC AGC ATC TGC GGC AAC AAC CAG CTG TGC AAA AGC ATC TGC AAA     144
   Pro Cys Ser Ile Cys Gly Asn Asn Gln Leu Cys Lys Ser Ile Cys Lys
            35                  40                  45
   ACC ATC CCG AGC AAC AAA CCG AAA AAG                                 171
   Thr Ile Pro Ser Asn Lys Pro Lys Lys
        50                  55
(2) INFORMATIONS POUR LA SEQ ID NO: 40:
   (i) CARACTERISTIQUES DE LA SEQUENCE:
      (A) LONGUEUR: 165 paires de bases
      (B) TYPE: nucléotide
      (C) NOMBRE DE BRINS: simple
      (D) CONFIGURATION: linéaire
   (ii) TYPE DE MOLECULE: ADN
   (ix) CARACTERISTIQUE:
      (A) NOM/CLE: CDS
      (B) EMPLACEMENT:1..165
   (xi) DESCRIPTION DE LA SEQUENCE: SEQ ID NO: 40:
   AGC ACC CAG ACC AAC AAA CCG AGC ACC AAA AGC CGT AGC AAA AAC CCG        48
   Ser Thr Gln Thr Asn Lys Pro Ser Thr Lys Ser Arg Ser Lys Asn Pro
     1               5                  10                  15
   CCG AAA AAA CCG AAA GAT GAT TAC CAC TTC GAA GTG TTC AAC TTC GTG        96
   Pro Lys Lys Pro Lys Asp Asp Tyr His Phe Glu Val Phe Asn Phe Val
                20                  25                  30
   CCC TGC AGC ATC TGC GGC AAC AAC CAG CTG TGC AAA AGC ATC TGC AAA       144
   Pro Cys Ser Ile Cys Gly Asn Asn Gln Leu Cys Lys Ser Ile Cys Lys
            35                  40                  45
   ACC ATC CCG AGC AAC AAA CCG                                           165
   Thr Ile Pro Ser Asn Lys Pro
        50                  55
(2) INFORMATIONS POUR LA SEQ ID NO: 41:
   (i) CARACTERISTIQUES DE LA SEQUENCE:
      (A) LONGUEUR: 159 paires de bases
      (B) TYPE: nucléotide
      (C) NOMBRE DE BRINS: simple
      (D) CONFIGURATION: linéaire
   (ii) TYPE DE MOLECULE: ADN
   (ix) CARACTERISTIQUE:
      (A) NOM/CLE: CDS
      (B) EMPLACEMENT:1..159
   (xi) DESCRIPTION DE LA SEQUENCE: SEQ ID NO: 41:
   AGC ACC CAG ACC AAC AAA CCG AGC ACC AAA AGC CGT AGC AAA AAC CCG      48
   Ser Thr Gln Thr Asn Lys Pro Ser Thr Lys Ser Arg Ser Lys Asn Pro
    1               5                  10                  15
   CCG AAA AAA CCG AAA GAT GAT TAC CAC TTC GAA GTG TTC AAC TTC GTG      96
   Pro Lys Lys Pro Lys Asp Asp Tyr His Phe Glu Val Phe Asn Phe Val
               20                  25                  30
   CCC TGC AGC ATC TGC GGC AAC AAC CAG CTG TGC AAA AGC ATC TGC AAA     144
   Pro Cys Ser Ile Cys Gly Asn Asn Gln Leu Cys Lys Ser Ile Cys Lys
           35                  40                  45
   ACC ATC CCG AGC AAC                                                 159
   Thr Ile Pro Ser Asn
       50
(2) INFORMATIONS POUR LA SEQ ID NO: 42:
   (i) CARACTERISTIQUES DE LA SEQUENCE:
      (A) LONGUEUR: 153 paires de bases
      (B) TYPE: nucléotide
      (C) NOMBRE DE BRINS: simple
      (D) CONFIGURATION: linéaire
   (ii) TYPE DE MOLECULE: ADN
   (ix) CARACTERISTIQUE:
      (A) NOM/CLE: CDS
      (B) EMPLACEMENT:1..153
   (xi) DESCRIPTION DE LA SEQUENCE: SEQ ID NO: 42:
   AGC ACC CAG ACC AAC AAA CCG AGC ACC AAA AGC CGT AGC AAA AAC CCG      48
   Ser Thr Gln Thr Asn Lys Pro Ser Thr Lys Ser Arg Ser Lys Asn Pro
    1               5                  10                  15
   CCG AAA AAA CCG AAA GAT GAT TAC CAC TTC GAA GTG TTC AAC TTC GTG      96
   Pro Lys Lys Pro Lys Asp Asp Tyr His Phe Glu Val Phe Asn Phe Val
               20                  25                  30
   CCC TGC AGC ATC TGC GGC AAC AAC CAG CTG TGC AAA AGC ATC TGC AAA     144
   Pro Cys Ser Ile Cys Gly Asn Asn Gln Leu Cys Lys Ser Ile Cys Lys
           35                  40                  45
   ACC ATC CCG                                                         153
   Thr Ile Pro
       50
(2) INFORMATIONS POUR LA SEQ ID NO: 43:
   (i) CARACTERISTIQUES DE LA SEQUENCE:
      (A) LONGUEUR: 99 paires de bases
      (B) TYPE: nucléotide
      (C) NOMBRE DE BRINS: simple
      (D) CONFIGURATION: linéaire
   (ii) TYPE DE MOLECULE: ADN
   (ix) CARACTERISTIQUE:
      (A) NOM/CLE: CDS
      (B) EMPLACEMENT:1..99
   (xi) DESCRIPTION DE LA SEQUENCE: SEQ ID NO: 43:
   AAA CCG AAA GAT GAT TAC CAC TTC GAA GTG TTC AAC TTC GTG CCC TGC      48
   Lys Pro Lys Asp Asp Tyr His Phe Glu Val Phe Asn Phe Val Pro Cys
     1               5                  10                  15
   AGC ATC TGC GGC AAC AAC CAG CTG TGC AAA AGC ATC TGC AAA ACC ATC      96
   Ser Ile Cys Gly Asn Asn Gln Leu Cys Lys Ser Ile Cys Lys Thr Ile
                20                  25                  30
   CCG                                                                  99
   Pro
(2) INFORMATIONS POUR LA SEQ ID NO: 44:
   (i) CARACTERISTIQUES DE LA SEQUENCE:
      (A) LONGUEUR: 183 paires de bases
      (B) TYPE: nucléotide
      (C) NOMBRE DE BRINS: simple
      (D) CONFIGURATION: linéaire
   (ii) TYPE DE MOLECULE: ADN
   (ix) CARACTERISTIQUE:
      (A) NOM/CLE: CDS
      (B) EMPLACEMENT:1..183
   (xi) DESCRIPTION DE LA SEQUENCE: SEQ ID NO: 44:
   AGC ACC CAG ACC AAC AAA CCG AGC ACC AAA AGC CGT AGC AAA AAC CCG      48
   Ser Thr Gln Thr Asn Lys Pro Ser Thr Lys Ser Arg Ser Lys Asn Pro
     1               5                  10                  15
   CCG AAA AAA CCG AAA GAT GAT TAC CAC TTC GAA GTG TTC AAC TTC GTG      96
   Pro Lys Lys Pro Lys Asp Asp Tyr His Phe Glu Val Phe Asn Phe Val
                20                  25                  30
   CCC AGC AGC ATC TGC GGC AAC AAC CAG CTG TGC AAA AGC ATC AGC AAA     144
   Pro Ser Ser Ile Cys Gly Asn Asn Gln Leu Cys Lys Ser Ile Ser Lys
            35                  40                  45
   ACC ATC CCG AGC AAC AAA CCG AAA AAG AAA CCG ACC ATC                 183
   Thr Ile Pro Ser Asn Lys Pro Lys Lys Lys Pro Thr Ile
        50                  55                  60
(2) INFORMATIONS POUR LA SEQ ID NO: 45:
   (i) CARACTERISTIQUES DE LA SEQUENCE:
      (A) LONGUEUR: 177 paires de bases
      (B) TYPE: nucléotide
      (C) NOMBRE DE BRINS: simple
      (D) CONFIGURATION: linéaire
   (ii) TYPE DE MOLECULE: ADN
   (ix) CARACTERISTIQUE:
      (A) NOM/CLE: CDS
      (B) EMPLACEMENT:1..177
   (xi) DESCRIPTION DE LA SEQUENCE: SEQ ID NO: 45:
   AGC ACC CAG ACC AAC AAA CCG AGC ACC AAA AGC CGT AGC AAA AAC CCG      48
   Ser Thr Gln Thr Asn Lys Pro Ser Thr Lys Ser Arg Ser Lys Asn Pro
     1               5                  10                  15
   CCG AAA AAA CCG AAA GAT GAT TAC CAC TTC GAA GTG TTC AAC TTC GTG      96
   Pro Lys Lys Pro Lys Asp Asp Tyr His Phe Glu Val Phe Asn Phe Val
                20                  25                  30
   CCC AGC AGC ATC TGC GGC AAC AAC CAG CTG TGC AAA AGC ATC AGC AAA     144
   Pro Ser Ser Ile Cys Gly Asn Asn Gln Leu Cys Lys Ser Ile Ser Lys
            35                  40                  45
   ACC ATC CCG AGC AAC AAA CCG AAA AAG AAA CCG                         177
   Thr Ile Pro Ser Asn Lys Pro Lys Lys Lys Pro
        50                  55
(2) INFORMATIONS POUR LA SEQ ID NO: 46:
   (i) CARACTERISTIQUES DE LA SEQUENCE:
      (A) LONGUEUR: 171 paires de bases
      (B) TYPE: nucléotide
      (C) NOMBRE DE BRINS: simple
      (D) CONFIGURATION: linéaire
   (ii) TYPE DE MOLECULE: ADN
   (ix) CARACTERISTIQUE:
      (A) NOM/CLE: CDS
      (B) EMPLACEMENT:1..171
   (xi) DESCRIPTION DE LA SEQUENCE: SEQ ID NO: 46:
   AGC ACC CAG ACC AAC AAA CCG AGC ACC AAA AGC CGT AGC AAA AAC CCG        48
   Ser Thr Gln Thr Asn Lys Pro Ser Thr Lys Ser Arg Ser Lys Asn Pro
     1               5                  10                  15
   CCG AAA AAA CCG AAA GAT GAT TAC CAC TTC GAA GTG TTC AAC TTC GTG        96
   Pro Lys Lys Pro Lys Asp Asp Tyr His Phe Glu Val Phe Asn Phe Val
                20                  25                  30
   CCC AGC AGC ATC TGC GGC AAC AAC CAG CTG TGC AAA AGC ATC AGC AAA       144
   Pro Ser Ser Ile Cys Gly Asn Asn Gln Leu Cys Lys Ser Ile Ser Lys
            35                  40                  45
   ACC ATC CCG AGC AAC AAA CCG AAA AAG                                   171
   Thr Ile Pro Ser Asn Lys Pro Lys Lys
        50                  55
(2) INFORMATIONS POUR LA SEQ ID NO: 47:
   (i) CARACTERISTIQUES DE LA SEQUENCE:
      (A) LONGUEUR: 165 paires de bases
      (B) TYPE: nucléotide
      (C) NOMBRE DE BRINS: simple
      (D) CONFIGURATION: linéaire
   (ii) TYPE DE MOLECULE: ADN
   (ix) CARACTERISTIQUE:
      (A) NOM/CLE: CDS
      (B) EMPLACEMENT:1..165
   (xi) DESCRIPTION DE LA SEQUENCE: SEQ ID NO: 47:
   AGC ACC CAG ACC AAC AAA CCG AGC ACC AAA AGC CGT AGC AAA AAC CCG      48
   Ser Thr Gln Thr Asn Lys Pro Ser Thr Lys Ser Arg Ser Lys Asn Pro
     1               5                  10                  15
   CCG AAA AAA CCG AAA GAT GAT TAC CAC TTC GAA GTG TTC AAC TTC GTG      96
   Pro Lys Lys Pro Lys Asp Asp Tyr His Phe Glu Val Phe Asn Phe Val
                20                  25                  30
   CCC AGC AGC ATC TGC GGC AAC AAC CAG CTG TGC AAA AGC ATC AGC AAA     144
   Pro Ser Ser Ile Cys Gly Asn Asn Gln Leu Cys Lys Ser Ile Ser Lys
            35                  40                  45
   ACC ATC CCG AGC AAC AAA CCG                                         165
   Thr Ile Pro Ser Asn Lys Pro
        50                  55
(2) INFORMATIONS POUR LA SEQ ID NO: 48:
   (i) CARACTERISTIQUES DE LA SEQUENCE:
      (A) LONGUEUR: 159 poires de bases
      (B) TYPE: nucléotide
      (C) NOMBRE DE BRINS: simple
      (D) CONFIGURATION: linéaire
   (ii) TYPE DE MOLECULE: ADN
   (ix) CARACTERISTIQUE:
      (A) NOM/CLE: CDS
      (B) EMPLACEMENT:1..159
   (xi) DESCRIPTION DE LA SEQUENCE: SEQ ID NO: 48:
(2) INFORMATIONS POUR LA SEQ ID NO: 49:
   (i) CARACTERISTIQUES DE LA SEQUENCE:
      (A) LONGUEUR: 153 paires de bases
      (B) TYPE: nucléotide
      (C) NOMBRE DE BRINS: simple
      (D) CONFIGURATION: linéaire
   (ii) TYPE DE MOLECULE: ADN
   (ix) CARACTERISTIQUE:
      (A) NOM/CLE: CDS
      (B) EMPLACEMENT:1..153
   (xi) DESCRIPTION DE LA SEQUENCE: SEQ ID NO: 49:
   AGC ACC CAG ACC AAC AAA CCG AGC ACC AAA AGC CGT AGC AAA AAC CCG      48
   Ser Thr Gln Thr Asn Lys Pro Ser Thr Lys Ser Arg Ser Lys Asn Pro
     1               5                  10                  15
   CCG AAA AAA CCG AAA GAT GAT TAC CAC TTC GAA GTG TTC AAC TTC GTG      96
   Pro Lys Lys Pro Lys Asp Asp Tyr His Phe Glu Val Phe Asn Phe Val
                    20                  25                  30
   CCC AGC AGC ATC TGC GGC AAC AAC CAG CTG TGC AAA AGC ATC AGC AAA     144
   Pro Ser Ser Ile Cys Gly Asn Asn Gln Leu Cys Lys Ser Ile Ser Lys
                35                  40                  45
   ACC ATC CCG                                                         153
   Thr Ile Pro
        50
(2) INFORMATIONS POUR LA SEQ ID NO: 50:
   (i) CARACTERISTIQUES DE LA SEQUENCE:
      (A) LONGUEUR: 99 paires de bases
      (B) TYPE: nucléotide
      (C) NOMBRE DE BRINS: simple
      (D) CONFIGURATION: linéaire
   (ii) TYPE DE MOLECULE: ADN
   (ix) CARACTERISTIQUE:
      (A) NOM/CLE: CDS
      (B) EMPLACEMENT:1..99
   (xi) DESCRIPTION DE LA SEQUENCE: SEQ ID NO: 91:
(2) INFORMATIONS POUR LA SEQ ID NO: 51:
   (i) CARACTERISTIQUES DE LA SEQUENCE:
      (A) LONGUEUR: 303 paires de bases
      (B) TYPE: nucléotide
      (C) NOMBRE DE BRINS: simple
      (D) CONFIGURATION: linéaire
   (ii) TYPE DE MOLECULE: ADN
   (ix) CARACTERISTIQUE:
      (A) NOM/CLE: CDS
      (B) EMPLACEMENT:1..303
   (xi) DESCRIPTION DE LA SEQUENCE: SEQ ID NO: 51:
   CAA AAC AGA AAA ATC AAA GGT CAA TCA ACA CTA CCA GCC ACA AGA AAA      48
   Gln Asn Arg Lys Ile Lys Gly Gln Ser Thr Leu Pro Ala Thr Arg Lys
     1               5                  10                  15
   CCA CCA ATT AAT CCA TCA GGA AGC ATC CCA CCA GAA AAC CAT CAA GAC      96
   Pro Pro Ile Asn Pro Ser Gly Ser Ile Pro Pro Glu Asn His Gln Asp
                20                  25                  30
   CAC AAC AAC TTC CAA ACA CTC CCC TAT GTT CCC TGC AGT ACA TGT GAA     144
   His Asn Asn Phe Gln Thr Leu Pro Tyr Val Pro Cys Ser Thr Cys Glu
            35                  40                  45
   GGT AAT CTT GCA TGC TTA TCA CTC TGC CAT ATT GAG ACG GAA AGA GCA     192
   Gly Asn Leu Ala Cys Leu Ser Leu Cys His Ile Glu Thr Glu Arg Ala
        50                  55                  60
   CCA AGC AGA GCA CCA ACA ATC ACC CTC AAA AAG ACA CCA AAA CCA AAA     240
   Pro Ser Arg Ala Pro Thr Ile Thr Leu Lys Lys Thr Pro Lys Pro Lys
    65                  70                  75                  80
   ACC ACA AAA AAG CCA ACC AAG ACA ACA ATC CAT CAC AGA ACC AGC CCA     288
   Thr Thr Lys Lys Pro Thr Lys Thr Thr Ile His His Arg Thr Ser Pro
                    85                  90                  95
   GAA ACC AAA CTG CAA                                                 303
   Glu Thr Lys Leu Gln
               100
(2) INFORMATIONS POUR LA SEQ ID NO: 52:
   (i) CARACTERISTIQUES DE LA SEQUENCE:
      (A) LONGUEUR: 303 paires de bases
      (B) TYPE: nucléotide
      (C) NOMBRE DE BRINS: simple
      (D) CONFIGURATION: linéaire
   (ii) TYPE DE MOLECULE: ADN
   (ix) CARACTERISTIQUE:
      (A) NOM/CLE: CDS
      (B) EMPLACEMENT:1..303
   (xi) DESCRIPTION DE LA SEQUENCE: SEQ ID NO: 52:
   CAA AAC AGA AAA ATC AAA GGT CAA TCA ACA CTA CCA GCC ACA AGA AAA      48
   Gln Asn Arg Lys Ile Lys Gly Gln Ser Thr Leu Pro Ala Thr Arg Lys
    1               5                  10                  15
   CCA CCA ATT AAT CCA TCA GGA AGC ATC CCA CCA GAA AAC CAT CAA GAC      96
   Pro Pro Ile Asn Pro Ser Gly Ser Ile Pro Pro Glu Asn His Gln Asp
               20                  25                  30
   CAC AAC AAC TTC CAA ACA CTC CCC TAT GTT CCC AGC AGT ACA TGT GAA     144
   His Asn Asn Phe Gln Thr Leu Pro Tyr Val Pro Ser Ser Thr Cys Glu
           35                  40                  45
   GGT AAT CTT GCA TGC TTA TCA CTC AGC CAT ATT GAG ACG GAA AGA GCA     192
   Gly Asn Leu Ala Cys Leu Ser Leu Ser His Ile Glu Thr Glu Arg Ala
       50                  55                  60
   CCA AGC AGA GCA CCA ACA ATC ACC CTC AAA AAG ACA CCA AAA CCA AAA     240
   Pro Ser Arg Ala Pro Thr Ile Thr Leu Lys Lys Thr Pro Lys Pro Lys
   65                  70                  75                  80
   ACC ACA AAA AAG CCA ACC AAG ACA ACA ATC CAT CAC AGA ACC AGC CCA     288
   Thr Thr Lys Lys Pro Thr Lys Thr Thr Ile His His Arg Thr Ser Pro
                   85                  90                  95
   GAA ACC AAA CTG CAA                                                 303
   Glu Thr Lys Leu Gln
               100
(2) INFORMATIONS POUR LA SEQ ID NO: 53:
   (i) CARACTERISTIQUES DE LA SEQUENCE:
      (A) LONGUEUR: 183 paires de bases
      (B) TYPE: nucléotide
      (C) NOMBRE DE BRINS: simple
      (D) CONFIGURATION: linéaire
   (ii) TYPE DE MOLECULE: ADN
   (ix) CARACTERISTIQUE:
      (A) NOM/CLE: CDS
      (B) EMPLACEMENT:1..183
   (xi) DESCRIPTION DE LA SEQUENCE: SEQ ID NO: 53:
   CTA CCA GCC ACA AGA AAA CCA CCA ATT AAT CCA TCA GGA AGC ATC CCA     48
   Leu Pro Ala Thr Arg Lys Pro Pro Ile Asn Pro Ser Gly Ser Ile Pro
     1               5                  10                  15
   CCA GAA AAC CAT CAA GAC CAC AAC AAC TTC CAA ACA CTC CCC TAT GTT     96
   Pro Glu Asn His Gln Asp His Asn Asn Phe Gln Thr Leu Pro Tyr Val
                20                  25                  30
   CCC TGC AGT ACA TGT GAA GGT AAT CTT GCA TGC TTA TCA CTC TGC CAT    144
   Pro Cys Ser Thr Cys Glu Gly Asn Leu Ala Cys Leu Ser Leu Cys His
            35                  40                  45
   ATT GAG ACG GAA AGA GCA CCA AGC AGA GCA CCA ACA ATC                183
   Ile Glu Thr Glu Arg Ala Pro Ser Arg Ala Pro Thr Ile
        50                  55                  60
(2) INFORMATIONS POUR LA SEQ ID NO: 54:
   (i) CARACTERISTIQUES DE LA SEQUENCE:
      (A) LONGUEUR: 177 paires de bases
      (B) TYPE: nucléotide
      (C) NOMBRE DE BRINS: simple
      (D) CONFIGURATION: linéaire
   (ii) TYPE DE MOLECULE: ADN
   (ix) CARACTERISTIQUE:
      (A) NOM/CLE: CDS
      (B) EMPLACEMENT:1..177
   (xi) DESCRIPTION DE LA SEQUENCE: SEQ ID NO: 54:
   CTA CCA GCC ACA AGA AAA CCA CCA ATT AAT CCA TCA GGA AGC ATC CCA     48
   Leu Pro Ala Thr Arg Lys Pro Pro Ile Asn Pro Ser Gly Ser Ile Pro
     1               5                  10                  15
   CCA GAA AAC CAT CAA GAC CAC AAC AAC TTC CAA ACA CTC CCC TAT GTT     96
   Pro Glu Asn His Gln Asp His Asn Asn Phe Gln Thr Leu Pro Tyr Val
                20                  25                  30
   CCC TGC AGT ACA TGT GAA GGT AAT CTT GCA TGC TTA TCA CTC TGC CAT    144
   Pro Cys Ser Thr Cys Glu Gly Asn Leu Ala Cys Leu Ser Leu Cys His
            35                  40                  45
   ATT GAG ACG GAA AGA GCA CCA AGC AGA GCA CCA                        177
   Ile Glu Thr Glu Arg Ala Pro Ser Arg Ala Pro
        50                  55
(2) INFORMATIONS POUR LA SEQ ID NO: 55:
   (i) CARACTERISTIQUES DE LA SEQUENCE:
      (A) LONGUEUR: 171 paires de bases
      (B) TYPE: nucléotide
      (C) NOMBRE DE BRINS: simple
      (D) CONFIGURATION: linéaire
   (ii) TYPE DE MOLECULE: ADN
   (ix) CARACTERISTIQUE:
      (A) NOM/CLE: CDS
      (B) EMPLACEMENT:1..171
   (xi) DESCRIPTION DE LA SEQUENCE: SEQ ID NO: 55:
   CTA CCA GCC ACA AGA AAA CCA CCA ATT AAT CCA TCA GGA AGC ATC CCA      48
   Leu Pro Ala Thr Arg Lys Pro Pro Ile Asn Pro Ser Gly Ser Ile Pro
     1               5                  10                  15
   CCA GAA AAC CAT CAA GAC CAC AAC AAC TTC CAA ACA CTC CCC TAT GTT      96
   Pro Glu Asn His Gln Asp His Asn Asn Phe Gln Thr Leu Pro Tyr Val
                20                  25                  30
   CCC TGC AGT ACA TGT GAA GGT AAT CTT GCA TGC TTA TCA CTC TGC CAT     144
   Pro Cys Ser Thr Cys Glu Gly Asn Leu Ala Cys Leu Ser Leu Cys His
            35                  40                  45
   ATT GAG ACG GAA AGA GCA CCA AGC AGA                                 171
   Ile Glu Thr Glu Arg Ala Pro Ser Arg
        50                  55
(2) INFORMATIONS POUR LA SEQ ID NO: 56:
   (i) CARACTERISTIQUES DE LA SEQUENCE:
      (A) LONGUEUR: 165 paires de bases
      (B) TYPE: nucléotide
      (C) NOMBRE DE BRINS: simple
      (D) CONFIGURATION: linéaire
   (ii) TYPE DE MOLECULE: ADN
   (ix) CARACTERISTIQUE:
      (A) NOM/CLE: CDS
      (B) EMPLACEMENT:1..165
   (xi) DESCRIPTION DE LA SEQUENCE: SEQ ID NO: 56:
   CTA CCA GCC ACA AGA AAA CCA CCA ATT AAT CCA TCA GGA AGC ATC CCA      48
   Leu Pro Ala Thr Arg Lys Pro Pro Ile Asn Pro Ser Gly Ser Ile Pro
     1               5                  10                  15
   CCA GAA AAC CAT CAA GAC CAC AAC AAC TTC CAA ACA CTC CCC TAT GTT      96
   Pro Glu Asn His Gln Asp His Asn Asn Phe Gln Thr Leu Pro Tyr Val
                20                  25                  30
   CCC TGC AGT ACA TGT GAA GGT AAT CTT GCA TGC TTA TCA CTC TGC CAT     144
   Pro Cys Ser Thr Cys Glu Gly Asn Leu Ala Cys Leu Ser Leu Cys His
            35                  40                  45
   ATT GAG ACG GAA AGA GCA CCA                                         165
   Ile Glu Thr Glu Arg Ala Pro
        50                  55
(2) INFORMATIONS POUR LA SEQ ID NO: 57:
   (i) CARACTERISTIQUES DE LA SEQUENCE:
      (A) LONGUEUR: 159 paires de bases
      (B) TYPE: nucléotide
      (C) NOMBRE DE BRINS: simple
      (D) CONFIGURATION: linéaire
   (ii) TYPE DE MOLECULE: ADN
   (ix) CARACTERISTIQUE:
      (A) NOM/CLE: CDS
      (B) EMPLACEMENT:1..159
   (xi) DESCRIPTION DE LA SEQUENCE: SEQ ID NO: 57:
   CTA CCA GCC ACA AGA AAA CCA CCA ATT AAT CCA TCA GGA AGC ATC CCA      48
   Leu Pro Ala Thr Arg Lys Pro Pro Ile Asn Pro Ser Gly Ser Ile Pro
     1               5                  10                  15
   CCA GAA AAC CAT CAA GAC CAC AAC AAC TTC CAA ACA CTC CCC TAT GTT      96
   Pro Glu Asn His Gln Asp His Asn Asn Phe Gln Thr Leu Pro Tyr Val
                20                  25                  30
   CCC TGC AGT ACA TGT GAA GGT AAT CTT GCA TGC TTA TCA CTC TGC CAT     144
   Pro Cys Ser Thr Cys Glu Gly Asn Leu Ala Cys Leu Ser Leu Cys His
            35                  40                  45
   ATT GAG ACG GAA AGA                                                 159
   Ile Glu Thr Glu Arg
        50
(2) INFORMATIONS POUR LA SEQ ID NO: 58:
   (i) CARACTERISTIQUES DE LA SEQUENCE:
      (A) LONGUEUR: 153 paires de bases
      (B) TYPE: nucléotide
      (C) NOMBRE DE BRINS: simple
      (D) CONFIGURATION: linéaire
   (ii) TYPE DE MOLECULE: ADN
   (ix) CARACTERISTIQUE:
      (A) NOM/CLE: CDS
      (B) EMPLACEMENT:1..153
   (xi) DESCRIPTION DE LA SEQUENCE: SEQ ID NO: 58:
   CTA CCA GCC ACA AGA AAA CCA CCA ATT AAT CCA TCA GGA AGC ATC CCA      48
   Leu Pro Ala Thr Arg Lys Pro Pro Ile Asn Pro Ser Gly Ser Ile Pro
     1               5                  10                  15
   CCA GAA AAC CAT CAA GAC CAC AAC AAC TTC CAA ACA CTC CCC TAT GTT      96
   Pro Glu Asn His Gln Asp His Asn Asn Phe Gln Thr Leu Pro Tyr Val
                20                  25                  30
   CCC TGC AGT ACA TGT GAA GGT AAT CTT GCA TGC TTA TCA CTC TGC CAT     144
   Pro Cys Ser Thr Cys Glu Gly Asn Leu Ala Cys Leu Ser Leu Cys His
            35                  40                  45
   ATT GAG ACG                                                         153
   Ile Glu Thr
        50
(2) INFORMATIONS POUR LA SEQ ID NO: 59:
   (i) CARACTERISTIQUES DE LA SEQUENCE:
      (A) LONGUEUR: 99 paires de bases
      (B) TYPE: nucléotide
      (C) NOMBRE DE BRINS: simple
      (D) CONFIGURATION: linéaire
   (ii) TYPE DE MOLECULE: ADN
   (ix) CARACTERISTIQUE:
      (A) NOM/CLE: CDS
      (B) EMPLACEMENT:1..99
   (xi) DESCRIPTION DE LA SEQUENCE: SEQ ID NO: 59:
   AAC CAT CAA GAC CAC AAC AAC TTC CAA ACA CTC CCC TAT GTT CCC TGC      48
   Asn His Gln Asp His Asn Asn Phe Gln Thr Leu Pro Tyr Val Pro Cys
     1               5                  10                  15
   AGT ACA TGT GAA GGT AAT CTT GCA TGC TTA TCA CTC TGC CAT ATT GAG      96
   Ser Thr Cys Glu Gly Asn Leu Ala Cys Leu Ser Leu Cys His Ile Glu
               20                  25                  30
   ACG                                                                  99
   Thr
(2) INFORMATIONS POUR LA SEQ ID NO: 60:
   (i) CARACTERISTIQUES DE LA SEQUENCE:
      (A) LONGUEUR: 183 paires de bases
      (B) TYPE: nucléotide
      (C) NOMBRE DE BRINS: simple
      (D) CONFIGURATION: linéaire
   (ii) TYPE DE MOLECULE: ADN
   (ix) CARACTERISTIQUE:
      (A) NOM/CLE: CDS
      (B) EMPLACEMENT:1..183
   (xi) DESCRIPTION DE LA SEQUENCE: SEQ ID NO: 60:
   CTA CCA GCC ACA AGA AAA CCA CCA ATT AAT CCA TCA GGA AGC ATC CCA      48
   Leu Pro Ala Thr Arg Lys Pro Pro Ile Asn Pro Ser Gly Ser Ile Pro
     1               5                  10                  15
   CCA GAA AAC CAT CAA GAC CAC AAC AAC TTC CAA ACA CTC CCC TAT GTT      96
   Pro Glu Asn His Gln Asp His Asn Asn Phe Gln Thr Leu Pro Tyr Val
               20                  25                  30
   CCC AGC AGT ACA TGT GAA GGT AAT CTT GCA TGC TTA TCA CTC AGC CAT     144
   Pro Ser Ser Thr Cys Glu Gly Asn Leu Ala Cys Leu Ser Leu Ser His
           35                  40                  45
   ATT GAG ACG GAA AGA GCA CCA AGC AGA GCA CCA ACA ATC                 183
   Ile Glu Thr Glu Arg Ala Pro Ser Arg Ala Pro Thr Ile
       50                  55                  60
(2) INFORMATIONS POUR LA SEQ ID NO: 61:
   (i) CARACTERISTIQUES DE LA SEQUENCE:
      (A) LONGUEUR: 177 paires de bases
      (B) TYPE: nucléotide
      (C) NOMBRE DE BRINS: simple
      (D) CONFIGURATION: linéaire
   (ii) TYPE DE MOLECULE: ADN
   (ix) CARACTERISTIQUE:
      (A) NOM/CLE: CDS
      (B) EMPLACEMENT:1..177
   (xi) DESCRIPTION DE LA SEQUENCE: SEQ ID NO: 61:
   CTA CCA GCC ACA AGA AAA CCA CCA ATT AAT CCA TCA GGA AGC ATC CCA      48
   Leu Pro Ala Thr Arg Lys Pro Pro Ile Asn Pro Ser Gly Ser Ile Pro
     1               5                  10                  15
   CCA GAA AAC CAT CAA GAC CAC AAC AAC TTC CAA ACA CTC CCC TAT GTT      96
   Pro Glu Asn His Gln Asp His Asn Asn Phe Gln Thr Leu Pro Tyr Val
                    20                  25                  30
   CCC AGC AGT ACA TGT GAA GGT AAT CTT GCA TGC TTA TCA CTC AGC CAT     144
   Pro Ser Ser Thr Cys Glu Gly Asn Leu Ala Cys Leu Ser Leu Ser His
                35                  40                  45
   ATT GAG ACG GAA AGA GCA CCA AGC AGA GCA CCA                         177
   Ile Glu Thr Glu Arg Ala Pro Ser Arg Ala Pro
        50                  55
(2) INFORMATIONS POUR LA SEQ ID NO: 62:
   (i) CARACTERISTIQUES DE LA SEQUENCE:
      (A) LONGUEUR: 171 paires de bases
      (B) TYPE: nucléotide
      (C) NOMBRE DE BRINS: simple
      (D) CONFIGURATION: linéaire
   (ii) TYPE DE MOLECULE: ADN
   (ix) CARACTERISTIQUE:
      (A) NOM/CLE: CDS
      (B) EMPLACEMENT:1..171
   (xi) DESCRIPTION DE LA SEQUENCE: SEQ ID NO: 62:
   CTA CCA GCC ACA AGA AAA CCA CCA ATT AAT CCA TCA GGA AGC ATC CCA        48
   Leu Pro Ala Thr Arg Lys Pro Pro Ile Asn Pro Ser Gly Ser Ile Pro
     1               5                  10                  15
   CCA GAA AAC CAT CAA GAC CAC AAC AAC TTC CAA ACA CTC CCC TAT GTT        96
   Pro Glu Asn His Gln Asp His Asn Asn Phe Gln Thr Leu Pro Tyr Val
                    20                  25                  30
   CCC AGC AGT ACA TGT GAA GGT AAT CTT GCA TGC TTA TCA CTC AGC CAT       144
   Pro Ser Ser Thr Cys Glu Gly Asn Leu Ala Cys Leu Ser Leu Ser His
                35                  40                  45
   ATT GAG ACG GAA AGA GCA CCA AGC AGA                                   171
   Ile Glu Thr Glu Arg Ala Pro Ser Arg
        50                  55
(2) INFORMATIONS POUR LA SEQ ID NO: 63:
   (i) CARACTERISTIQUES DE LA SEQUENCE:
      (A) LONGUEUR: 165 paires de bases
      (B) TYPE: nucléotide
      (C) NOMBRE DE BRINS: simple
      (D) CONFIGURATION: linéaire
   (ii) TYPE DE MOLECULE: ADN
   (ix) CARACTERISTIQUE:
      (A) NOM/CLE: CDS
      (B) EMPLACEMENT:1..165
   (xi) DESCRIPTION DE LA SEQUENCE: SEQ ID NO: 63:
   CTA CCA GCC ACA AGA AAA CCA CCA ATT AAT CCA TCA GGA AGC ATC CCA        48
   Leu Pro Ala Thr Arg Lys Pro Pro Ile Asn Pro Ser Gly Ser Ile Pro
     1               5                  10                  15
   CCA GAA AAC CAT CAA GAC CAC AAC AAC TTC CAA ACA CTC CCC TAT GTT        96
   Pro Glu Asn His Gln Asp His Asn Asn Phe Gln Thr Leu Pro Tyr Val
                    20                  25                  30
   CCC AGC AGT ACA TGT GAA GGT AAT CTT GCA TGC TTA TCA CTC AGC CAT       144
   Pro Ser Ser Thr Cys Glu Gly Asn Leu Ala Cys Leu Ser Leu Ser His
                35                  40                  45
   ATT GAG ACG GAA AGA GCA CCA                                           165
   Ile Glu Thr Glu Arg Ala Pro
        50                  55
(2) INFORMATIONS POUR LA SEQ ID NO: 64:
   (i) CARACTERISTIQUES DE LA SEQUENCE:
      (A) LONGUEUR: 159 paires de bases
      (B) TYPE: nucléotide
      (C) NOMBRE DE BRINS: simple
      (D) CONFIGURATION: linéaire
   (ii) TYPE DE MOLECULE: ADN
   (ix) CARACTERISTIQUE:
      (A) NOM/CLE: CDS
      (B) EMPLACEMENT:1..159
   (xi) DESCRIPTION DE LA SEQUENCE: SEQ ID NO: 64:
   CTA CCA GCC ACA AGA AAA CCA CCA ATT AAT CCA TCA GGA AGC ATC CCA      48
   Leu Pro Ala Thr Arg Lys Pro Pro Ile Asn Pro Ser Gly Ser Ile Pro
     1               5                  10                  15
   CCA GAA AAC CAT CAA GAC CAC AAC AAC TTC CAA ACA CTC CCC TAT GTT      96
   Pro Glu Asn His Gln Asp His Asn Asn Phe Gln Thr Leu Pro Tyr Val
                    20                  25                  30
   CCC AGC AGT ACA TGT GAA GGT AAT CTT GCA TGC TTA TCA CTC AGC CAT     144
   Pro Ser Ser Thr Cys Glu Gly Asn Leu Ala Cys Leu Ser Leu Ser His
                35                  40                  45
   ATT GAG ACG GAA AGA                                                 159
   Ile Glu Thr Glu Arg
        50
(2) INFORMATIONS POUR LA SEQ ID NO: 65:
   (i) CARACTERISTIQUES DE LA SEQUENCE:
      (A) LONGUEUR: 153 paires de bases
      (B) TYPE: nucléotide
      (C) NOMBRE DE BRINS: simple
      (D) CONFIGURATION: linéaire
   (ii) TYPE DE MOLECULE: ADN
   (ix) CARACTERISTIQUE:
      (A) NOM/CLE: CDS
      (B) EMPLACEMENT:1..153
   (xi) DESCRIPTION DE LA SEQUENCE: SEQ ID NO: 65:
   CTA CCA GCC ACA AGA AAA CCA CCA ATT AAT CCA TCA GGA AGC ATC CCA    48
   Leu Pro Ala Thr Arg Lys Pro Pro Ile Asn Pro Ser Gly Ser Ile Pro
     1               5                  10                  15
   CCA GAA AAC CAT CAA GAC CAC AAC AAC TTC CAA ACA CTC CCC TAT GTT    96
   Pro Glu Asn His Gln Asp His Asn Asn Phe Gln Thr Leu Pro Tyr Val
                20                  25                  30
   CCC AGC AGT ACA TGT GAA GGT AAT CTT GCA TGC TTA TCA CTC AGC CAT   144
   Pro Ser Ser Thr Cys Glu Gly Asn Leu Ala Cys Leu Ser Leu Ser His
            35                  40                  45
   ATT GAG ACG                                                       153
   Ile Glu Thr
        50
(2) INFORMATIONS POUR LA SEQ ID NO: 66:
   (i) CARACTERISTIQUES DE LA SEQUENCE:
      (A) LONGUEUR: 99 paires de bases
      (B) TYPE: nucléotide
      (C) NOMBRE DE BRINS: simple
      (D) CONFIGURATION: linéaire
   (ii) TYPE DE MOLECULE: ADN
   (ix) CARACTERISTIQUE:
      (A) NOM/CLE: CDS
      (B) EMPLACEMENT:1..99
   (xi) DESCRIPTION DE LA SEQUENCE: SEQ ID NO: 66:
   AAC CAT CAA GAC CAC AAC AAC TTC CAA ACA CTC CCC TAT GTT CCC AGC    48
   Asn His Gln Asp His Asn Asn Phe Gln Thr Leu Pro Tyr Val Pro Ser
     1               5                  10                  15
   AGT ACA TGT GAA GGT AAT CTT GCA TGC TTA TCA CTC AGC CAT ATT GAG    96
   Ser Thr Cys Glu Gly Asn Leu Ala Cys Leu Ser Leu Ser His Ile Glu
                20                  25                  30
   ACG                                                                99
   Thr
(2) INFORMATIONS POUR LA SEQ ID NO: 67:
   (i) CARACTERISTIQUES DE LA SEQUENCE:
      (A) LONGUEUR: 51 paires de bases
      (B) TYPE: nucléotide
      (C) NOMBRE DE BRINS: simple
      (D) CONFIGURATION: linéaire
   (ii) TYPE DE MOLECULE: ADN
   (ix) CARACTERISTIQUE:
      (A) NOM/CLE: CDS
      (B) EMPLACEMENT:1..51
   (xi) DESCRIPTION DE LA SEQUENCE: SEQ ID NO: 67:
   GTT CCC TGC AGT ACA TGT GAA GGT AAT CTT GCA TGC TTA TCA CTC TGC      48
   Val Pro Cys Ser Thr Cys Glu Gly Asn Leu Ala Cys Leu Ser Leu Cys
    1               5                  10                  15
   CAT                                                                  51
   His
(2) INFORMATIONS POUR LA SEQ ID NO: 68:
   (i) CARACTERISTIQUES DE LA SEQUENCE:
      (A) LONGUEUR: 51 paires de bases
      (B) TYPE: nucléotide
      (C) NOMBRE DE BRINS: simple
      (D) CONFIGURATION: linéaire
   (ii) TYPE DE MOLECULE: ADN
   (ix) CARACTERISTIQUE:
      (A) NOM/CLE: CDS
      (B) EMPLACEMENT:1..51
   (xi) DESCRIPTION DE LA SEQUENCE: SEQ ID NO: 68:
   GTT CCC AGC AGT ACA TGT GAA GGT AAT CTT GCA TGC TTA TCA CTC AGC      48
   Val Pro Ser Ser Thr Cys Glu Gly Asn Leu Ala Cys Leu Ser Leu Ser
    1               5                  10                  15
   CAT                                                                  51
   His
(2) INFORMATIONS POUR LA SEQ ID NO: 69:
   (i) CARACTERISTIQUES DE LA SEQUENCE:
      (A) LONGUEUR: 17 acides aminés
      (B) TYPE: acide aminé
      (C) NOMBRE DE BRINS: simple
      (D) CONFIGURATION: linéaire
   (ii) TYPE DE MOLECULE: peptide
   (ix) CARACTERISTIQUE:
      (A) NOM/CLE: Modified-site
      (B) EMPLACEMENT:12
      (D) AUTRES INFORMATIONS:/Xaa signifie Orn
   (ix) CARACTERISTIQUE:
      (A) NOM/CLE: Modified-site
      (B) EMPLACEMENT:16
      (D) AUTRES INFORMATIONS:/Xaa signifie Orn
   (xi) DESCRIPTION DE LA SEQUENCE: SEQ ID NO: 69:
   Val Pro Asp Ser Thr Asp Glu Gly Asn Leu Ala Xaa Leu Ser Leu Xaa
   1               5                   10                  15
   His
(2) INFORMATIONS POUR LA SEQ ID NO: 70:
   (i) CARACTERISTIQUES DE LA SEQUENCE:
      (A) LONGUEUR: 17 acides aminés.
      (B) TYPE: acide aminé
      (C) NOMBRE DE BRINS: simple
      (D) CONFIGURATION: linéaire
   (ii) TYPE DE MOLECULE: peptide
   (ix) CARACTERISTIQUE:
      (A) NOM/CLE: Modified-site
      (B) EMPLACEMENT:12
      (D) AUTRES INFORMATIONS:/Xaa signifie Orn
   (xi) DESCRIPTION DE LA SEQUENCE: SEQ ID NO: 70:
   Val Pro Ser Ser Thr Asp Glu Gly Asn Leu Ala Xaa Leu Ser Leu Ser
   1               5                   10                  15
   His
(2) INFORMATIONS POUR LA SEQ ID NO: 71:
   (i) CARACTERISTIQUES DE LA SEQUENCE:
      (A) LONGUEUR: 42 poires de bases
      (B) TYPE: nucléotide
      (C) NOMBRE DE BRINS: simple
      (D) CONFIGURATION: linéaire
   (ii) TYPE DE MOLECULE: ADN
   (ix) CARACTERISTIQUE:
      (A) NOM/CLE: CDS
      (B) EMPLACEMENT:1..42
   (xi) DESCRIPTION DE LA SEQUENCE: SEQ ID NO: 71:
   AGT ACA TGT GAA GGT AAT CTT GCA TGC TTA TCA CTC TGC CAT          42
   Ser Thr Cys Glu Gly Asn Leu Ala Cys Leu Ser Leu Cys His
     1               5                  10
(2) INFORMATIONS POUR LA SEQ ID NO: 72:
   (i) CARACTERISTIQUES DE LA SEQUENCE:
      (A) LONGUEUR: 42 paires de bases
      (B) TYPE: nucléotide
      (C) NOMBRE DE BRINS: simple
      (D) CONFIGURATION: linéaire
   (ii) TYPE DE MOLECULE: ADN
   (ix) CARACTERISTIQUE:
      (A) NOM/CLE: CDS
      (B) EMPLACEMENT:1..42
   (xi) DESCRIPTION DE LA SEQUENCE: SEQ ID NO: 72:
   AGT ACA TGT GAA GGT AAT CTT GCA TGC TTA TCA CTC AGC CAT          42
   Ser Thr Cys Glu Gly Asn Leu Ala Cys Leu Ser Leu Ser His
     1               5                  10
(2) INFORMATIONS POUR LA SEQ ID NO: 73:
   (i) CARACTERISTIQUES DE LA SEQUENCE:
      (A) LONGUEUR: 14 acides aminés
      (B) TYPE: acide aminé
      (C) NOMBRE DE BRINS: simple
      (D) CONFIGURATION: linéaire
   (ii) TYPE DE MOLECULE: peptide
   (ix) CARACTERISTIQUE:
      (A) NOM/CLE: Modified-site
      (B) EMPLACEMENT:9
      (D) AUTRES INFORMATIONS:/Xaa signifie Orn
   (xi) DESCRIPTION DE LA SEQUENCE: SEQ ID NO: 73:
   Ser Thr Asp Glu Gly Asn Leu Ala Xaa Leu Ser Leu Ser His
   1 5 10
(2) INFORMATIONS POUR LA SEQ ID NO: 74:
   (i) CARACTERISTIQUES DE LA SEQUENCE:
      (A) LONGUEUR: 657 paires de bases
      (B) TYPE: nucléotide
      (C) NOMBRE DE BRINS: simple
      (D) CONFIGURATION: linéaire
   (ii) TYPE DE MOLECULE: ADN
   (ix) CARACTERISTIQUE:
      (A) NOM/CLE: CDS
      (B) EMPLACEMENT:1..657
   (xi) DESCRIPTION DE LA SEQUENCE: SEQ ID NO: 74:
   AAA TAT GGA GTA AGT GAC TAT TAC AAG AAT CTA ATC AAC AAT GCC AAA 48
   Lys Tyr Gly Val Ser Asp Tyr Tyr Lys Asn Leu Ile Asn Asn Ala Lys
   1 5 10 15
   ACT GTT GAA GGC GTA AAA GAC CTT CAA GCA CAA GTT GTT GAA TCA GCG      96
   Thr Val Glu Gly Val Lys Asp Leu Gln Ala Gln Val Val Glu Ser Ala
               20                  25                  30
   AAG AAA GCG CGT ATT TCA GAA GCA ACA GAT GGC TTA TCT GAT TTC TTG     144
   Lys Lys Ala Arg Ile Ser Glu Ala Thr Asp Gly Leu Ser Asp Phe Leu
           35                  40                  45
   AAA TCA CAA ACA CCT GCT GAA GAT ACT GTT AAA TCA ATT GAA TTA GCT     192
   Lys Ser Gln Thr Pro Ala Glu Asp Thr Val Lys Ser Ile Glu Leu Ala
       50                  55                  60
   GAA GCT AAA GTC TTA GCT AAC AGA GAA CTT GAC AAA TAT GGA GTA AGT     240
   Glu Ala Lys Val Leu Ala Asn Arg Glu Leu Asp Lys Tyr Gly Val Ser
   65                  70                  75                  80
   GAC TAT CAC AAG AAC CTA ATC AAC AAT GCC AAA ACT GTT GAA GGT GTA     288
   Asp Tyr His Lys Asn Leu Ile Asn Asn Ala Lys Thr Val Glu Gly Val
                   85                  90                  95
   AAA GAC CTT CAA GCA CAA GTT GTT GAA TCA GCG AAG AAA GCG CGT ATT     336
   Lys Asp Leu Gln Ala Gln Val Val Glu Ser Ala Lys Lys Ala Arg Ile
               100                 105                 110
   TCA GAA GCA ACA GAT GGC TTA TCT GAT TTC TTG AAA TCA CAA ACA CCT     384
   Ser Glu Ala Thr Asp Gly Leu Ser Asp Phe Leu Lys Ser Gln Thr Pro
           115                 120                 125
   GCT GAA GAT ACT GTT AAA TCA ATT GAA TTA GCT GAA GCT AAA GTC TTA     432
   Ala Glu Asp Thr Val Lys Ser Ile Glu Leu Ala Glu Ala Lys Val Leu
       130                 135                 140
   GCT AAC AGA GAA CTT GAC AAA TAT GGA GTA AGT GAC TAT TAC AAG AAC     480
   Ala Asn Arg Glu Leu Asp Lys Tyr Gly Val Ser Asp Tyr Tyr Lys Asn
   145                 150                 155                 160
   CTA ATC AAC AAT GCC AAA ACT GTT GAA GGT GTA AAA GCA CTG ATA GAT     528
   Leu Ile Asn Asn Ala Lys Thr Val Glu Gly Val Lys Ala Leu Ile Asp
                   165                 170                 175
   GAA ATT TTA GCT GCA TTA CCT AAG ACT GAC ACT TAC AAA TTA ATC CTT     576
   Glu Ile Leu Ala Ala Leu Pro Lys Thr Asp Thr Tyr Lys Leu Ile Leu
               180                 185                 190
   AAT GGT AAA ACA TTG AAA GGC GAA ACA ACT ACT GAA GCT GTT GAT GCT     624
   Asn Gly Lys Thr Leu Lys Gly Glu Thr Thr Thr Glu Ala Val Asp Ala
           195                 200                 205
   GCT ACT GCA AGA TCT TTC AAT TTC CCT ATC CTC                        657
   Ala Thr Ala Arg Ser Phe Asn Phe Pro Ile Leu
      210                     215
(2) INFORMATIONS POUR LA SEQ ID NO: 75:
   (i) CARACTERISTIQUES DE LA SEQUENCE:
      (A) LONGUEUR: 324 paires de bases
      (B) TYPE: nucléotide
      (C) NOMBRE DE BRINS: simple
      (D) CONFIGURATION: linéaire
   (ii) TYPE DE MOLECULE: ADN
   (ix) CARACTERISTIQUE:
      (A) NOM/CLE: CDS
      (B) EMPLACEMENT:1..324
   (xi) DESCRIPTION DE LA SEQUENCE: SEQ ID NO: 75:
   AAA TAT GGA GTA AGT GAC TAT CAC AAG AAC CTA ATC AAC AAT GCC AAA      48
   Lys Tyr Gly Val Ser Asp Tyr His Lys Asn Leu Ile Asn Asn Ala Lys
    1               5                  10                  15
   ACT GTT GAA GGT GTA AAA GAC CTT CAA GCA CAA GTT GTT GAA TCA GCG      96
   Thr Val Glu Gly Val Lys Asp Leu Gln Ala Gln Val Val Glu Ser Ala
               20                  25                  30
   AAG AAA GCG CGT ATT TCA GAA GCA ACA GAT GGC TTA TCT GAT TTC TTG     144
   Lys Lys Ala Arg Ile Ser Glu Ala Thr Asp Gly Leu Ser Asp Phe Leu
           35                  40                  45
   AAA TCA CAA ACA CCT GCT GAA GAT ACT GTT AAA TCA ATT GAA TTA GCT     192
   Lys Ser Gln Thr Pro Ala Glu Asp Thr Val Lys Ser Ile Glu Leu Ala
       50                  55                  60
   GAA GCT AAA GTC TTA GCT AAC AGA GAA CTT GAC AAA TAT GGA GTA AGT     240
   Glu Ala Lys Val Leu Ala Asn Arg Glu Leu Asp Lys Tyr Gly Val Ser
   65                  70                  75                  80
   GAC TAT TAC AAG AAC CTA ATC AAC AAT GCC AAA ACT GTT GAA GGT GTA     288
   Asp Tyr Tyr Lys Asn Leu Ile Asn Asn Ala Lys Thr Val Glu Gly Val
                   85                  90                  95
   AAA GCA CTG ATA GAT GAA ATT TTA GCT GCA TTA CCT                      324
   Lys Ala Leu Ile Asp Glu Ile Leu Ala Ala Leu Pro
               100                 105
(2) INFORMATIONS POUR LA SEQ ID NO: 76:
   (i) CARACTERISTIQUES DE LA SEQUENCE:
      (A) LONGUEUR: 1050 paires de bases
      (B) TYPE: nucléotide
      (C) NOMBRE DE BRINS: simple
      (D) CONFIGURATION: linéaire
   (ii) TYPE DE MOLECULE: ADN
   (ix) CARACTERISTIQUE:
      (A) NOM/CLE: CDS
      (B) EMPLACEMENT:1..1050
   (xi) DESCRIPTION DE LA SEQUENCE: SEQ ID NO: 76:
   ATG AAA GCA ATT TTC GTA CTG AAT GCG CAA CAC GAT GAA GCC GTA GAC      48
   Met Lys Ala Ile Phe Val Leu Asn Ala Gln His Asp Glu Ala Val Asp
     1               5                  10                  15
   GCG AAT TTC GAC CAA TTC AAC AAA TAT GGA GTA AGT GAC TAT TAC AAG      96
   Ala Asn Phe Asp Gln Phe Asn Lys Tyr Gly Val Ser Asp Tyr Tyr Lys
                20                  25                  30
   AAT CTA ATC AAC AAT GCC AAA ACT GTT GAA GGC GTA AAA GAC CTT CAA     144
   Asn Leu Ile Asn Asn Ala Lys Thr Val Glu Gly Val Lys Asp Leu Gln
            35                  40                  45
   GCA CAA GTT GTT GAA TCA GCG AAG AAA GCG CGT ATT TCA GAA GCA ACA     192
   Ala Gln Val Val Glu Ser Ala Lys Lys Ala Arg Ile Ser Glu Ala Thr
        50                  55                  60
   GAT GGC TTA TCT GAT TTC TTG AAA TCA CAA ACA CCT GCT GAA GAT ACT     240
   Asp Gly Leu Ser Asp Phe Leu Lys Ser Gln Thr Pro Ala Glu Asp Thr
    65                  70                  75                  80
   GTT AAA TCA ATT GAA TTA GCT GAA GCT AAA GTC TTA GCT AAC AGA GAA     288
   Val Lys Ser Ile Glu Leu Ala Glu Ala Lys Val Leu Ala Asn Arg Glu
                    85                  90                  95
   CTT GAC AAA TAT GGA GTA AGT GAC TAT CAC AAG AAC CTA ATC AAC AAT     336
   Leu Asp Lys Tyr Gly Val Ser Asp Tyr His Lys Asn Leu Ile Asn Asn
               100                 105                 110
   GCC AAA ACT GTT GAA GGT GTA AAA GAC CTT CAA GCA CAA GTT GTT GAA      384
   Ala Lys Thr Val Glu Gly Val Lys Asp Leu Gln Ala Gln Val Val Glu
           115                 120                 125
   TCA GCG AAG AAA GCG CGT ATT TCA GAA GCA ACA GAT GGC TTA TCT GAT      432
   Ser Ala Lys Lys Ala Arg Ile Ser Glu Ala Thr Asp Gly Leu Ser Asp
       130                 135                 140
   TTC TTG AAA TCA CAA ACA CCT GCT GAA GAT ACT GTT AAA TCA ATT GAA      480
   Phe Leu Lys Ser Gln Thr Pro Ala Glu Asp Thr Val Lys Ser Ile Glu
   145                 150                 155                 160
   TTA GCT GAA GCT AAA GTC TTA GCT AAC AGA GAA CTT GAC AAA TAT GGA      528
   Leu Ala Glu Ala Lys Val Leu Ala Asn Arg Glu Leu Asp Lys Tyr Gly
                   165                 170                 175
   GTA AGT GAC TAT TAC AAG AAC CTA ATC AAC AAT GCC AAA ACT GTT GAA      576
   Val Ser Asp Tyr Tyr Lys Asn Leu Ile Asn Asn Ala Lys Thr Val Glu
               180                 185                 190
   GGT GTA AAA GCA CTG ATA GAT GAA ATT TTA GCT GCA TTA CCT AAG ACT      624
   Gly Val Lys Ala Leu Ile Asp Glu Ile Leu Ala Ala Leu Pro Lys Thr
           195                 200                 205
   GAC ACT TAC AAA TTA ATC CTT AAT GGT AAA ACA TTG AAA GGC GAA ACA      672
   Asp Thr Tyr Lys Leu Ile Leu Asn Gly Lys Thr Leu Lys Gly Glu Thr
       210                 215                 220
   ACT ACT GAA GCT GTT GAT GCT GCT ACT GCA AGA TCT TTC AAT TTC CCT      720
   Thr Thr Glu Ala Val Asp Ala Ala Thr Ala Arg Ser Phe Asn Phe Pro
   225                 230                 235                 240
   ATC CTC GAG AAT TCC ATG ACC GTG AAA ACC AAA AAC ACC ACG ACC ACC      768
   Ile Leu Glu Asn Ser Met Thr Val Lys Thr Lys Asn Thr Thr Thr Thr
                   245                 250                 255
   CAG ACC CAG CCG AGC AAA CCG ACC ACC AAA CAG CGT CAG AAC AAA CCG      816
   Gln Thr Gln Pro Ser Lys Pro Thr Thr Lys Gln Arg Gln Asn Lys Pro
               260                 265                 270
   CCG AAC AAA CCG AAC AAC GAT TTC CAT TTC GAA GTG TTC AAC TTC GTG      864
   Pro Asn Lys Pro Asn Asn Asp Phe His Phe Glu Val Phe Asn Phe Val
           275                 280                 285
   CCG TGC AGC ATC TGC AGC AAC AAC CCG ACC TGC TGG GCG ATC TGC AAA      912
   Pro Cys Ser Ile Cys Ser Asn Asn Pro Thr Cys Trp Ala Ile Cys Lys
       290                 295                 300
   CGT ATC CCG AAC AAA AAA CCG GGC AAA AAA ACC ACG ACC AAA CCG ACC     960
   Arg Ile Pro Asn Lys Lys Pro Gly Lys Lys Thr Thr Thr Lys Pro Thr
   305                 310                 315                 320
   AAA AAA CCG ACC TTC AAA ACC ACC AAA AAA GAT CAT AAA CCG CAG ACC    1008
   Lys Lys Pro Thr Phe Lys Thr Thr Lys Lys Asp His Lys Pro Gln Thr
                   325                 330                 335
   ACC AAA CCG AAA GAA GTG CCG ACC ACC AAA CCG GTC GAC TAA            1050
   Thr Lys Pro Lys Glu Val Pro Thr Thr Lys Pro Val Asp
               340                 345
(2) INFORMATIONS POUR LA SEQ ID NO: 77:
   (i) CARACTERISTIQUES DE LA SEQUENCE:
      (A) LONGUEUR: 1071 paires de bases
      (B) TYPE: nucléotide
      (C) NOMBRE DE BRINS: simple
      (D) CONFIGURATION: linéaire
   (ii) TYPE DE MOLECULE: ADN
   (ix) CARACTERISTIQUE:
      (A) NOM/CLE: CDS
      (B) EMPLACEMENT:1..10̸71
   (xi) DESCRIPTION DE LA SEQUENCE: SEQ ID NO: 77:
   ATG AAA GCA ATT TTC GTA CTG AAT GCG CAA CAC GAT GAA GCC GTA GAC      48
   Met Lys Ala Ile Phe Val Leu Asn Ala Gln His Asp Glu Ala Val Asp
     1               5                  10                  15
   GCG AAT TTC GAC CAA TTC AAC AAA TAT GGA GTA AGT GAC TAT TAC AAG      96
   Ala Asn Phe Asp Gln Phe Asn Lys Tyr Gly Val Ser Asp Tyr Tyr Lys
               20                  25                  30
   AAT CTA ATC AAC AAT GCC AAA ACT GTT GAA GGC GTA AAA GAC CTT CAA     144
   Asn Leu Ile Asn Asn Ala Lys Thr Val Glu Gly Val Lys Asp Leu Gln
           35                  40                  45
   GCA CAA GTT GTT GAA TCA GCG AAG AAA GCG CGT ATT TCA GAA GCA ACA     192
   Ala Gln Val Val Glu Ser Ala Lys Lys Ala Arg Ile Ser Glu Ala Thr
       50                  55                  60
   GAT GGC TTA TCT GAT TTC TTG AAA TCA CAA ACA CCT GCT GAA GAT ACT      240
   Asp Gly Leu Ser Asp Phe Leu Lys Ser Gln Thr Pro Ala Glu Asp Thr
   65                  70                  75                  80
   GTT AAA TCA ATT GAA TTA GCT GAA GCT AAA GTC TTA GCT AAC AGA GAA      288
   Val Lys Ser Ile Glu Leu Ala Glu Ala Lys Val Leu Ala Asn Arg Glu
                   85                  90                  95
   CTT GAC AAA TAT GGA GTA AGT GAC TAT CAC AAG AAC CTA ATC AAC AAT      336
   Leu Asp Lys Tyr Gly Val Ser Asp Tyr His Lys Asn Leu Ile Asn Asn
               100                 105                 110
   GCC AAA ACT GTT GAA GGT GTA AAA GAC CTT CAA GCA CAA GTT GTT GAA      384
   Ala Lys Thr Val Glu Gly Val Lys Asp Leu Gln Ala Gln Val Val Glu
           115                 120                 125
   TCA GCG AAG AAA GCG CGT ATT TCA GAA GCA ACA GAT GGC TTA TCT GAT      432
   Ser Ala Lys Lys Ala Arg Ile Ser Glu Ala Thr Asp Gly Leu Ser Asp
       130                 135                 140
   TTC TTG AAA TCA CAA ACA CCT GCT GAA GAT ACT GTT AAA TCA ATT GAA      480
   Phe Leu Lys Ser Gln Thr Pro Ala Glu Asp Thr Val Lys Ser Ile Glu
   145                 150                 155                 160
   TTA GCT GAA GCT AAA GTC TTA GCT AAC AGA GAA CTT GAC AAA TAT GGA      528
   Leu Ala Glu Ala Lys Val Leu Ala Asn Arg Glu Leu Asp Lys Tyr Gly
                   165                 170                 175
   GTA AGT GAC TAT TAC AAG AAC CTA ATC AAC AAT GCC AAA ACT GTT GAA      576
   Val Ser Asp Tyr Tyr Lys Asn Leu Ile Asn Asn Ala Lys Thr Val Glu
               180                 185                 190
   GGT GTA AAA GCA CTG ATA GAT GAA ATT TTA GCT GCA TTA CCT AAG ACT      624
   Gly Val Lys Ala Leu Ile Asp Glu Ile Leu Ala Ala Leu Pro Lys Thr
           195                 200                 205
   GAC ACT TAC AAA TTA ATC CTT AAT GGT AAA ACA TTG AAA GGC GAA ACA      672
   Asp Thr Tyr Lys Leu Ile Leu Asn Gly Lys Thr Leu Lys Gly Glu Thr
       210                 215                 220
   ACT ACT GAA GCT GTT GAT GCT GCT ACT GCA AGA TCT TTC AAT TTC CCT      720
   Thr Thr Glu Ala Val Asp Ala Ala Thr Ala Arg Ser Phe Asn Phe Pro
   225                 230                 235                 240
   ATC CTC GAG AAT TCG AGC TCG GTA CCC GGG GAT CCT ATG ACC GTG AAA      768
   Ile Leu Glu Asn Ser Ser Ser Val Pro Gly Asp Pro Met Thr Val Lys
                   245                 250                 255
   ACC AAA AAC ACC ACG ACC ACC CAG ACC CAG CCG AGC AAA CCG ACC ACC      816
   Thr Lys Asn Thr Thr Thr Thr Gln Thr Gln Pro Ser Lys Pro Thr Thr
               260                 265                 270
   AAA CAG CGT CAG AAC AAA CCG CCG AAC AAA CCG AAC AAC GAT TTC CAT      864
   Lys Gln Arg Gln Asn Lys Pro Pro Asn Lys Pro Asn Asn Asp Phe His
           275                 280                 285
   TTC GAA GTG TTC AAC TTC GTG CCG AGC AGC ATC TGC AGC AAC AAC CCG      912
   Phe Glu Val Phe Asn Phe Val Pro Ser Ser Ile Cys Ser Asn Asn Pro
       290                 295                 300
   ACC TGC TGG GCG ATC AGC AAA CGT ATC CCG AAC AAA AAA CCG GGC AAA      960
   Thr Cys Trp Ala Ile Ser Lys Arg Ile Pro Asn Lys Lys Pro Gly Lys
   305                 310                 315                 320
   AAA ACC ACG ACC AAA CCG ACC AAA AAA CCG ACC TTC AAA ACC ACC AAA     1008
   Lys Thr Thr Thr Lys Pro Thr Lys Lys Pro Thr Phe Lys Thr Thr Lys
                   325                 330                 335
   AAA GAT CAT AAA CCG CAG ACC ACC AAA CCG AAA GAA GTG CCG ACC ACC     1056
   Lys Asp His Lys Pro Gln Thr Thr Lys Pro Lys Glu Val Pro Thr Thr
               340                 345                 350
   AAA CCG GTC GAC TAA                                                 1071
   Lys Pro Val Asp
           355
(2) INFORMATIONS POUR LA SEQ ID NO: 78:
   (i) CARACTERISTIQUES DE LA SEQUENCE:
      (A) LONGUEUR: 726 poires de bases
      (B) TYPE: nucléotide
      (C) NOMBRE DE BRINS: simple
      (D) CONFIGURATION: linéaire
   (ii) TYPE DE MOLECULE: ADN
   (ix) CARACTERISTIQUE:
      (A) NOM/CLE: CDS
      (B) EMPLACEMENT:1..726
   (xi) DESCRIPTION DE LA SEQUENCE: SEQ ID NO: 78:
   ATG AAA GCA ATT TTC GTA CTG AAT GCG CAA CAC GAT GAA GCC GTA GAC 48
   Met Lys Ala Ile Phe Val Leu Asn Ala Gln His Asp Glu Ala Val Asp
   1 5 10 15
   GCG AAT TTC GAC CAA TTC AAC AAA TAT GGA GTA AGT GAC TAT TAC AAG 96
   Ala Asn Phe Asp Gln Phe Asn Lys Tyr Gly Val Ser Asp Tyr Tyr Lys
   20 25 30
   AAT CTA ATC AAC AAT GCC AAA ACT GTT GAA GGC GTA AAA GAC CTT CAA 144
   Asn Leu Ile Asn Asn Ala Lys Thr Val Glu Gly Val Lys Asp Leu Gln
   35 40 45
   GCA CAA GTT GTT GAA TCA GCG AAG AAA GCG CGT ATT TCA GAA GCA ACA 192
   Ala Gln Val Val Glu Ser Ala Lys Lys Ala Arg Ile Ser Glu Ala Thr
   50 55 60
   GAT GGC TTA TCT GAT TTC TTG AAA TCA CAA ACA CCT GCT GAA GAT ACT 240
   Asp Gly Leu Ser Asp Phe Leu Lys Ser Gln Thr Pro Ala Glu Asp Thr
   65 70 75 80
   GTT AAA TCA ATT GAA TTA GCT GAA GCT AAA GTC TTA GCT AAC AGA GAA 288
   Val Lys Ser Ile Glu Leu Ala Glu Ala Lys Val Leu Ala Asn Arg Glu
   85 90 95
   CTT GAC AAA TAT GGA GTA AGT GAC TAT CAC AAG AAC CTA ATC AAC AAT 336
   Leu Asp Lys Tyr Gly Val Ser Asp Tyr His Lys Asn Leu Ile Asn Asn
   100 105 110
   GCC AAA ACT GTT GAA GGT GTA AAA GAC CTT CAA GCA CAA GTT GTT GAA 384
   Ala Lys Thr Val Glu Gly Val Lys Asp Leu Gln Ala Gln Val Val Glu
   115 120 125
   TCA GCG AAG AAA GCG CGT ATT TCA GAA GCA ACA GAT GGC TTA TCT GAT 432
   Ser Ala Lys Lys Ala Arg Ile Ser Glu Ala Thr Asp Gly Leu Ser Asp
   130 135 140
   TTC TTG AAA TCA CAA ACA CCT GCT GAA GAT ACT GTT AAA TCA ATT GAA 480
   Phe Leu Lys Ser Gln Thr Pro Ala Glu Asp Thr Val Lys Ser Ile Glu
   145 150 155 160
   TTA GCT GAA GCT AAA GTC TTA GCT AAC AGA GAA CTT GAC AAA TAT GGA 528
   Leu Ala Glu Ala Lys Val Leu Ala Asn Arg Glu Leu Asp Lys Tyr Gly
   165 170 175
   GTA AGT GAC TAT TAC AAG AAC CTA ATC AAC AAT GCC AAA ACT GTT GAA 576
   Val Ser Asp Tyr Tyr Lys Asn Leu Ile Asn Asn Ala Lys Thr Val Glu
   180 185 190
   GGT GTA AAA GCA CTG ATA GAT GAA ATT TTA GCT GCA TTA CCT AAG ACT      624
   Gly Val Lys Ala Leu Ile Asp Glu Ile Leu Ala Ala Leu Pro Lys Thr
           195                 200                 205
   GAC ACT TAC AAA TTA ATC CTT AAT GGT AAA ACA TTG AAA GGC GAA ACA      672
   Asp Thr Tyr Lys Leu Ile Leu Asn Gly Lys Thr Leu Lys Gly Glu Thr
       210                 215                 220
   ACT ACT GAA GCT GTT GAT GCT GCT ACT GCA AGA TCT TTC AAT TTC CCT      720
   Thr Thr Glu Ala Val Asp Ala Ala Thr Ala Arg Ser Phe Asn Phe Pro
   225                 230                 235                 240
   ATC CTC                                                              726
   Ile Leu